# EUROPEAN PATENT APPLICATION

(11) **EP 4 559 912 A1**
(43) Date of publication of application: **28.05.2025**
(21) Application number: 23842368.5
(22) Date of filing: 19.07.2023
(51) Int. Cl.: C07D 417/04, C07D 417/14, A61K 31/433, A61K 31/497, A61P 35/00

(54) **SULFUR-CONTAINING HETEROAROMATIC RING COMPOUND, PHARMACEUTICAL COMPOSITION THEREOF, AND USE THEREOF**

(30) Priority: 19.07.2022 CN 202210845550; 02.08.2022 CN 202210924422; 29.08.2022 CN 202211043823; 07.09.2022 CN 202211077255; 13.10.2022 CN 202211239150; 21.10.2022 CN 202211298632; 02.11.2022 CN 202211365392; 06.01.2023 CN 202310023132; 16.03.2023 CN 202310253404; 29.05.2023 CN 202310617938; 03.07.2023 CN 202310800336
(71) Applicant: Evopoint Biosciences Co., Ltd., Suzhou, Jiangsu 215000 (CN)
(72) Inventor: HU, Yonghan, Suzhou, Jiangsu 215000 (CN); WAN, Dawei, Suzhou, Jiangsu 215000 (CN); MI, Guorui, Suzhou, Jiangsu 215000 (CN); CHEN, Hao, Suzhou, Jiangsu 215000 (CN); XU, Jialiang, Suzhou, Jiangsu 215000 (CN); XU, Jiajing, Suzhou, Jiangsu 215000 (CN); MA, Ben, Suzhou, Jiangsu 215000 (CN); WEI, Haiyang, Suzhou, Jiangsu 215000 (CN)
(74) Representative: Michalski Hüttermann & Partner Patentanwälte mbB
(86) International application number: PCT/CN2023/108212
(87) International publication number: WO 2024/017306

(57) **Abstract**

Disclosed are a sulfur-containing heteroaromatic ring compound, a pharmaceutical composition thereof, and a use thereof. Specifically disclosed are a compound as represented by formula (I), a pharmaceutically acceptable salt thereof, a solvate thereof, or a solvate of a pharmaceutically acceptable salt thereof. The sulfur-containing heteroaromatic ring compound of the present invention has good PARG inhibitory activity.

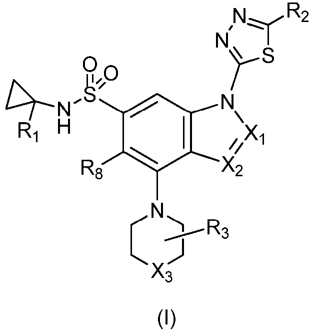

## Description

The present application claims priority to the following Chinese patent applications:
Chinese patent application No. 2022108455508 filed on July 19, 2022;
Chinese patent application No. 2022109244222 filed on August 02, 2022;
Chinese patent application No. 2022110438233 filed on August 29, 2022;
Chinese patent application No. 2022110772559 filed on September 07, 2022;
Chinese patent application No. 2022112391509 filed on October 13, 2022;
Chinese patent application No. 2022112986321 filed on October 21, 2022;
Chinese patent application No. 2022113653922 filed on November 02, 2022;
Chinese patent application No. 2023100231325 filed on January 06, 2023;
Chinese patent application No. 2023102534040 filed on March 16, 2023;
Chinese patent application No. 2023106179387 filed on May 29, 2023; and
Chinese patent application No. 2023108003365 filed on July 03, 2023.

The contents of the Chinese patent applications are incorporated herein by reference in their entireties.

### TECHNICAL FIELD

The present disclosure relates to a sulfur-containing heteroaromatic ring compound, a pharmaceutical composition thereof, and use thereof.

### BACKGROUND

Cancer is caused by uncontrolled and unregulated cell proliferation. The result of this typically rapid proliferation is high levels of oxidative stress within the tumor, which damages DNA and causes a significant increase in DNA mutation rate. Thus, the survival of tumor cells is heavily dependent on DNA damage repair mechanisms.

Single-strand breaks (SSBs) in DNA are the most common type of damage in cells. In the repair mechanisms of single-strand break repair (SSBR) and base excision repair (BER), PARG (poly ADP-ribose glycohydrolase) and PARP (poly ADP-ribose polymerase), along with many other proteins, are involved in the repair. Cancer cells may become addicted to a particular DNA repair pathway when other DNA repair mechanisms fail. Tumors bearing mutations in proteins involved in double-strand break repair are generally more sensitive to PARP inhibitors of SSBR.

PARP inhibitors are currently undergoing numerous clinical trials in which the concept of synthetic lethality or chemosensitization is being explored. It has been reported that PARP inhibitors have clinical resistance, so there is a need to find alternative inhibitors targeting DNA damage repair mechanisms. Since PARG knockout results in a reduction in SSBR rate to the same extent as PARP 1 knockout, PARG inhibition may have a therapeutic advantage in PARP inhibitor-resistant cells.

### SUMMARY

The technical problem to be solved in the present disclosure is for overcoming the defect of insufficient types of PARG inhibitors in the prior art. Therefore, the present disclosure provides a sulfur-containing heteroaromatic ring compound with a novel structure, a pharmaceutical composition thereof, and use thereof. The sulfur-containing heteroaromatic ring compound of the present disclosure has good PARG inhibitory activity.

The present disclosure solves the above-mentioned technical problem through the following technical solutions.

The present disclosure provides a compound represented by formula (I), a pharmaceutically acceptable salt thereof, a solvate thereof, or a solvate of the pharmaceutically acceptable salt thereof: 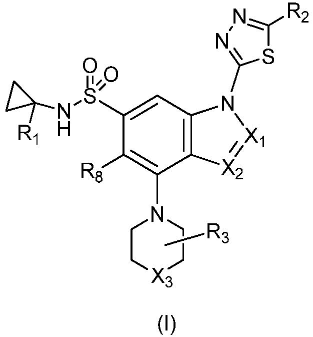 wherein
X₁ and X₂ are independently N or CR₇;
R₇ is independently hydrogen, deuterium, halogen, CN, OH, NRₐR_{b}, C₁-C₆ alkyl, or C₁-C₆ alkyl substituted with 1 or more R₇₋₂;
Rₐ and R_{b} are independently hydrogen or C₁-C₆ alkyl;
R₇₋₂ is independently deuterium, halogen, CN, OH, or NRₐR_{b};
R₈ is hydrogen or halogen;
R₁ is hydrogen, deuterium, halogen, OH, CN, NRₐR_{b}, C₂-C₄ alkynyl, C₁-C₆ alkyl, or C₁-C₆ alkyl substituted with 1 or more R₁₋₂;
R₁₋₂ is independently deuterium, halogen, OH, or NRₐR_{b};
R₂ is hydrogen, deuterium, OH, C₁-C₆ alkyl, C₃-C₆ cycloalkyl, C₁-C₆ alkyl substituted with 1 or more R₂₋₁, or C₃-C₆ cycloalkyl substituted with 1 or more R₂₋₂;
R₂₋₁ and R₂₋₂ are independently deuterium, OH, halogen, or NRₐR_{b};
R₃ is hydrogen, deuterium, OH, NRₐR_{b}, C₁-C₆ alkyl, or C₁-C₆ alkyl substituted with 1 or more R₃₋₂;
R₃₋₂ is independently deuterium, OH, NRₐR_{b}, or halogen;
X₃ is N-(C=O)-L-CR₅R₆-OR₁₁ or N-(C=O)-(C=O)-R₁₂;
L is (CR₉R₁₀)m;
R₉ and R₁₀ are independently hydrogen, deuterium, OH, CN, N(R₉₋₁)₂, C₁-C₆ alkyl, or C₁-C₆ alkyl substituted with one or more R₉₋₂;
R₉₋₁ is independently hydrogen or C₁-C₆ alkyl;
R₉₋₂ is independently deuterium, OH, N(R₉₋₁)₂, or halogen;
m is 0 or 1;
R₅ and R₆ are independently hydrogen, deuterium, OH, CN, NRₐR_{b}, C₁-C₆ alkyl, C₃-C₆ cycloalkyl, C₁-C₆ alkyl substituted with 1 or more R₅₋₂, or C₃-C₆ cycloalkyl substituted with 1 or more R₅₋₃;
R₅₋₂ and R₅₋₃ are independently deuterium, OH, NRₐR_{b}, or halogen; or
R₅ and R₆, together with the carbon atom to which they are attached, form C₃-C₆ cycloalkyl, 3- to 6-membered heterocycloalkyl, C₃-C₆ cycloalkyl substituted with 1 or more R₅₋₃, or 3- to 6-membered heterocycloalkyl substituted with 1 or more R₅₋₄, wherein in each "3- to 6-membered heterocycloalkyl", the number of the heteroatom is independently 1 or 2, and the type of the heteroatom is independently selected from one or more of N, O, and S;
R₅₋₃ and R₅₋₄ are independently deuterium, hydroxy, NRₐR_{b}, or halogen;
R₁₁ is hydrogen, C₁-C₆ alkyl, or C₁-C₆ alkyl substituted with 1 or more halogens;
R₁₂ is NRₐR_{b}, C₁-C₆ alkyl, C₃-C₆ cycloalkyl, C₁-C₆ alkyl substituted with 1 or more R₁₂₋₁, or C₃-C₆ cycloalkyl substituted with one or more R₁₂₋₂;
R₁₂₋₁ and R₁₂₋₂ are independently halogen, OH, CN, or NRₐR_{b}.

In certain preferred embodiments of the present disclosure, certain groups in the compound represented by formula (I), the pharmaceutically acceptable salt thereof, the solvate thereof, and the solvate of the pharmaceutically acceptable salt thereof are defined as follows, and groups not mentioned are as described in any one of the embodiments of the present disclosure (referred to as "in a certain embodiment of the present disclosure").

In a certain embodiment of the present disclosure, each "halogen" may independently be F, Cl, Br, or I, such as F.

In a certain embodiment of the present disclosure, each "C₁-C₆ alkyl" may independently be methyl, ethyl, n-propyl, isopropyl, n-butyl, sec-butyl, or *tert-butyl,* such as methyl, ethyl, isopropyl, or *tert-*butyl.

In a certain embodiment of the present disclosure, each "C₂-C₄ alkynyl" may independently be ethynyl, propinyl, or propargyl, such as ethynyl.

In a certain embodiment of the present disclosure, each "C₃-C₆ cycloalkyl" may independently be cyclopropyl, cyclobutyl, cyclopentyl, or cyclohexyl, such as cyclopropyl.

In a certain embodiment of the present disclosure, each "3- to 6-membered heterocycloalkyl" may independently be 3- to 6-membered heterocycloalkyl having 1 or 2 heteroatoms of N and/or O, such as oxiranyl, aziridinyl, oxetanyl, azetidinyl, tetrahydropyrrolyl, tetrahydrofuranyl, tetrahydropyranyl, piperidinyl, morpholinyl, or piperazinyl.

In a certain embodiment of the present disclosure, X₂ is CR₇.

In a certain embodiment of the present disclosure, R₇ is independently hydrogen or halogen.

In a certain embodiment of the present disclosure, R₈ is hydrogen.

In a certain embodiment of the present disclosure, R₁ is hydrogen, CN, C₂-C₄ alkynyl, or C₁-C₆ alkyl, preferably CN, C₂-C₄ alkynyl, or C₁-C₆ alkyl, and further preferably CN or C₁-C₆ alkyl.

In a certain embodiment of the present disclosure, R₂ is C₁-C₆ alkyl or C₁-C₆ alkyl substituted with 1, 2, or 3 R₂₋₁, preferably methyl or methyl substituted with 1, 2, or 3 R₂₋₁, and further preferably methyl substituted with 1, 2, or 3 R₂₋₁.

In a certain embodiment of the present disclosure, R₂₋₁ is halogen.

In a certain embodiment of the present disclosure, R₂ is C₁-C₆ alkyl or C₁-C₆ alkyl substituted with 1, 2, or 3 R₂₋₁, wherein R₂₋₁ is halogen;
preferably, R₂ is methyl or methyl substituted with 1, 2, or 3 R₂₋₁, wherein R₂₋₁ is halogen;
more preferably, R₂ is methyl substituted with 1, 2, or 3 R₂₋₁, wherein R₂₋₁ is halogen.

In a certain embodiment of the present disclosure, R₃ is hydrogen, C₁-C₆ alkyl, or C₁-C₆ alkyl substituted with 1, 2, or 3 R₃₋₂, preferably C₁-C₆ alkyl or C₁-C₆ alkyl substituted with 1, 2, or 3 R₃₋₂, and further preferably methyl or methyl substituted with 1, 2, or 3 R₃₋₂.

In a certain embodiment of the present disclosure, R₃₋₂ is independently halogen.

In a certain embodiment of the present disclosure, R₃ is hydrogen, C₁-C₆ alkyl, or C₁-C₆ alkyl substituted with 1, 2, or 3 R₃₋₂, wherein R₃₋₂ is independently halogen;
preferably, R₃ is C₁-C₆ alkyl or C₁-C₆ alkyl substituted with 1, 2, or 3 R₃₋₂, wherein R₃₋₂ is independently halogen;
more preferably, R₃ is methyl or methyl substituted with 1, 2, or 3 R₃₋₂, wherein R₃₋₂ is independently halogen.

In a certain embodiment of the present disclosure, R₉ and R₁₀ are independently hydrogen, N(R₉₋₁)₂, or C₁-C₆ alkyl, such as N(R₉₋₁)₂ or C₁-C₆ alkyl, preferably C₁-C₆ alkyl.

In a certain embodiment of the present disclosure, R₉₋₁ is independently hydrogen.

In a certain embodiment of the present disclosure, R₉ and R₁₀ are independently N(R₉₋₁)₂ or C₁-C₆ alkyl, wherein R₉₋₁ is independently hydrogen.

In a certain embodiment of the present disclosure, R₁₀ is hydrogen.

In a certain embodiment of the present disclosure, m is 0.

In a certain embodiment of the present disclosure, R₅ and R₆ are independently hydrogen, deuterium, C₁-C₆ alkyl, C₃-C₆ cycloalkyl, C₁-C₆ alkyl substituted with 1, 2, or 3 R₅₋₂, or C₃-C₆ cycloalkyl substituted with 1, 2, or 3 R₅₋₃, preferably hydrogen, C₁-C₆ alkyl, C₃-C₆ cycloalkyl, or C₁-C₆ alkyl substituted with 1, 2, or 3 R₅₋₂, and further preferably C₁-C₆ alkyl or C₁-C₆ alkyl substituted with 1, 2, or 3 R₅₋₂.

In a certain embodiment of the present disclosure, R₅₋₂ and R₅₋₃ are independently OH or halogen, preferably halogen.

In a certain embodiment of the present disclosure, R₅ and R₆ are independently hydrogen, deuterium, C₁-C₆ alkyl, C₃-C₆ cycloalkyl, C₁-C₆ alkyl substituted with 1, 2, or 3 R₅₋₂, or C₃-C₆ cycloalkyl substituted with 1, 2, or 3 R₅₋₃, wherein R₅₋₂ and R₅₋₃ are each independently OH or halogen;
preferably, R₅ and R₆ are independently hydrogen, C₁-C₆ alkyl, C₃-C₆ cycloalkyl, or C₁-C₆ alkyl substituted with 1, 2, or 3 R₅₋₂, wherein R₅₋₂ is independently OH or halogen;
more preferably, R₅ and R₆ are independently C₁-C₆ alkyl or C₁-C₆ alkyl substituted with 1, 2, or 3 R₅₋₂, wherein R₅₋₂ is independently halogen.

In a certain embodiment of the present disclosure, R₅ is hydrogen or C₁-C₆ alkyl, and R₆ is hydrogen, C₁-C₆ alkyl, C₃-C₆ cycloalkyl, or C₁-C₆ alkyl substituted with 1 or more R₅₋₂, wherein R₅₋₂ is independently OH or halogen.

In a certain embodiment of the present disclosure, X₁ and X₂ are independently N or CR₇;
R₇ is independently hydrogen or halogen;
R₈ is hydrogen or halogen;
R₁ is CN, C₂-C₄ alkynyl, or C₁-C₆ alkyl;
R₂ is C₁-C₆ alkyl or C₁-C₆ alkyl substituted with 1, 2, or 3 R₂₋₁;
R₂₋₁ is halogen;
R₃ is hydrogen, C₁-C₆ alkyl, or C₁-C₆ alkyl substituted with 1, 2, or 3 R₃₋₂;
R₃₋₂ is independently halogen;
X₃ is N-(C=O)-L-CR₅R₆-OR₁₁ or N-(C=O)-(C=O)-R₁₂;
L is (CHR₉)m;
R₉ is N(R₉₋₁)₂ or C₁-C₆ alkyl;
R₉₋₁ is independently hydrogen;
m is 0 or 1;
R₅ and R₆ are independently hydrogen, C₁-C₆ alkyl, C₃-C₆ cycloalkyl, or C₁-C₆ alkyl substituted with 1, 2, or 3 R₅₋₂;
R₅₋₂ is independently OH or halogen;
R₁₁ is hydrogen, C₁-C₆ alkyl, or C₁-C₆ alkyl substituted with 1, 2, or 3 halogens;
R₁₂ is N(R₁₂₋₁)₂, C₁-C₆ alkyl, or C₃-C₆ cycloalkyl;
R₁₂₋₁ is independently hydrogen.

In a certain embodiment of the present disclosure, X₁ is N or CH.

In a certain embodiment of the present disclosure, X₂ is N, CH, or CF, preferably CH or CF.

In a certain embodiment of the present disclosure, 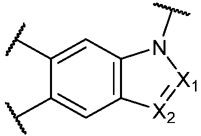 is 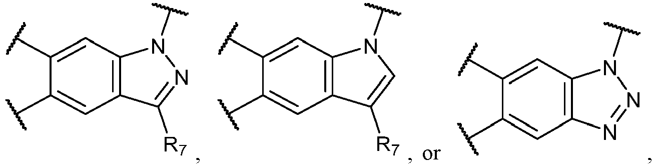 such as

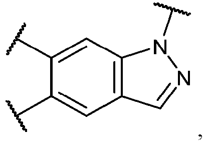

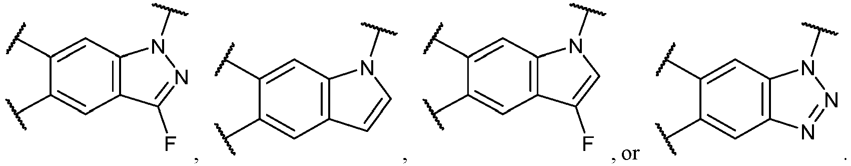

In a certain embodiment of the present disclosure, R₈ is H or F, preferably H.

In a certain embodiment of the present disclosure, R₁ is hydrogen, CN, ethynyl, or methyl, such as CN, ethynyl, or methyl, preferably CN or methyl, and further preferably CN.

In a certain embodiment of the present disclosure, R₂ is methyl or CHF₂, preferably CHF₂.

In a certain embodiment of the present disclosure, R₃ is hydrogen, methyl, or CH₂F, preferably methyl or CH₂F.

In a certain embodiment of the present disclosure, 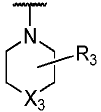 is 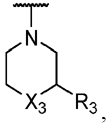 such as

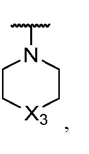

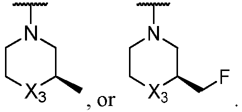

In a certain embodiment of the present disclosure, R₅ is hydrogen or methyl.

In a certain embodiment of the present disclosure, R₆ is hydrogen, methyl, isopropyl, *tert-butyl,* cyclopropyl, methyl substituted with 1, 2, or 3 fluorine atoms, *tert-butyl* substituted with 1, 2, or 3 fluorine atoms, or *tert-butyl* substituted with 1, 2, or 3 OH groups, such as hydrogen, methyl, isopropyl, *tert-butyl,* cyclopropyl, CF₃,

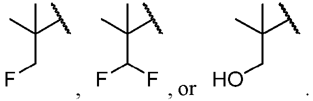

In a certain embodiment of the present disclosure, R₆ is methyl, *tert-butyl,* methyl substituted with 1, 2, or 3 fluorine atoms, or *tert-butyl* substituted with 1, 2, or 3 fluorine atoms, such as methyl, *tert-butyl,* CF₃,

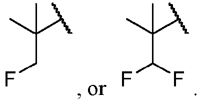

In a certain embodiment of the present disclosure, R₅ is hydrogen or methyl, and

R₆ is hydrogen, methyl, ethyl, n-propyl, isopropyl, n-butyl, isobutyl, *tert-butyl,* cyclopropyl, methyl substituted with 1, 2, or 3 fluorine atoms, *tert-butyl* substituted with 1, 2, or 3 fluorine atoms, or *tert-*butyl substituted with 1, 2, or 3 OH groups, such as hydrogen, methyl, isopropyl, *tert-butyl,* cyclopropyl, CF₃,

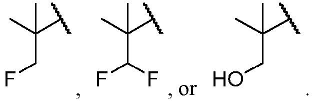

In a certain embodiment of the present disclosure, R₅ is hydrogen or methyl, and
R₆ is methyl, *tert-butyl,* methyl substituted with 1, 2, or 3 fluorine atoms, or *tert-butyl* substituted with 1, 2, or 3 fluorine atoms, such as methyl, *tert-butyl,* CF₃,

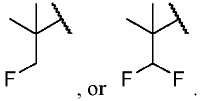

In a certain embodiment of the present disclosure, R₁₁ is hydrogen, methyl, ethyl, or methyl substituted with 1, 2, or 3 fluorine atoms, such as hydrogen, methyl, ethyl, or CHF₂.

In a certain embodiment of the present disclosure, R₁₂ is isopropyl, amino, cyclopropyl, *tert-butyl,* or methyl.

In a certain embodiment of the present disclosure, 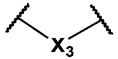 is

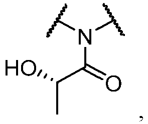

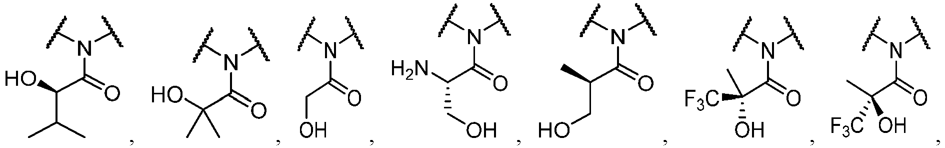

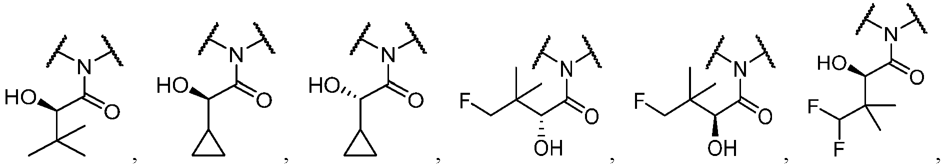

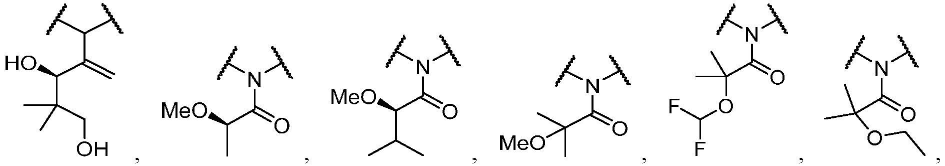

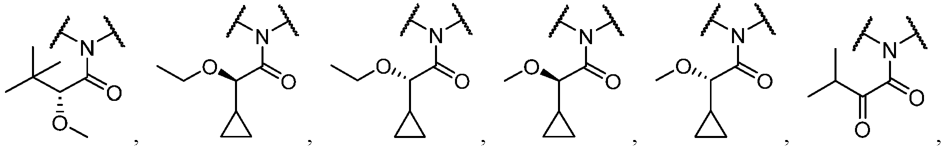

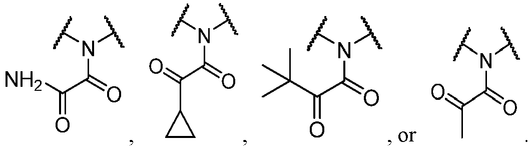

In a certain embodiment of the present disclosure, the compound represented by formula (I) is a compound represented by formula (I-1), (I-2), or (I-3): 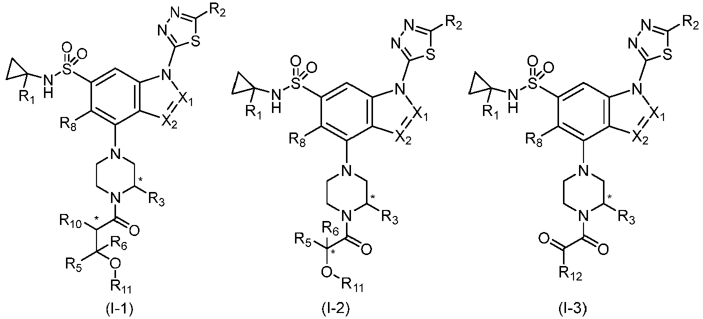 wherein the carbon atom marked with "*" indicates that when the carbon atom is a chiral carbon atom, it is independently in R configuration, S configuration, or a mixture thereof; X₁, X₂, R₈, R₁, R₂, R₃, R₁₀, R₅, R₆, R₁₁, and R₁₂ are as defined in any one of the above embodiments.

In a certain embodiment of the present disclosure, the compound represented by formula (I) is a compound represented by formula (I-5), (I-6), (I-7), (I-8), (I-9), (I-10), (I-11), (I-12) or (I-13): 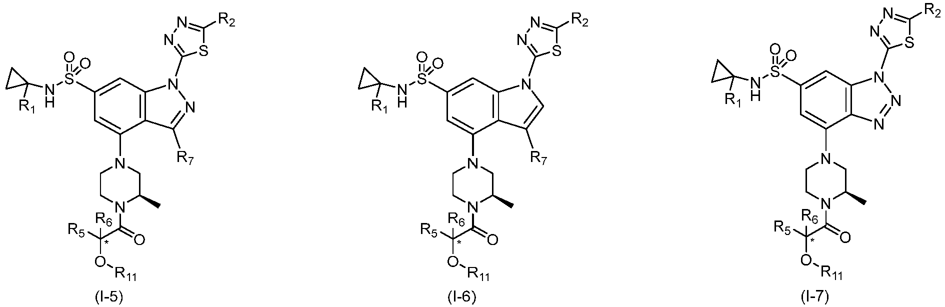 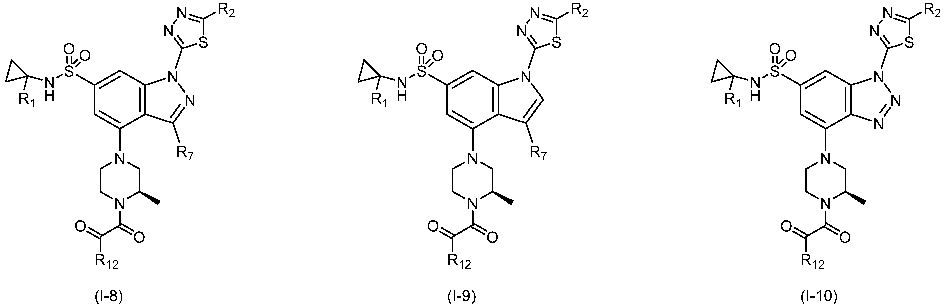 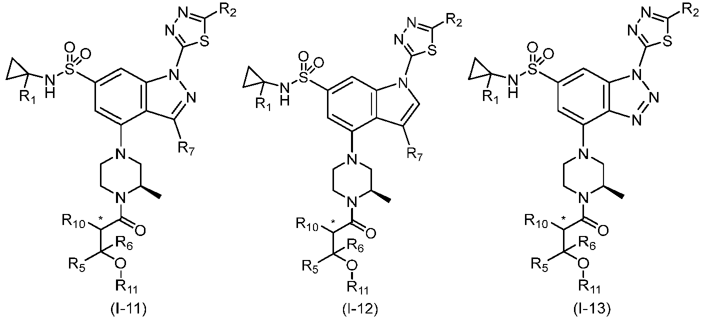
wherein the carbon atom marked with "*" indicates that when the carbon atom is a chiral carbon atom, it is independently in R configuration, S configuration, or a mixture thereof; R₁, R₂, R₇, R₅, R₆, R₁₁, and R₁₂ are as defined in any one of the above embodiments;
preferably, the formulas described above meet one or two of the following conditions:
I: R₂ is CHF₂; and
II: R₁ is CN.

In a certain embodiment of the present disclosure, the compound represented by formula (I) is any one of the following compounds:

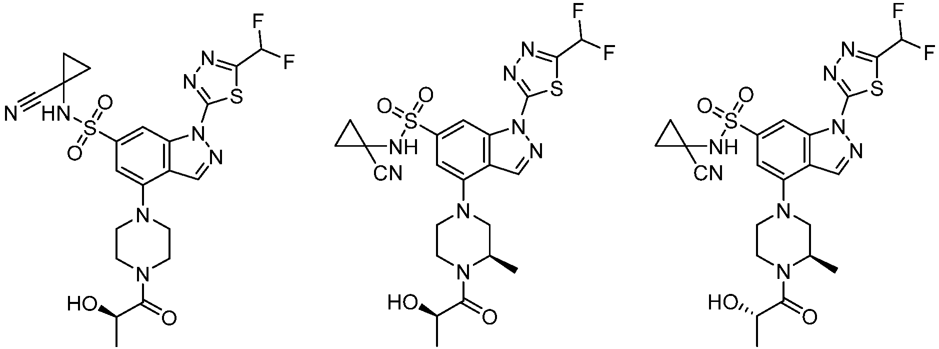

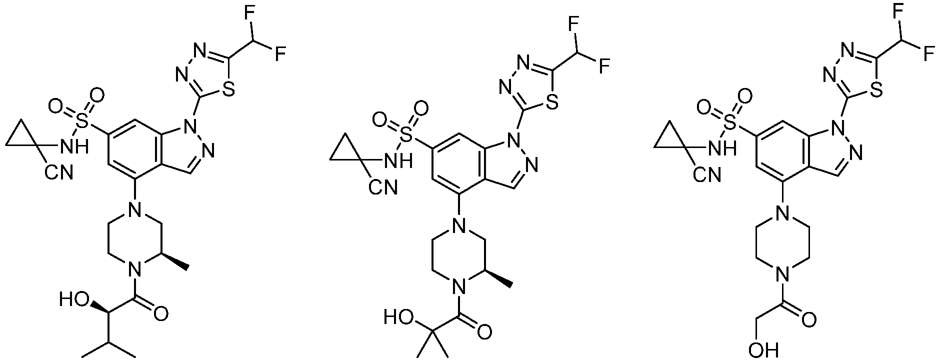

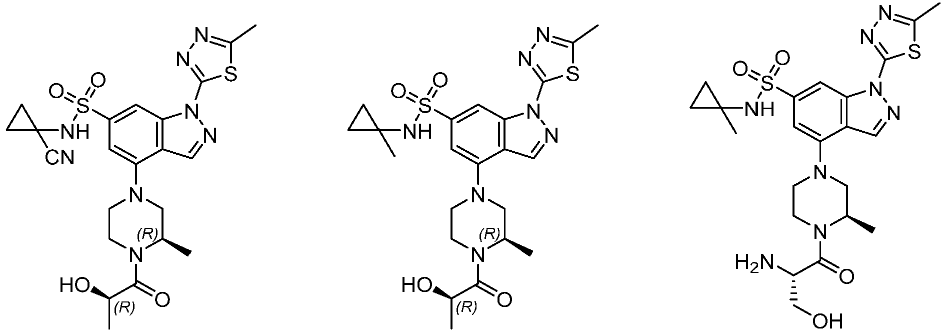

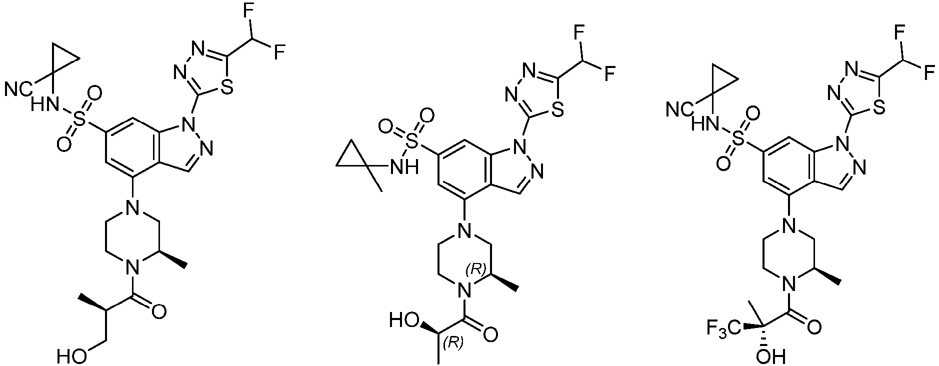

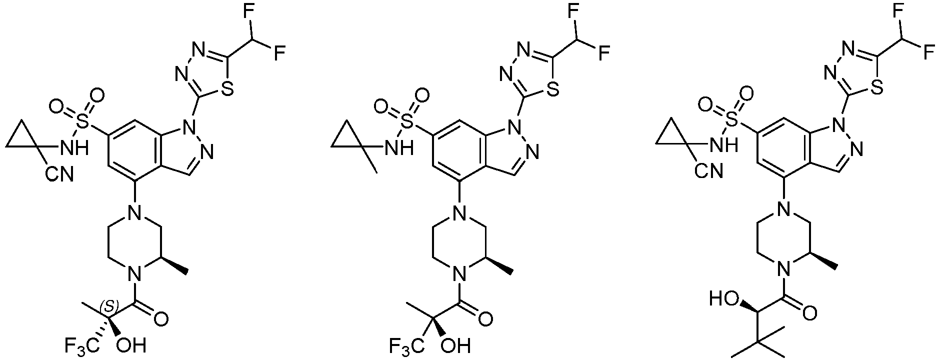

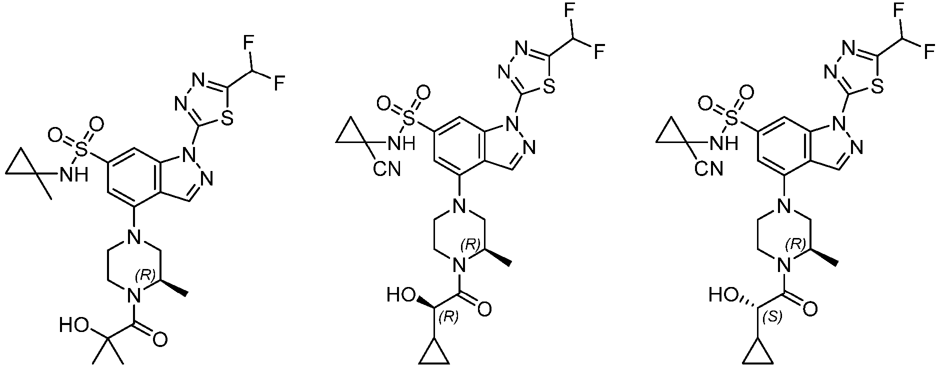

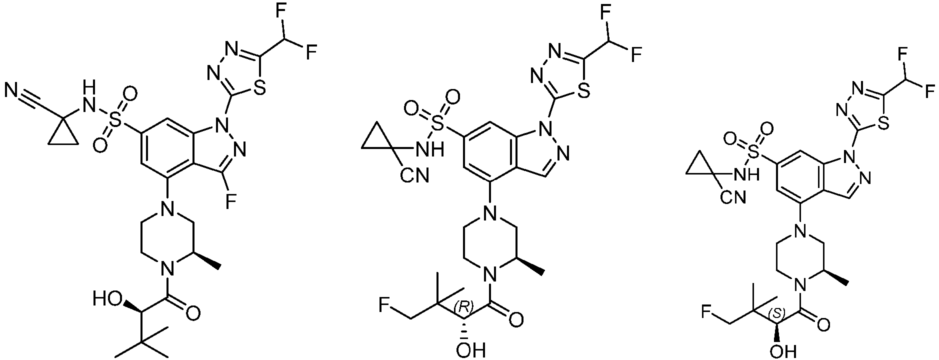

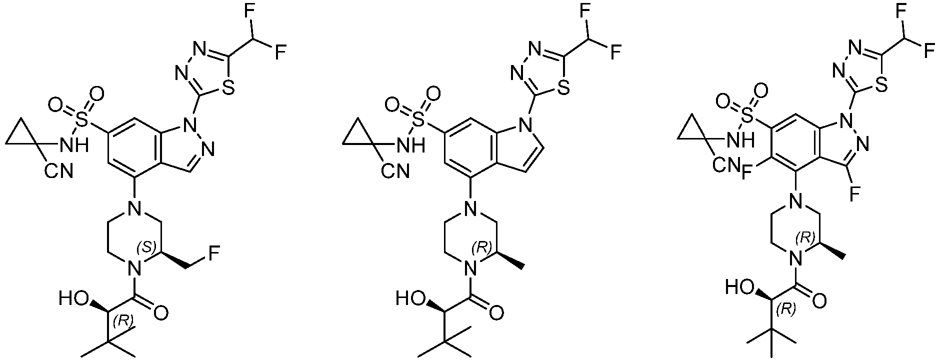

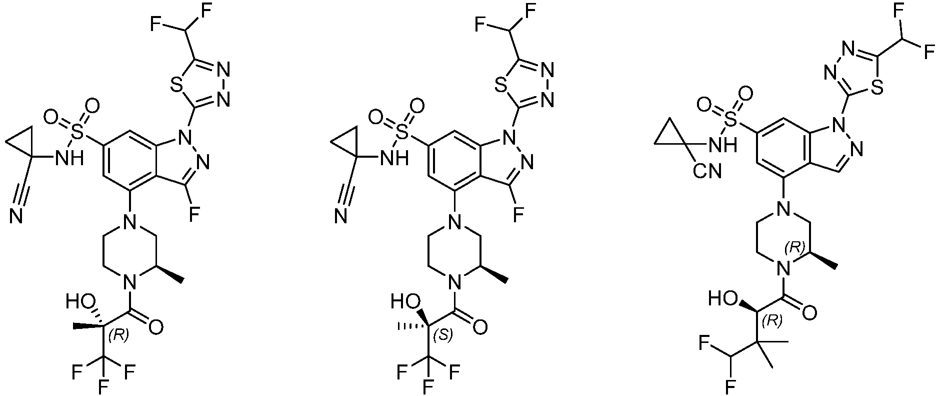

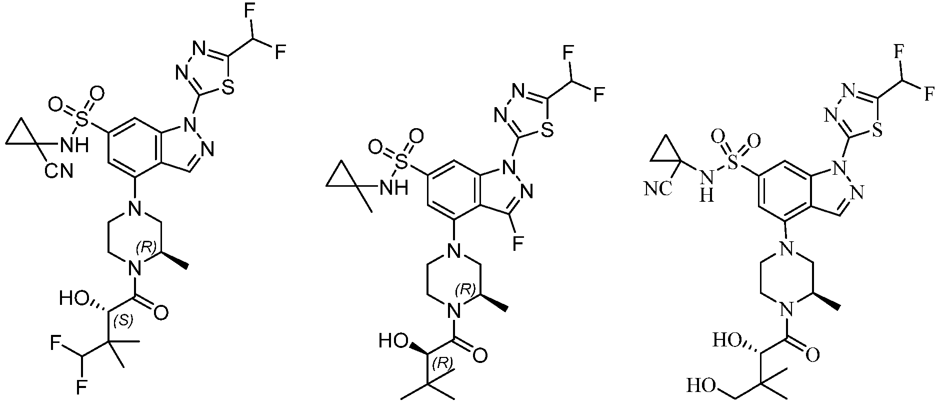

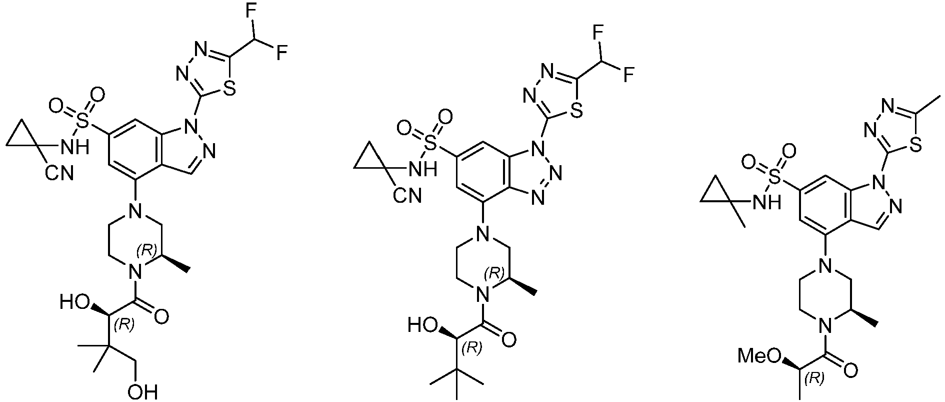

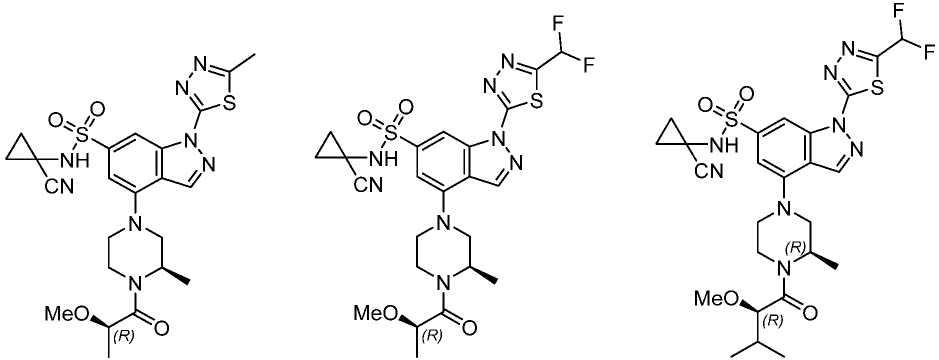

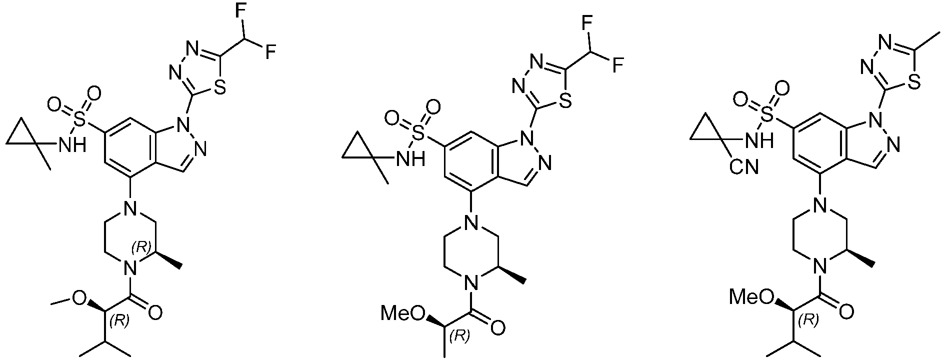

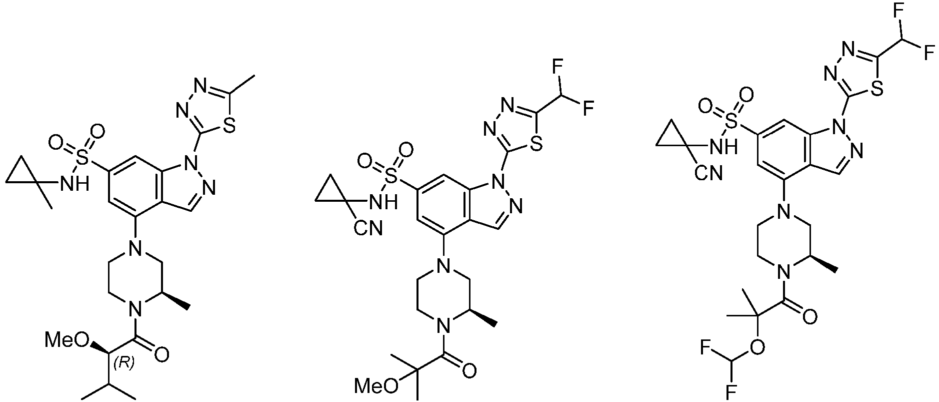

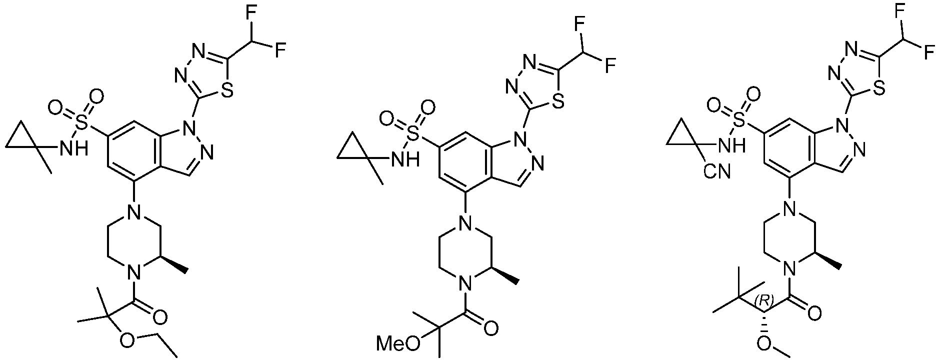

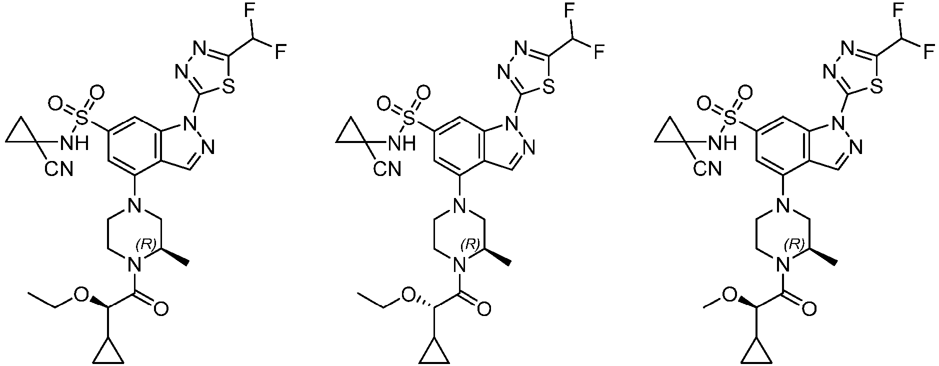

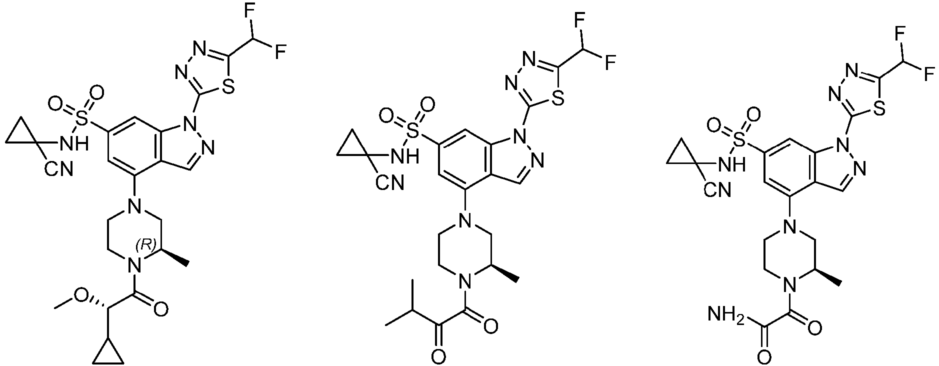

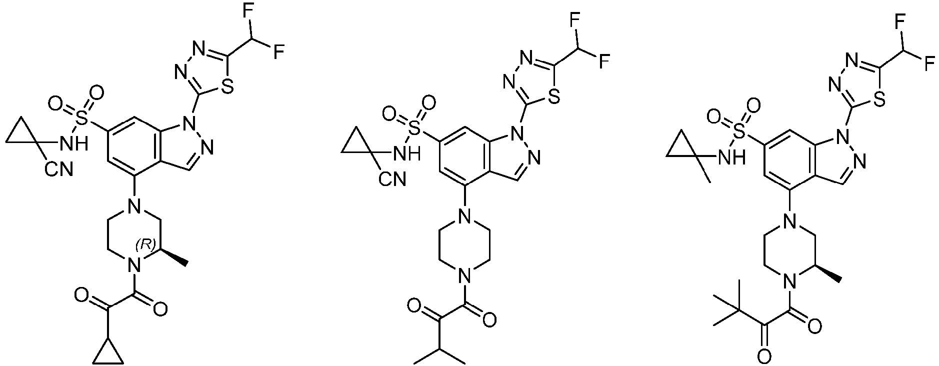

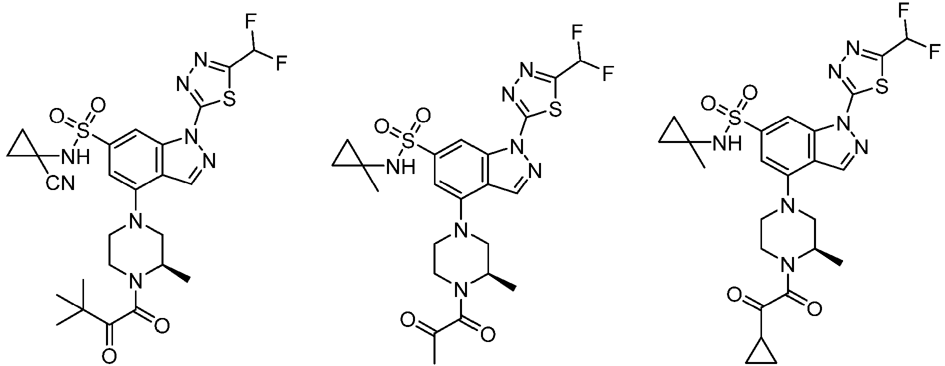

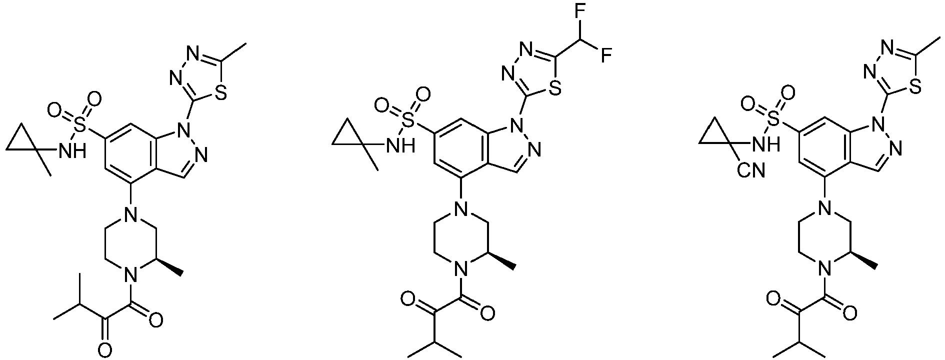

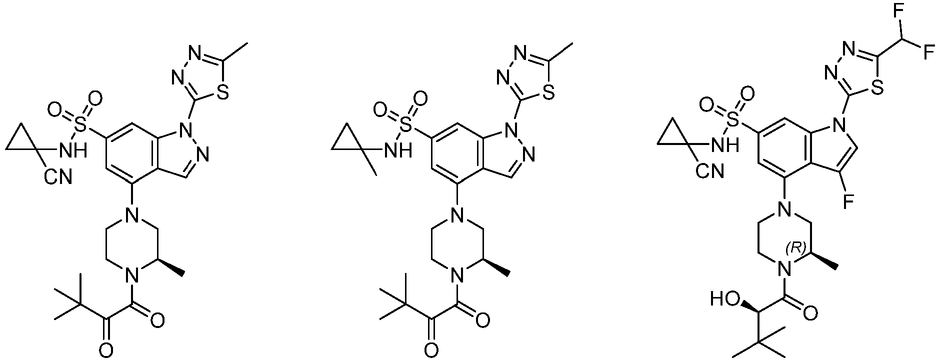

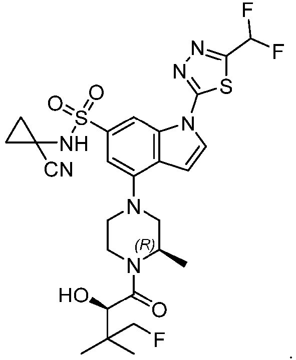

The present disclosure further provides a pharmaceutical composition, comprising a substance X and a pharmaceutically acceptable auxiliary material, wherein the substance X is the compound represented by formula (I), the pharmaceutically acceptable salt thereof, the solvate thereof, or the solvate of the pharmaceutically acceptable salt thereof as described in any one of the above embodiments.

The present disclosure further provides use of the substance X described above or the pharmaceutical composition described above in the preparation of a PARG inhibitor.

The present disclosure further provides use of the substance X described above or the pharmaceutical composition described above in the preparation of a medicament for treating a PARG-related disease, wherein preferably, the PARG-related disease is ovarian cancer, breast cancer, pancreatic cancer, prostate cancer, or gastric cancer.

The present disclosure further provides use of the substance X described above or the pharmaceutical composition described above in the preparation of a medicament for preventing and/or treating a cancer, wherein preferably, the cancer is ovarian cancer, breast cancer, pancreatic cancer, prostate cancer, or gastric cancer.

Unless otherwise defined, the terms used in the present disclosure have the following meanings:

As will be understood by those skilled in the art, 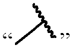 used in the structural formulas describing groups herein means that the corresponding groups are connected to other fragments and groups in the compound through this site, according to conventions used in the art.

As used herein, a substituent may be indicated with a single dash "-" indicating that the named substituent is connected to a parent moiety by a single bond.

The term "more" refers to 2, 3, or 4.

The term "halogen" refers to F, Cl, Br, or I.

The term "alkyl" refers to a liner or branched alkyl group with a specified number of carbon atoms (e.g., C₁-C₆). Examples of alkyl include methyl, ethyl, n-propyl, isopropyl, n-butyl, *tert-butyl,* isobutyl, sec-butyl, and similar alkyl groups thereof.

The term "alkynyl" refers to a liner or branched hydrocarbon group with one or more triple bonds and a specified number of carbon atoms (e.g., C₂-C₄). The one or more carbon-carbon triple bonds may be either internal or terminal.

The term "cycloalkyl" refers to a saturated cyclic group with a specified number of ring carbon atoms (e.g., C₃-C₆), the ring atoms consisting only of carbon atoms. Examples of cycloalkyl include, but are not limited to, cyclopropyl, cyclobutyl, cyclopentyl, cyclohexyl, and the like.

The term "heterocycloalkyl" refers to a saturated cyclic group with a specified number of ring atoms (e.g., 3-6 members), a specified number of heteroatoms (e.g., 1 or 2), and a specified heteroatom type (1, 2, or 3 of N, O, and S). Examples of heterocycloalkyl include, but are not limited to, oxiranyl, aziridinyl, oxetanyl, azetidinyl, tetrahydropyrrolyl, tetrahydrofuranyl, tetrahydropyranyl, piperidinyl, morpholinyl or piperazinyl, and the like.

When any variable (e.g., R₂₋₁) occurs multiple times within the definition of a compound, the definition of the variable at each position is independent of its definition at other positions, and their meanings are independent of one another and do not affect one another.

The term "pharmaceutically acceptable salt" refers to a salt prepared from the compound of the present disclosure with a relatively non-toxic, pharmaceutically acceptable acid or base. When the compound of the present disclosure contains a relatively acidic functional group, a base addition salt can be obtained by contacting a neutral form of the compound with a sufficient amount of a pharmaceutically acceptable base in a pure solution or a suitable inert solvent. When the compound of the present disclosure contains a relatively basic functional group, an acid addition salt can be obtained by contacting a neutral form of the compound with a sufficient amount of a pharmaceutically acceptable acid in a pure solution or a suitable inert solvent.

The term "solvate" refers to a substance formed by combining the compound of the present disclosure with a stoichiometric or non-stoichiometric amount of a solvent. Solvent molecules in the solvate may be present in an ordered or disordered arrangement.

The terms "pharmaceutically acceptable salt" and "solvate" in the "solvate of the pharmaceutically acceptable salt" as described above respectively refer to a substance prepared from the compound of the present disclosure with a relatively non-toxic, pharmaceutically acceptable acid or base, and a substance formed by combining the compound of the present disclosure with a stoichiometric or non-stoichiometric amount of a solvent.

The term "pharmaceutically acceptable auxiliary material" refers to excipients and additives used in the manufacture of a pharmaceutical product and in the formulation of a pharmaceutical formula, and refers to all substances, other than the active ingredient, contained in a pharmaceutical preparation. See Volume IV of Pharmacopoeia of The People's Republic of China (2020 Edition), or Handbook of Pharmaceutical Excipients (Raymond C Rowe, 2009 Sixth Edition).

In the present disclosure, the "inhibitor" can be used in a mammalian organism; it can also be used *in vitro,* mainly for experimental purposes, for example, used as a standard sample or a control sample to provide comparison, or prepared into a kit according to conventional methods in the art.

The term "treat", "treating", or "treatment" refers to therapeutic therapy. When specific conditions are involved, "treat", "treating" or "treatment" refers to: (1) relieving one or more biological manifestations of a disease or condition, (2) interfering with (a) one or more points in a biological cascade that causes or results in a condition or (b) one or more biological manifestations of a condition, (3) ameliorating one or more symptoms, effects or side effects associated with a condition, or ameliorating one or more symptoms, effects or side effects associated with a condition or treatment thereof, or (4) slowing the progression of a condition or one or more biological manifestations of a condition.

The term "prevent", "preventing", or "prevention" refers to a reduction in the risk of acquiring or developing a disease or disorder.

The positive and progressive effect of the present disclosure is as follows: The compound of the present disclosure has relatively good inhibitory activity against PARG.

### DETAILED DESCRIPTION

The following examples further illustrate the present disclosure, but the present disclosure is not limited thereto. Experimental procedures without specified conditions in the following examples were performed in accordance with conventional procedures and conditions, or in accordance with product instructions.

The reagents and starting materials used in the present disclosure are all commercially available or can be prepared according to WO2021055744A1, for example, by following References 4-5 and the intermediate methods for subsequent related compounds in WO2021055744A1.

### Example 1

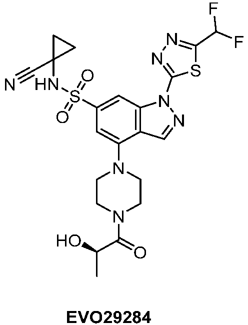

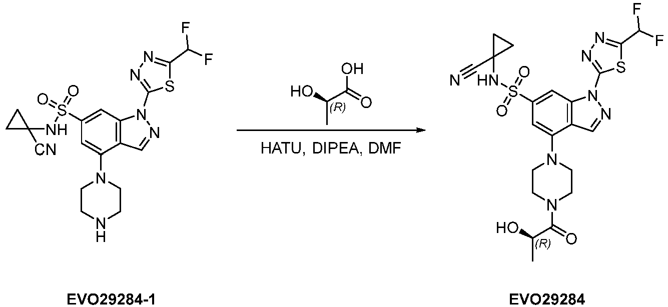

To a solution of compound EVO29284-1 (100.0 mg, 0.21 mmol) in N,N-dimethylformamide (5 mL) were added *O*-(7-azabenzotriazol-1-yl)-*N*,*N*,*N*',*N*'-tetramethyluronium hexafluorophosphate (158.0 mg, 0.42 mmol), *N*,*N*-diisopropylethylamine (134.0 mg, 1.04 mmol), and (R)-2-hydroxypropanoic acid (22.5 mg, 0.25 mmol) at room temperature, and the reaction mixture was stirred at 25 °C for 2 h. The reaction mixture was then separated and purified by reversed-phase column chromatography (methanol:water = 1:1) to give EVO29284 (50.0 mg, yield: 43.00%). LCMS (ESI) m/z = 553.0 [M+H]⁺. ¹HNMR (400 MHz, DMSO-d6) δ 8.98 (s, 1H), 8.51 (s, 1H), 7.61 (t, *J* = 53.1 Hz, 1H), 7.18 (s, 1H), 5.07 (d, *J* = 6.8 Hz, 1H), 4.51 (p, *J* = 6.5 Hz, 1H), 3.89 - 3.70 (m, 4H), 3.47 (d, *J* = 13.1 Hz, 4H), 1.45 (dd, *J* = 8.2, 5.5 Hz, 2H), 1.32 (dd, *J =* 8.3, 5.5 Hz, 2H), 1.25 (d, *J* = 6.5 Hz, 3H).

### Example 2

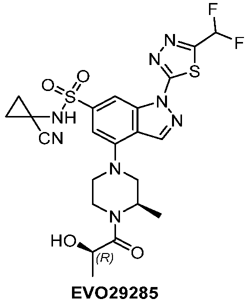

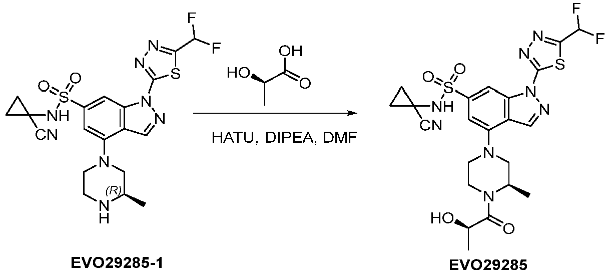

At room temperature, compound EVO292825-1 (120.0 mg, 0.24 mmol) was dissolved in *N,N-*dimethylformamide (5 mL), and then (*R*)-2-hydroxypropanoic acid (26.0 mg, 0.29 mmol), *O*-(7-azabenzotriazol-1-yl)-*N*,*N*,*N*',*N*'-tetramethyluronium hexafluorophosphate (184.0 mg, 0.49 mmol), *and N,N-*diisopropylethylamine (156.0 mg, 1.21 mmol) were added. The mixture was allowed to react at room temperature for 2 h. Water (20 mL) was added to quench the reaction, and the mixture was extracted with ethyl acetate (20 mL × 3). The organic phases were combined, dried over anhydrous sodium sulfate, filtered, and concentrated, and the residue was separated and purified by silica gel column chromatography (petroleum ether:ethyl acetate = 1:2) to give EVO29285 (30.0 mg, yield: 22.00%). LCMS (ESI) m/z = 567.1 [M+H]⁺. ¹HNMR (400 MHz, DMSO-d6) δ 9.33 (s, 1H), 8.87 (s, 1H), 8.48 (s, 1H), 7.60 (t,*J* = 53.1 Hz, 1H), 7.15 (s, 1H), 5.03 (s, 1H), 4.55 (d,*J* = 71.9 Hz, 2H, 4.09 - 3.62 (m, 3H), 3.29 (s, 3H), 1.47 - 1.43 (m, 2H), 1.32 (d,*J* = 2.6 Hz, 2H), 1.25 (d,*J* = 6.5 Hz, 5H).

### Example 3

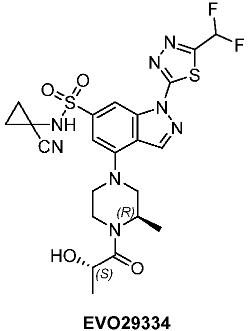

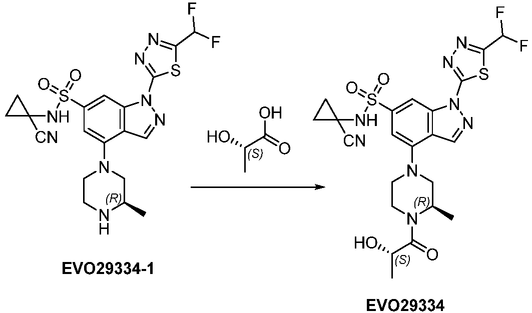

To a solution of compound EVO29334-1 (150.0 mg, 0.31 mmol) in N,N-dimethylformamide (5 mL) were added *O*-(7-azabenzotriazol-1-yl)-*N*,*N*,*N*',*N*'-tetramethyluronium hexafluorophosphate (178.0 mg, 0.47 mmol), *N*,*N*-diisopropylethylamine (201.0 mg, 1.56 mmol), and L-lactic acid (77.0 mg, 0.38 mmol, 90% in water) at room temperature, and the reaction mixture was stirred at 25 °C for 2 h. Water (30 mL) was added to the reaction mixture, and the resulting mixture was extracted with ethyl acetate (30 mL × 3). The organic phases were combined, dried over anhydrous sodium sulfate, and filtered, and the residue was separated and purified by silica gel column chromatography (petroleum ether:ethyl acetate = 1:3) to give EVO29334 (95.0 mg, yield: 56.0%). LCMS (ESI) m/z = 567.1 [M+H]⁺. ¹H NMR (400 MHz, DMSO-*d*₆) δ 9.34 (s, 1H), 8.86 (s, 1H), 8.48 (d, *J =* 10.2 Hz, 1H), 7.60 (t, *J =* 53.1 Hz, 1H), 7.15 (d, *J =* 10.1 Hz, 1H), 4.97 (d, *J =* 7.2 Hz, 1H), 4.76 - 4.27 (m, 2H), 4.08 - 3.61 (m, 3H), 3.14 (t, *J* = 46.9 Hz, 2H), 1.44 (d, *J* = 2.7 Hz, 3H), 1.34 - 1.26 (m, 4H), 1.22 (dd, *J =* 11.1, 5.7 Hz, 3H).

### Example 4

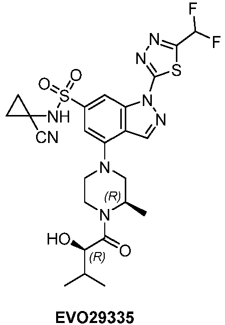

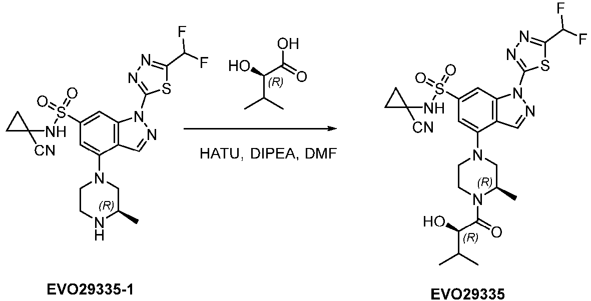

### Step 1:

To a solution of compound EVO29335-1 (100.0 mg, 0.20 mmol) in N,N-dimethylformamide (5 mL) were added *O*-(7-azabenzotriazol-1-yl)-*N*,*N*,*N*',*N*'-tetramethyluronium hexafluorophosphate (115.0 mg, 0.30 mmol), *N*,*N*-diisopropylethylamine (130.0 mg, 1.01 mmol), and (2R)-3-methyl-2-hydroxybutyric acid (29.0 mg, 0.24 mmol) at room temperature, and the reaction mixture was stirred at 25 °C for 2 h. The reaction mixture was then separated and purified by reversed-phase column chromatography (methanol:water = 1:1) and silica gel column chromatography (petroleum ether:ethyl acetate = 1:1) to give EVO29335 (25.0 mg, yield: 20.8%). LCMS (ESI) m/z = 595.1 [M+H]⁺. ¹HNMR (400 MHz, DMSO-*d*₆) δ 8.86 (s, 1H), 8.48 (s, 1H), 7.61 (t, *J = 53.1* Hz, 1H), 7.16 (s, 1H), 4.98 (d, *J =* 79.1 Hz, 1H), 4.53 (t, *J =* 59.4 Hz, 1H), 4.15 - 3.98 (m, 2H), 3.91 - 3.61 (m, 3H), 3.18 (d, *J = 8.3* Hz, 1H), 2.06 - 1.85 (m, 1H), 1.51 - 1.40 (m, 3H), 1.31 (t, *J =* 6.5 Hz, 4H), 1.24 (s, 1H), 0.89 (t, *J =* 7.4 Hz, 6H).

### Example 5

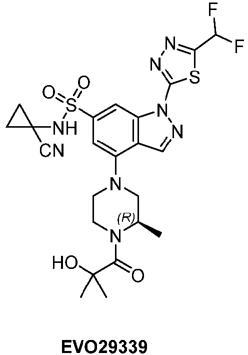

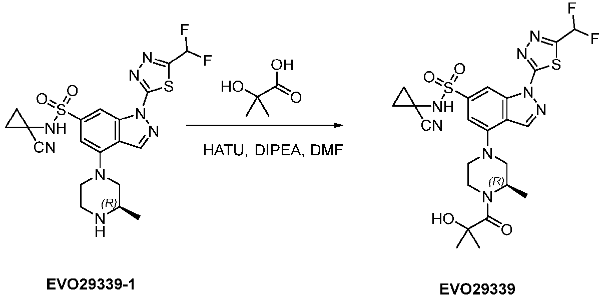

### Step 1:

To a solution of compound EVO29339-1 (100.0 mg, 0.20 mmol) in N,N-dimethylformamide (5 mL) were added *O*-(7-azabenzotriazol-1-yl)-*N*,*N*,*N*',*N*'-tetramethyluronium hexafluorophosphate (115.0 mg, 0.30 mmol), *N*,*N*-diisopropylethylamine (130.0 mg, 1.01 mmol), and 2-methyl-2-hydroxypropanoic acid (25.0 mg, 0.24 mmol) at room temperature, and the reaction mixture was stirred at 25 °C for 2 h. The reaction mixture was then separated and purified by reversed-phase column chromatography (methanol:water = 1:1) and silica gel column chromatography (petroleum ether:ethyl acetate = 1:1) to give EVO29339 (24.4 mg, yield: 20.8%). LCMS (ESI) m/z = 581.0 [M+H]⁺. ¹H NMR (400 MHz, DMSO-*d*₆) δ 9.33 (s, 1H), 8.87 (s, 1H), 8.46 (s, 1H), 7.61 (t, *J =* 53.1 Hz, 1H), 7.15 (s, 1H), 5.51 (s, 1H), 4.75 (d, *J* = 54.5 Hz, 1H), 3.84 (d, *J =* 12.1 Hz, 1H), 3.73 (d, *J =* 12.3 Hz, 1H), 3.34 (s, 1H), 3.10 (d, *J* = 119.6 Hz, 3H), 1.55 - 1.42 (m, 3H), 1.36 (d, *J =* 7.1 Hz, 6H), 1.31 (dd, *J =* 8.0, 5.3 Hz, 4H).

### Example 6

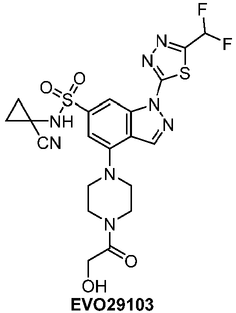

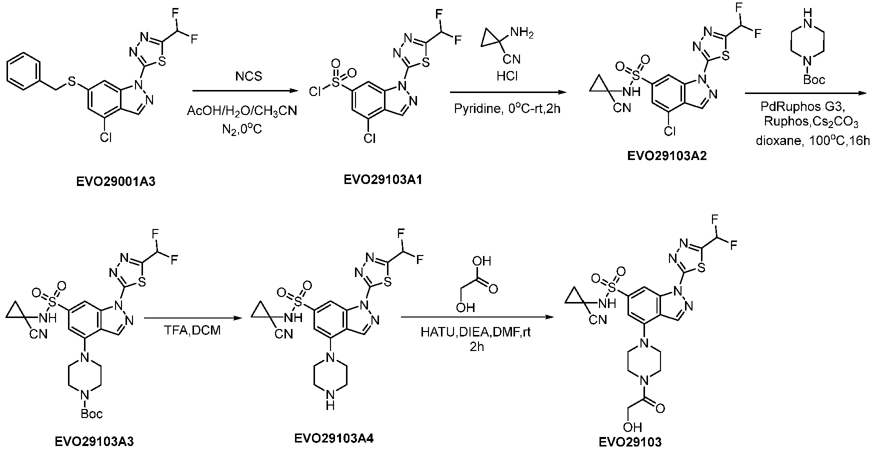

### Step 1:

To a flask were added compound EVO29001A3 (2.24 g, 5.49 mmol), acetic acid (20 mL), and water (5 mL) at room temperature. Under a nitrogen atmosphere, the mixture was cooled to 0 °C, and *N-*chlorosuccinimide (879.7 mg, 6.59 mmol) was added. Then, the mixture was stirred at 0-25 °C for 12 h. TLC analysis showed no starting material remaining. Water (60 mL) was added to the reaction mixture, and the aqueous phase was extracted with ethyl acetate (75 mL × 3). The organic phases were combined and dried over anhydrous sodium sulfate, and the filtrate was concentrated under reduced pressure to give a crude product as a solid, which was then separated and purified by silica gel column chromatography (ethyl acetate/petroleum ether = 0-50%) to give EVO29103A1 as a pale yellow solid (1.70 g, yield: 80.00%). LCMS showed no response.

### Step 2:

1-Amino-1-cyclopropanecarbonitrile hydrochloride (2.31 g, 19.48 mmol) and anhydrous pyridine (20 mL) were stirred in a flask under an ice-water bath, and compound EVO29103A1 (2.50 g, 6.49 mmol) was added to the flask. The mixture was allowed to react at room temperature for 2 h. LCMS analysis showed the completion of the reaction. The reaction mixture was concentrated to give a yellow oil, which was then separated and purified by silica gel column chromatography (ethyl acetate/petroleum ether = 0-50%) to give EVO29103A2 as a yellow solid (1.55 g, yield: 51.73%). LCMS (ESI) m/z = 431.0 [M+H]⁺.

### Step 3:

To a reaction flask were added EVO29103A2 (1.25 g, 2.90 mmol), methanesulfonato(2-dicyclohexylphosphino-2',6'-diisopropoxy-1,1'-biphenyl)(2-amino-1,1'-biphenyl-2-yl)palladium(II) (0.97 g, 1.16 mmol), 2-dicyclohexylphosphino-2',6'-diisopropoxy-1,1'-biphenyl (1.08 g, 2.32 mmol), cesium carbonate (8.72 g, 2.84 mmol), and 1,4-dioxane (45 mL). Under a nitrogen atmosphere, the mixture was stirred under an oil bath at 90 °C for 3 h. The reaction mixture was filtered through filter paper and washed with ethyl acetate (10 mL × 3), and the filtrate was concentrated under reduced pressure. The oily residue was separated and purified by silica gel column chromatography (methanol/dichloromethane = 0-25%) to give EVO29103A3 as a yellow foamy substance (1.20 g, yield: 71.00%). LCMS (ESI) m/z = 581.0 [M+H]⁺.

### Step 4:

To a reaction flask were added EVO29103A3 (200 mg, 0.34 mmol) and ethyl acetate (1 mL). With stirring under an ice-water bath, a solution of hydrogen chloride in ethyl acetate (2 mL, 4 M) was added, and the mixture was warmed to room temperature and stirred for 2 h. Reaction analysis showed the completion of the reaction. The reaction mixture was concentrated under reduced pressure to give a crude product of EVO29103A4, which was directly used in the next step. LCMS (ESI) m/z = 481.0 [M+H]⁺.

### Step 5:

To a reaction flask were added EVO29103A4 (90 mg, 0.19 mmol), glycolic acid (20 mg, 0.23 mmol), *O*-(7-azabenzotriazol-1-yl)-*N*,*N*,*N*',*N*'-tetramethyluronium hexafluorophosphate (100 mg, 0.38 mmol), *N*,*N*-diisopropylethylamine (73 mg, 0.57 mmol), and *N*,*N*-dimethylformamide (3 mL). The mixture was stirred at room temperature for 2 h. LCMS analysis showed the completion of the reaction. Water (30 mL) was added to the reaction mixture, and the resulting mixture was extracted with ethyl acetate (30 mL × 3). The organic phase was washed with water, dried, and concentrated to dryness by rotary evaporation, and the residue was separated and purified by silica gel column chromatography (methanol/dichloromethane = 0-10%) to give EVO29103 as a white solid (8.6 mg, yield: 9.30%). LCMS (ESI) m/z = 538.8 [M+H]⁺. ¹H NMR (DMSO-*d*₆, 400 MHz): δ 9.39 (s, 1H), 8.95 (s, 1H), 8.49 (s, 1H), 7.61 (t, *J* = 53.1 Hz, 1H), 7.16 (s, 1H), 4.71 (t, *J =* 5.5 Hz, 1H), 4.18 (d, *J* = 5.5 Hz, 2H), 3.70 (d, *J* = 42.7 Hz, 4H), 3.46 (s, 4H), 2.03 - 1.95 (m, 2H), 1.45 (m, 2H).

### Example 7

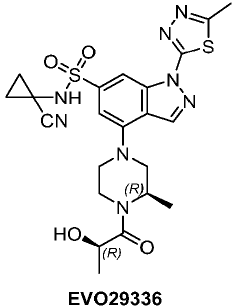

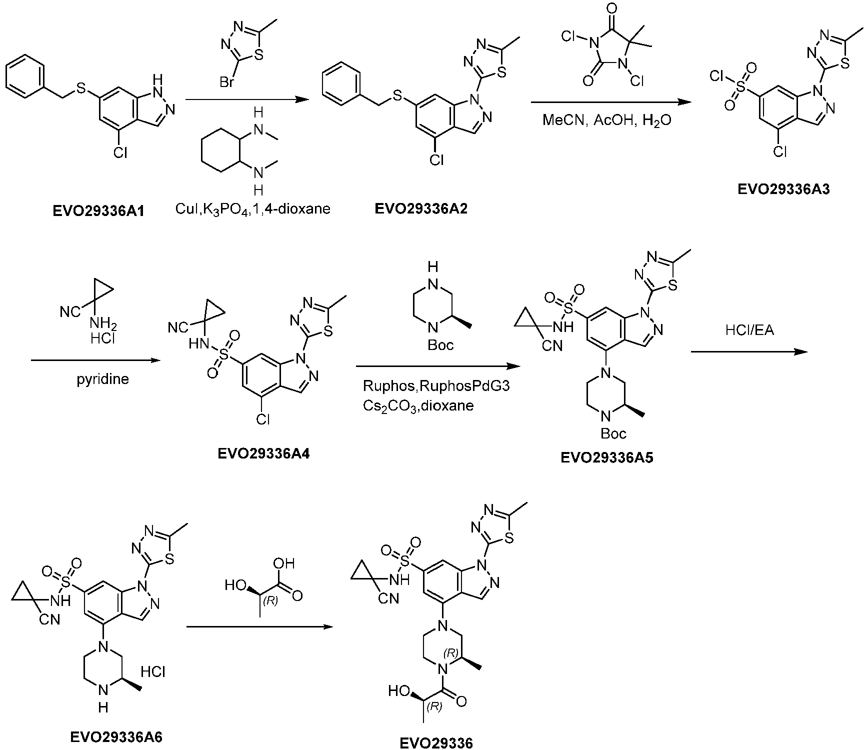

### Step 1:

EVO29336A1 (5.00 g, 20 mmol) was dissolved in 1,2-dioxane (60 mL), and then 2-bromo-5-methyl-1,3,4-thiadiazole (11.00 g, 60.07 mmol), N,N-dimethylcyclohexanediamine (2.84 g, 20 mmol), copper(I) iodide (1.90 g, 10.00 mmol), potassium phosphate (12.90 g, 60.07 mmol) were added. Under a nitrogen atmosphere, the reaction mixture was stirred at 90 °C for 35 h and then cooled to room temperature. An ammonium hydroxide solution (150 mL) was added, and the mixture was stirred at room temperature for 30 min and filtered. The filtrate was extracted with ethyl acetate (100 mL × 3). The organic phases were combined, dried over anhydrous sodium sulfate, and concentrated, and the residue was slurried with methanol (50 mL) to give EVO29336A2 (3.00 g, yield: 40.00%). LCMS (ESI) m/z = 372.7 [M+H]⁺.

### Step 2:

EVO29336A2 (7.20 g, 19.35 mmol) was dissolved in acetonitrile (40 mL) at 0 °C, and then acetic acid (4.5 mL) and water (1.7 mL) were added. 1,3-Dichloro-5,5-dimethylhydantoin (5.34 g, 34.83 mmol) was added in portions at 0 °C. The reaction mixture was stirred at 0 °C for 1 h, and the filtrate was concentrated to dryness by rotary evaporation to give EVO29336A3 as a pale yellow solid (6.7 g, based on theoretical yield), which was directly used in the next step without purification. LCMS showed no response.

### Step 3:

To EVO29336A3 (2.3 g, 6.72 mmol) dissolved in pyridine (67 mL) was added 1-cyanocyclopropylamine hydrochloride (1.60 g, 13.40 mmol) in portions at 0 °C, and the reaction mixture was stirred at 0 °C for 1 h and concentrated to remove most of the pyridine, followed by addition of ethyl acetate (100 mL). Then, 0.5 M hydrochloric acid was added at 0 °C to adjust the pH to 2, followed by layer separation. The aqueous phase was extracted with ethyl acetate (50 mL). The organic phases were combined, dried over anhydrous sodium sulfate, filtered, and concentrated, and the concentrate was slurried with methanol (50 mL) to give EVO29336A4 as a yellow solid (1.1 g, yield: 42.30%). LCMS (ESI) m/z = 395.0 [M+H]⁺.

### Step 4:

To EVO29336A4 (0.86 g, 2.18 mmol) dissolved in 1,4-dioxane (20 mL) were added (R)-1-N-Boc-2-methylpiperidine (1.09 g, 5.46 mmol), 2-dicyclohexylphosphino-2',6'-diisopropoxy-1,1'-biphenyl (814 mg, 1.74 mmol), methanesulfonato(2-dicyclohexylphosphino-2',6'-diisopropoxy-1,1'-biphenyl)(2-amino-1,1'-biphenyl-2-yl)palladium(II) (729 mg, 0.87 mmol), and cesium carbonate (3.55 g, 10.90 mmol). The mixture was stirred at 90 °C for 1 h under a nitrogen atmosphere, then cooled to room temperature, and directly separated and purified by silica gel column chromatography (petroleum ether/ethyl acetate = 40:1) to give EVO29336A5 as a yellow foamy solid (1.126 g, yield: 92.40%). LCMS (ESI) m/z = 559.0 [M+H]⁺.

### Step 5:

To EVO29336A5 (1.16 g, 2.07 mmol) dissolved in methanol (1.5 mL) was added a solution of hydrogen chloride in methanol (6 mL), and the reaction mixture was stirred at room temperature for 2 h and directly concentrated to give EVO29336A6 as a yellow foamy solid (0.9 g, yield: 94.80%). LCMS (ESI) m/z = 459.0 [M+H]⁺.

### Step 6:

To EVO29336A6 (90 mg, 0.20 mmol) dissolved in N,N-dimethylformamide (3 mL) were added D-lactic acid (36 mg, 0.39 mmol) and N,N-diisopropylethylamine (126.7 mg, 0.98 mmol), and then O-(7-azabenzotriazol-1-yl)-*N*,*N*,*N*',*N*'-tetramethyluronium hexafluorophosphate (138 mg, 0.36 mmol) was added in portions. The reaction mixture was stirred at room temperature for 2 h, and then directly separated and purified by silica gel column chromatography (dichloromethane/methanol = 80:1) to give EVO29336 as an off-white solid (25 g, yield: 24.00%). LCMS (ESI) m/z = 531.1 [M+H]⁺. ¹H NMR(DMSO-*d*₆, 400 MHz): δ 9.29 (s, 1H), 8.76 (s, 1H), 8.48 (s, 1H), 7.10 (s, 1H), 5.11 (d, *J*=59.1Hz, 1H), 4.65 (s, 1H), 4.54 - 4.42 (m, 1H), 4.17 (d, *J*=126.8 Hz, 1H), 3.83 (d, *J*=11.6 Hz, 1H), 3.72 (d, *J*=12.3 Hz, 1H), 3.30 - 3.25 (m, 2H), 3.07 (d, *J*=83.9 Hz, 1H), 2.76 (s, 3H), 1.44 (d, *J*=2.7 Hz, 1H), 1.31 (dd, *J*=5.0Hz, 5H), 1.25 (s, 1H).

### Example 8

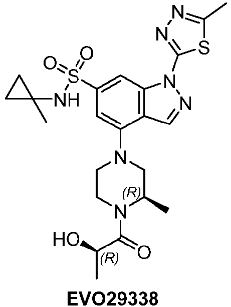

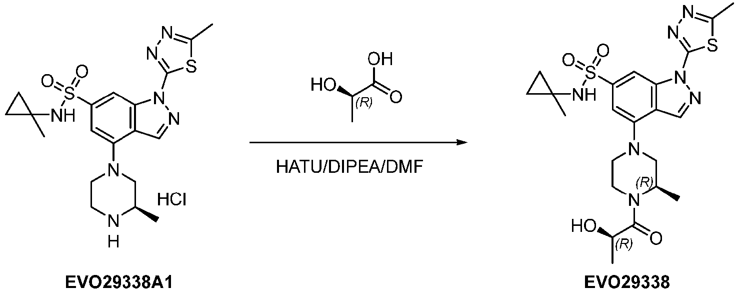

### Step 1:

To EVO29338A1 (90 mg, 0.20 mmol), *D*-lactic acid (36 mg, 0.39 mmol) and *N,N-*diisopropylethylamine (126.7 mg, 0.98 mmol) dissolved in *N*,*N*-dimethylformamide (3 mL) was added O-(7-azabenzotriazol-1-yl)-*N*,*N*,*N*',*N*'-tetramethyluronium hexafluorophosphate (138 mg, 0.36 mmol) in portions, and the reaction mixture was stirred at room temperature for 1.5 h. Water (70 mL) was added, and the mixture was extracted with ethyl acetate (50 mL × 2). The organic phases were combined, dried over anhydrous sodium sulfate, filtered, and concentrated, and the residue was separated and purified by silica gel column chromatography (dichloromethane/methanol = 70:1) to give EVO29338 as an off-white solid (28.7 mg, yield: 27.46%). LCMS (ESI) m/z = 520.2 [M+H]⁺. ¹H NMR(DMSO-*d*₆, 400 MHz): δ 8.72 (s, 1H), 8.44 (s, 1H), 8.27 (s, 1H), 7.10 (s, 1H), 5.03 (m,1H), 4.65 (s, 1H), 4.50 - 4.42 (m, 1H), 4.43 - 3.85 (m, 1H), 3.83 - 3.76 (m, 1H), 3.74 - 3.66 (m, 1H), 3.32 - 3.29 (m, 1H), 3.27 - 3.22 (m, 1H), 2.75 (s,1H), 1.51 - 1.46 (m, 2H), 1.45 - 1.38 (m, 2H), 1.44 - 1.23 (m, 4H), 1.07 (s, 3H), 0.67 - 0.61 (m, 2H), 0.42 - 0.39 (m, 2H).

### Example 9

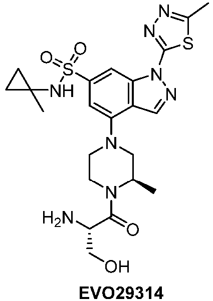

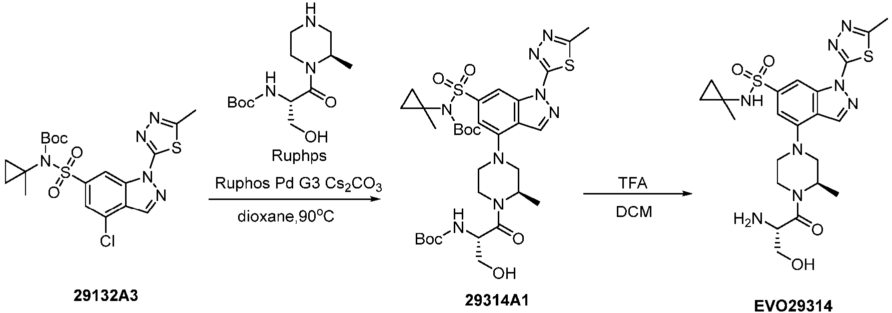

### Step 1:

To a solution of compound 29132A3 (0.20 g, 0.41 mmol) in 1,4-dioxane (8 mL) were added methanesulfonato(2-dicyclohexylphosphino-2',6'-diisopropoxy-1,1'-biphenyl)(2-amino-1,1'-biphenyl-2-yl)palladium(II) (0.07 g, 0.08 mmol), 2-dicyclohexylphosphino-2',6'-diisopropoxy-1,1'-biphenyl (0.075 g, 0.16 mmol), *tert-butyl* ((*S*)-3-hydroxy-1-((*R*)-2-methylpiperazin-1-yl)-1-oxopropan-2-yl)carbamate (0.24 g, 0.82 mmol) and cesium carbonate (0.67 g, 2.07 mmol) at room temperature, and the reaction mixture was stirred at 90 °C for 3 h. The reaction mixture was concentrated to remove the solvent, and the residue was separated and purified by silica gel column chromatography (ethyl acetate/petroleum ether) to give 29314A1 as a pale yellow solid (82.21 mg, yield: 27.00%). LCMS (ESI) m/z = 735.3 [M+H]⁺.

### Step 2:

To a solution of compound 29314A1 (82.21 mg, 0.11 mmol) in dichloromethane (1 mL) was added trifluoroacetic acid (0.20 mL) at room temperature, and the reaction mixture was stirred at 25 °C for 1 h. The reaction mixture was concentrated to remove the solvent, and the residue was dissolved in ethyl acetate/sodium bicarbonate solution, followed by liquid separation. The organic phase was washed once with saturated brine, dried over anhydrous sodium sulfate, and concentrated to give a crude product, which was then subjected to preparative reversed-phase chromatography to give EVO29314 as a pale yellow solid (16.30 mg, yield: 23.00%). LCMS (ESI) m/z = 535.2 [M+H]⁺. ¹H NMR (DMSO-*d*₆, 400 MHz): δ 8.72 (d, *J =* 4.5 Hz, 1H), 8.44 (d, *J =* 5.9 Hz, 1H), 7.12 - 7.05 (m, 1H), 4.79 - 4.65 (m, 1H), 4.50 (s, 1H), 4.33 (d, *J* = 14.2 Hz, 1H), 4.05 (d, *J =* 13.7 Hz, 1H), 3.89 - 3.61 (m, 4H), 3.55 - 3.39 (m, 2H), 3.25 (dd, *J =* 12.5, 3.6 Hz, 2H), 3.17 (d, *J =* 10.9 Hz, 1H), 2.95 (dd, *J =* 21.9, 11.7 Hz, 1H), 2.75 (s, 3H), 1.70 (s, 1H), 1.43 (d, *J =* 6.5 Hz, 1H), 1.28 (d, *J =* 6.9 Hz, 2H), 1.07 (s, 3H), 0.65 (q, *J* = 3.1 Hz, 2H), 0.43 - 0.36 (m, 2H).

### Example 10

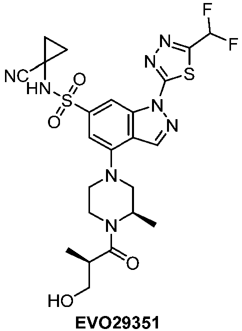

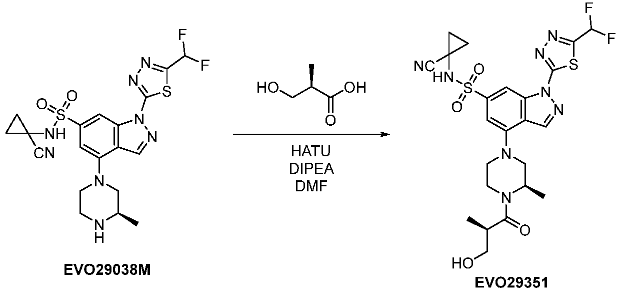

### Step 1:

EVO29038M (50.0 mg, 101.10 µmol) and (R)-2-hydroxymethylpropanoic acid (11.6 mg, 111.21 µmol) were dissolved in 5 mL of N,N-dimethylformamide, and then N,N-diisopropylethylamine (26.1 mg, 202.21 µmol) and *O*-(7-azabenzotriazol-1-yl)-*N*,*N*,*N*',*N*'-tetramethyluronium hexafluorophosphate (42.5 mg, 111.21 µmol) were added. The mixture was allowed to react at room temperature for 1.5 h. Water (10 mL) was added, and the mixture was extracted with ethyl acetate (10 mL × 3). The organic phases were combined, washed with saturated brine (20 mL), evaporated to dryness under reduced pressure, and subjected to preparative medium-pressure chromatography (petroleum ether/ethyl acetate = 2:1). The fractions were evaporated to dryness to give 100.0 mg of a yellow oil, which was then separated and purified by preparative high performance liquid chromatography to give EVO29351 as a yellowish-green solid (6.2 mg, yield: 11.00%). LCMS (ESI) m/z = 580.9 [M+H]⁺. ¹H NMR (DMSO-*d*₆, 400 MHz): δ 9.33 (s, 1H), 8.86 (s, 1H), 8.47 (s, 1H), 7.60 (t, *J =* 53.1 Hz, 1H), 7.13 (s, 1H), 4.74 (s, 1H), 4.65 (s, 1H), 4.51 - 4.23 (m, 1H), 4.03 (d, *J* = 13.2 Hz, 1H), 3.85 (d, *J = 9.1* Hz, 1H), 3.77 - 3.46 (m, 3H), 3.25 - 2.82 (m, 3H), 1.49 - 1.21 (m, 7H), 0.99 (s, 3H).

### Example 11

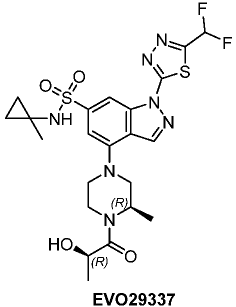

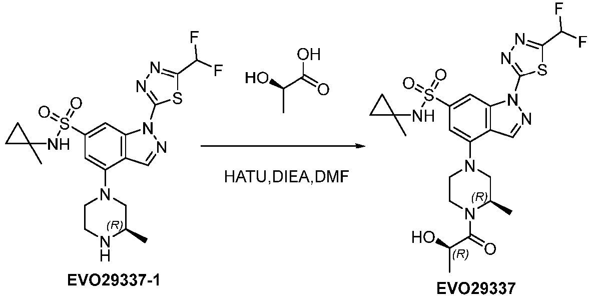

### Step 1:

To a solution of compound EVO29337-1 (100.0 mg, 0.21 mmol) in N,N-dimethylformamide (2 mL) were added *O*-(7-azabenzotriazol-1-yl)-*N*,*N*,*N*',*N*'-tetramethyluronium hexafluorophosphate (87.0 mg, 0.31 mmol), N,N-diisopropylethylamine (80.0 mg, 0.62 mmol), and D-lactic acid (22.0 mg, 0.25 mmol) at room temperature, and the reaction mixture was stirred at 25 °C for 2 h. The reaction mixture was concentrated to dryness by rotary evaporation to remove the solvent. An appropriate amount of water was added, and the mixture was extracted with ethyl acetate (3 × 20 mL). The organic phases were combined, dried over anhydrous sodium sulfate, and separated and purified by silica gel column chromatography (petroleum ether/ethyl acetate = 1:1) to give EVO29337 as a yellow solid (29 mg, yield: 25%). LCMS (ESI) m/z = 556.6 [M+H]⁺. ¹H NMR (400 MHz, DMSO-*d*₆) δ 9.30 (s, 1H), 8.75 (d, *J =* 2.7 Hz, 1H), 8.52 (d, *J* = 8.4 Hz, 1H), 7.13 (d, *J =* 8.7 Hz, 1H), 4.74 - 4.27 (m, 1H), 3.95 - 3.83 (m, 1H), 3.82 - 3.64 (m, 2H), 3.56 - 3.37 (m, 1H), 3.22 (dd, *J =* 11.9, 3.3 Hz, 1H), 3.15 - 3.00 (m, 2H), 2.76 (s, 3H), 1.48 - 1.43 (m, 3H), 1.39 (d, *J =* 6.7 Hz, 2H), 1.31 (dd, *J =* 8.1, 5.1 Hz, 2H), 1.14 (*dd, J* = 9.9, 4.2 Hz, 6H).

### Example 12

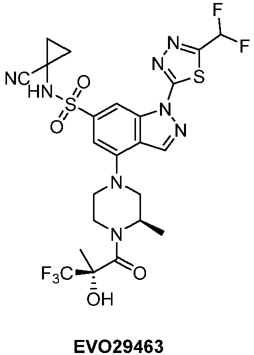

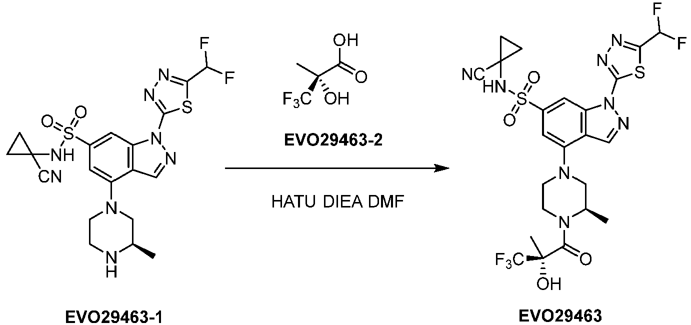

### Step 1:

At room temperature, compound EVO29463-1 (100.0 mg, 0.20 mmol) and compound EVO29463-2 (46.9 mg, 0.30 mmol) were dissolved in N,N-dimethylformamide (3 mL), and then O-(7-azabenzotriazol-1-yl)-*N*,*N*,*N*',*N*'-tetramethyluronium hexafluorophosphate (153.9 mg, 0.40 mmol), and *N,N-*diisopropylethylamine (78.3 mg, 0.61 mmol) were added. The mixture was allowed to react at room temperature for 2 h. Water (20 mL) was added to the reaction mixture to quench the reaction, and the resulting mixture was extracted with ethyl acetate (10 mL × 3). The organic phases were combined, washed with water (10 mL), then further washed once with saturated brine (10 mL), and concentrated, and the residue was separated and purified by silica gel column chromatography (ethyl acetate:petroleum ether = 3:1) to give a yellow solid, which was then separated and purified by reversed-phase column chromatography (water (0.1% formic acid):acetonitrile = 1:1), and lyophilized to give product EVO29463 as a yellow solid (15 mg, yield: 12%). LCMS (ESI) m/z = 635.1 [M+H]⁺. ¹H NMR (400 MHz, DMSO-*d*₆) δ 8.84 (s, 1H), 8.45 (s, 1H), 7.60 (t, *J* = 53.1 Hz, 1H), 7.13 (s, 2H), 5.46 - 5.03 (m, 1H), 4.69 (s, 2H), 3.78 (dd, *J =* 34.4, 13.3 Hz, 3H), 3.22 - 3.14 (m, 3H), 1.58 (d, *J = 20.3* Hz, 3H), 1.38 - 1.29 (m, 4H), 1.23 (s, 3H).

### Example 13

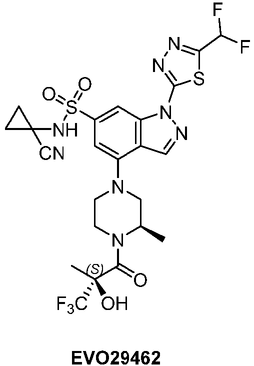

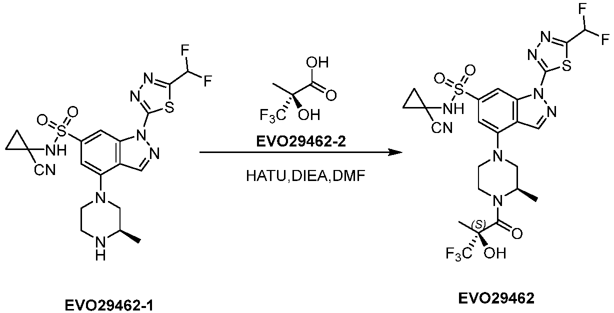

### Step 1:

Compound EVO29462-1 (100.0 mg, 0.20 mmol) was dissolved in N,N-dimethylformamide (2 mL), and then *O*-(7-azabenzotriazol-1-yl)-*N*,*N*,*N*',*N*'-tetramethyluronium hexafluorophosphate (115.0 mg, 0.30 mmol) and *N,N-*diisopropylethylamine (130.0 mg, 0.60 mmol) were added, followed by addition of EVO29462-2 (48.0 mg, 0.30 mmol). The mixture was stirred for reaction at room temperature for 2 h. TLC analysis showed no starting material remaining. Water (30 mL) was added, and the mixture was extracted with ethyl acetate (20 mL × 2). The organic phase was washed with saturated brine (20 mL), dried over anhydrous sodium sulfate, filtered, and concentrated, and the residue was separated and purified by column chromatography (petroleum ether/ethyl acetate = 2:1) to give compound EVO29462 (15 mg, yield: 11%). LCMS (ESI) m/z = 635 [M+H]⁺. ¹H NMR (400 MHz, DMSO-*d*₆) δ 9.34 (s, 1H), 8.89 (s, 1H), 8.49 (d, *J* = 17.4 Hz, 1H), 7.60 (t, *J =* 53.1 Hz, 1H), 7.25 (s, 1H), 7.15 (d, *J =* 12.8 Hz, 1H), 4.61 (t, *J*= 67.4 Hz, 3H), 3.82 (dd, *J =* 32.3, 4H), 1.59 (s, 3H), 1.45 (dd, *J =* 8.0, 3H), 1.37 - 1.28 (m, 4H).

### Example 14

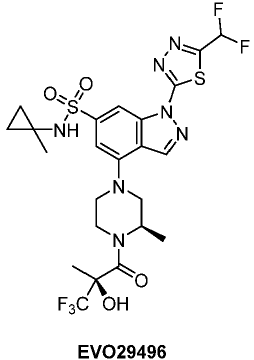

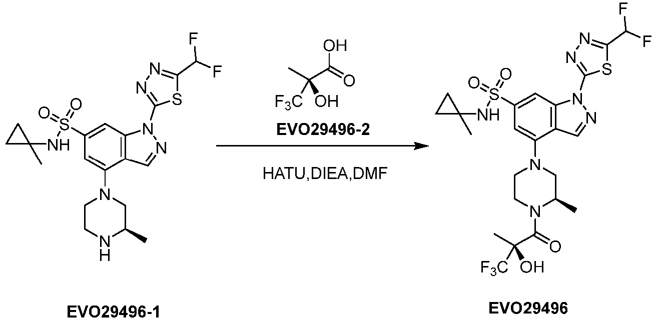

### Step 1:

Compound EVO29496-1 (100.0 mg, 0.20 mmol) was dissolved in *N,N*-dimethylformamide (2 mL), and then *O*-(7-azabenzotriazol-1-yl)-*N*,*N*,*N*',*N*'-tetramethyluronium hexafluorophosphate (115.0 mg, 0.30 mmol) and *N*,*N*-diisopropylethylamine (80.0 mg, 0.60 mmol) were added, followed by addition of EVO29496-2 (50.0 mg, 0.30 mmol). The mixture was stirred for reaction at room temperature for 2 h. Water (30 mL) was added, and the mixture was extracted with ethyl acetate (20 mL × 2). The organic phases were combined, washed with saturated brine (20 mL), dried over anhydrous sodium sulfate, filtered, and concentrated, and the residue was purified by column chromatography (petroleum ether:ethyl acetate = 1:1) to give compound EVO29496 (45 mg, yield: 34%). LCMS (ESI) m/z = 624.1 [M+H]⁺. ¹H NMR (400 MHz, DMSO-*d*₆) δ 8.85 (s, 1H), 8.45 (d, *J =* 16.2 Hz, 1H), 8.31 (s, 1H), 7.60 (t, *J =* 53.1 Hz, 1H), 7.21 (s, 1H), 7.13 (s, 1H), 5.39 - 4.19 (m, 2H), 3.93 - 3.35 (m, 4H), 3.24 (s, 1H), 1.58 (s, 3H), 1.52 - 1.28 (m, 3H), 1.08 (s, 3H), 0.66 (s, 2H), 0.41 (d, *J =* 1.6 Hz, 2H).

### Example 15

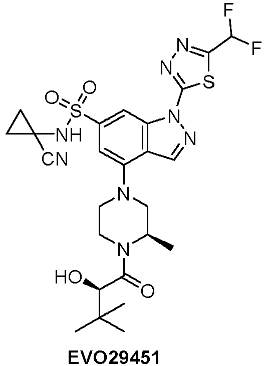

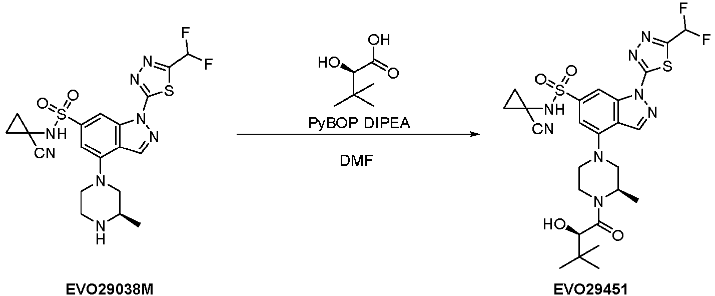

### Step 1:

(*R*)-2-Hydroxy-3,3-dimethylbutyric acid (32.1 mg, 242.65 µM) was dissolved in 5 mL of DMF, and then PyBOP (115.7 mg, 222.43 µM) and DIPEA (40.9 mg, 404.42 µM) were added. The mixture was stirred at room temperature for 10 min, and EVO29038M (100.0 mg, 202.21 µM) was added. The mixture was allowed to react at room temperature for 17 h. Water was added, and the mixture was extracted twice with ethyl acetate. The organic phases were combined, washed once with saturated brine, dried over anhydrous sodium sulfate, filtered, and concentrated under reduced pressure. The residue was subjected to preparative medium-pressure chromatography (DCM:MeOH = 20:1). The fractions were evaporated to dryness to give a yellow oil, which was then separated and purified by preparative high performance liquid chromatography to give EVO29451 as a yellow solid (19.2 mg, yield: 16%). LCMS (ESI) m/z = 609.1 [M+H]⁺. ¹H NMR (400 MHz, DMSO-*d*₆) δ 9.21 (s, 1H), 8.84 (s, 1H), 8.48 (s, 1H), 7.58 (t, *J =* 53.1 Hz, 1H), 7.14 (s, 1H), 4.80 (d, *J =* 7.5 Hz, 1H), 4.71 (s, 1H), 4.43-4.19 (m, 1H), 4.15 - 3.99 (m, 1H), 3.84 (d, *J* = 10.9 Hz, 1H), 3.77-3.62 (m, 2H), 3.36 (d, *J =* 11.2 Hz, 1H), 1.53 - 1.23 (m, 7H), 0.97 (s, 9H).

### Example 16

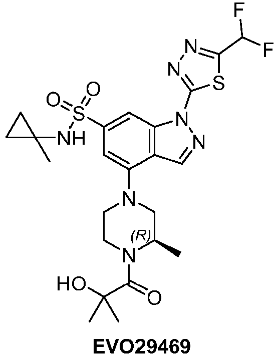

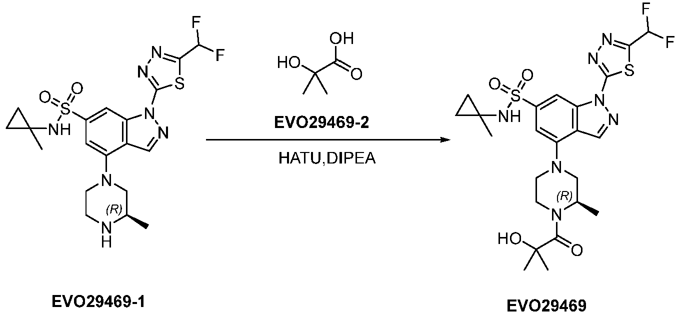

Compound EVO29469-1 (100 mg, 0.207 mmol), compound EVO29469-2 (55.2 mg, 0.42 mmol), *O*-(7-azabenzotriazol-1-yl)-*N*,*N*,*N*',*N*'-tetramethyluronium hexafluorophosphate (152.9 mg, 0.42 mmol), and *N*,*N*-diisopropylethylamine (117.4 mg, 0.91 mmol) were dissolved in N,N-dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:2) to give EVO29469 as a yellow solid (25 mg, yield: 20.8%). LCMS (ESI) m/z = 570.0 [M+H]⁺. ¹H NMR (400 MHz, DMSO) δ 8.83 (s, 1H), 8.42 (s, 1H), 8.32 (s, 1H), 7.60 (t, *J =* 53.0 Hz, 1H), 7.13 (s, 1H), 5.51 (s, 1H), 4.76 (d, *J* = 71.4 Hz, 1H), 3.78 (s, 1H), 3.73 - 3.55 (m, 2H), 3.09 (s, 2H), 1.35 (d, *J = 7.9* Hz, 6H), 1.27 (s, 2H), 1.24 - 1.23 (m, 1H), 1.08 (s, 3H), 0.66 (s, 2H), 0.40 (s, 2H).

### Example 17

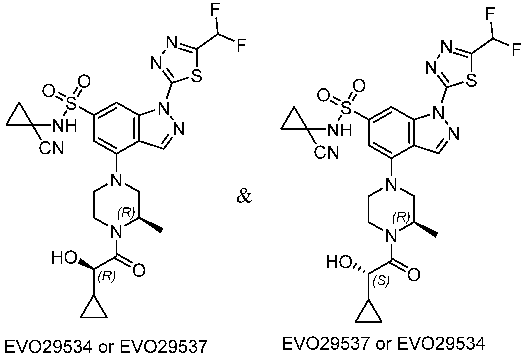

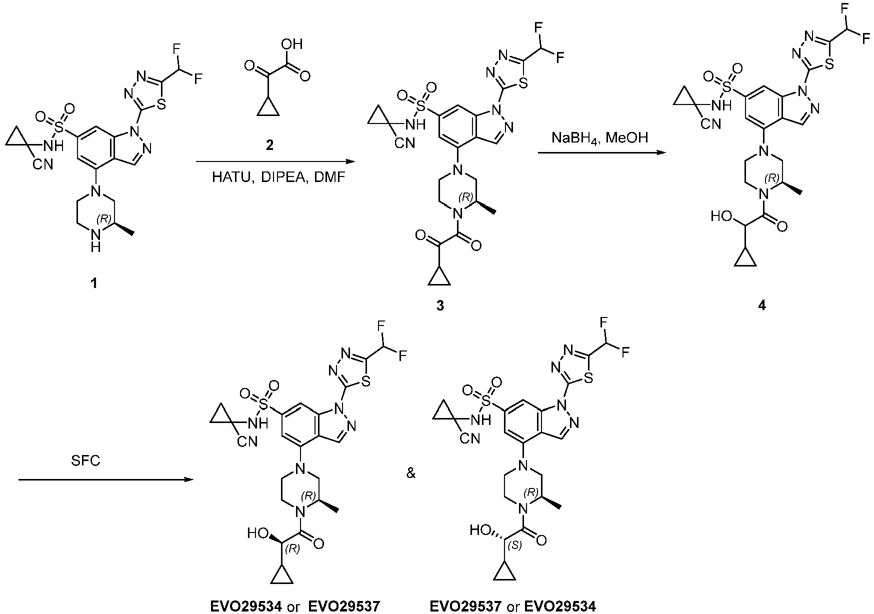

### Step 1:

Compound 1 (500.00 mg, 1.01 mmol), compound 2 (173.00 mg, 1.52 mmol), O-(7-azabenzotriazol-1-yl)-*N*,*N*,*N*',*N*'-tetramethyluronium hexafluorophosphate (577.60 mg, 1.52 mmol), and N,N-diisopropylethylamine (392.20 mg, 3.04 mmol) were dissolved in N,N-dimethylformamide (10 mL), and the reaction mixture was allowed to react at room temperature for 2 h. Water (20 mL) was added to the reaction mixture, and the resulting mixture was extracted with ethyl acetate (30 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 2:1) to give compound 3 as a yellow solid (540.00 mg, yield: 91.4%). LCMS (ESI) m/z = 591.1 [M+H]⁺.

### Step 2:

To compound 3 (520.00 mg, 0.88 mmol) in methanol (20 mL) was slowly added sodium borohydride (66.50 mg, 1.76 mmol), and the mixture was allowed to react at room temperature for 2 h. Water (20 mL) was added to the reaction mixture, and the resulting mixture was extracted with ethyl acetate (50 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:1 to 100% ethyl acetate) to give compound 4 as a yellow solid (430.00 mg, yield: 82.5%). LCMS (ESI) m/z = 593.2 [M+H]⁺.

### Step 3:

Compound 4 (100.00 mg, 0.17 mmol) was subjected to resolution by supercritical fluid chromatography (SFC) (chromatography column: ChiralCel OX, 250 × 30 mm I.D., 10 µm; mobile phases: CO₂ as phase A, ethanol as phase B; gradient: B 40%; flow rate: 150 mL/min; back pressure: 100 bar; column temperature: 38 °C; wavelength: 220 nm; cycle time: about 9 min; injection volume: 1.00 mL) to give EVO29534 (21.6 mg, retention time: 1.764 min) and EVO29537 (46.5 mg, retention time: 2.621 min). LCMS (ESI) m/z = 593.2 [M+H]⁺.

### EVO29534:

¹H NMR (400 MHz, DMSO-*d*₆) δ 9.33 (s, 1H), 8.87 (s, 1H), 8.48 (s, 1H), 7.63 (t, *J =* 52 Hz, 1H), 7.15 (s, 1H), 5.18-5.05 (m, 1H), 4.65 (s, 1H), 4.39-4.07 (m, 1H), 3.90-3.70 (m, 4H), 3.23 (s, 1H), 1.49-1.35 (m, 7H), 1.19-1.13 (m, 1H), 0.89 (s, 1H), 0.48-0.23 (m, 4H).

### EVO29537:

¹H NMR (400 MHz, DMSO-*d*₆) δ 9.34 (s, 1H), 8.86 (s, 1H), 8.48 (d, *J =* 10.5 Hz, 1H), 7.61 (t, J = 52.6 Hz, 1H), 7.16 (d, *J* = 11.8 Hz, 1H), 5.04 - 4.83 (m, 1H), 4.64 (d, *J* = 70.3 Hz, 1H), 3.98 (ddd, *J =* 97.0, 68.1, 55.4 Hz, 5H), 3.09 (d, *J =* 79.8 Hz, 2H), 1.36 (d, *J =* 50.6 Hz, 7H), 1.12 (s, 1H), 0.38 (d, *J =* 33.4 Hz, 4H).

### Example 18

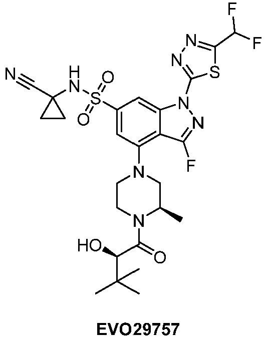

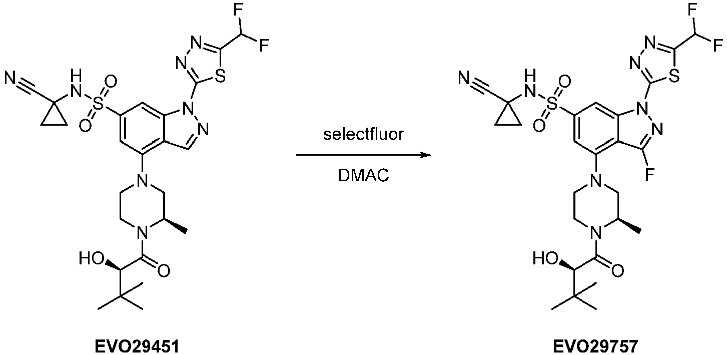

### Step 1:

EVO29451 (50.0 mg, 82.14 µmol) was dissolved in DMAC (3 mL), and then selectfluor (58.2 mg, 164.29 µmol) was added. The mixture was allowed to react at 60 °C for 14 h under an argon atmosphere. Water was added, and the mixture was extracted twice with ethyl acetate. The organic phases were combined, washed once with saturated brine, dried over anhydrous sodium sulfate, filtered, and concentrated under reduced pressure, and the residue was subjected to preparative medium-pressure chromatography (PE:EA = 1:1) and concentrated under reduced pressure to give a yellow oil (54.0 mg), which was then separated and purified by preparative high performance liquid chromatography to give EVO29757 as a yellow solid (7.0 mg, yield: 14%). LCMS (ESI) m/z = 627.2 [M+H]⁺. ¹H NMR (400 MHz, DMSO-*d*₆) δ 9.66 (s, 1H), 8.96 (d, *J* = 3.6 Hz, 1H), 8.58 (d, *J = 4.5* Hz, 1H), 7.62 (t, *J* = 53.1 Hz, 1H), 5.25 - 4.67 (m, 1H), 4.52 - 4.01 (m, 2H), 3.73 - 3.35 (m, 4H), 3.29 (s, 1H), 1.56 - 1.41 (m, 3H), 1.41 - 1.22 (m, 4H), 0.97 (s, 9H).

### Example 19

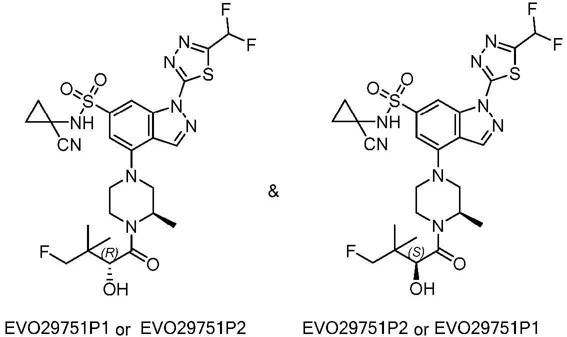

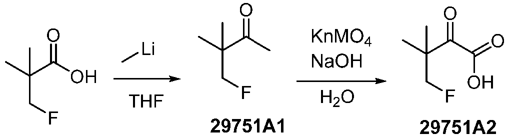

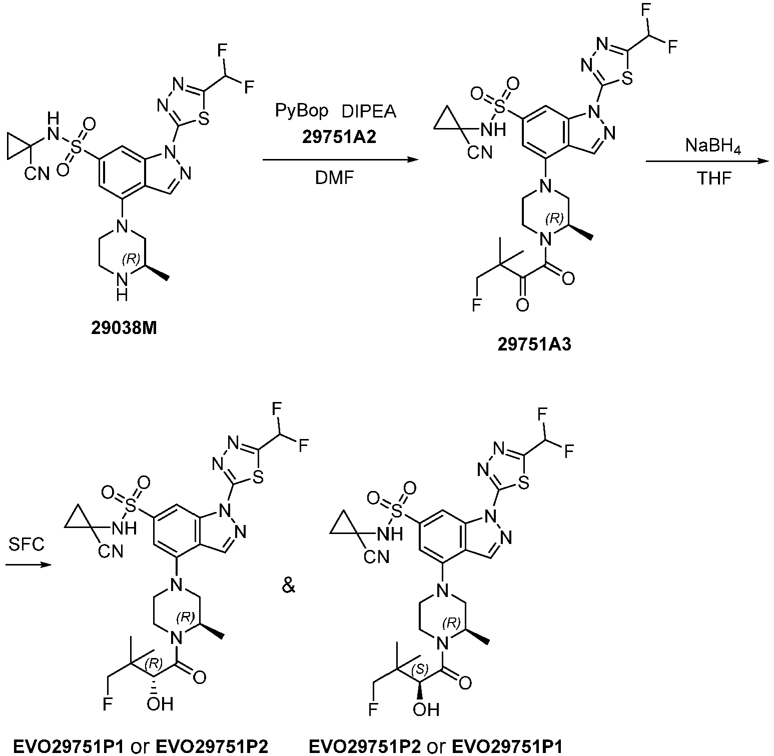

### Step 1:

To a solution of compound 3-fluoro-2,2-dimethylpropanoic acid (0.7 g, 5.83 mmol) in anhydrous tetrahydrofuran (20 mL) was added methyllithium (2.5 M in diethyoxymethane) (4.9 mL, 12.25 mmol) at - 78 °C under an argon atmosphere, and after the addition, the mixture was naturally warmed to room temperature and stirred for 10 h. A 1 M HCl solution (20 mL) was added to the reaction mixture, and the resulting mixture was extracted with ethyl acetate. The organic phase was concentrated to remove the solvent to give 29751A1 as a pale yellow oil (0.29 g, yield: 42.1%) (crude).

### Step 2:

Under an ice bath, sodium hydroxide (0.2 g, 4.91 mmol) was added to a solution of compound 29751A1 (0.29 g, 2.45 mmol) in water (10 mL), and then potassium permanganate (0.7 g, 4.42 mmol) was slowly added. The mixture was stirred at room temperature for 7 h. Ethanol (3 mL) was added to the reaction mixture, and the resulting mixture was reversely extracted with ethyl acetate. The aqueous phase was adjusted to pH 2 with 1 M HCl and then extracted with ethyl acetate. The organic phase was concentrated to remove the solvent to give 29751A2 as a colorless oil (0.26 g, yield: 71.6%). LCMS (ESI) m/z = 147.1 [M-1]⁻.

### Step 3:

Under an ice bath, DIPEA (0.45 g, 3.51 mmol) and PyBop (1.37 g, 2.63 mmol) were added to a solution of compound 29751A2 (0.26 g, 1.75 mmol) in DMF (5 mL), and the mixture was stirred for 0.5 h. 29038M (0.79 g, 1.59 mmol) was added to the reaction mixture, and the resulting mixture was stirred at room temperature for 4 h. The reaction mixture was concentrated to remove the solvent, and the residue was separated and purified by silica gel column chromatography (ethyl acetate/petroleum ether) to give 29751A3 as a pale yellow solid (0.52 g, yield: 52.1%). LCMS (ESI) m/z = 625.17 [M+H]⁺.

### Step 4:

Under an ice bath, NaBH₄ (37 mg, 1 mmol) was added to a solution of compound 29751A3 (0.52 g, 0.83 mmol) in tetrahydrofuran (5 mL), and the mixture was stirred at room temperature for 1 h. Water was added to the reaction mixture, and the resulting mixture was extracted with ethyl acetate. The organic phase was concentrated to remove the solvent, and the residue was separated and purified by silica gel column chromatography (ethyl acetate/petroleum ether) and subjected to resolution by SFC (instrument: WATERS 150 preparative SFC (SFC-26); chromatography column: ChiralPak IG, 250 × 30 mm I.D., 10 µm; mobile phases: CO₂ as phase A, ethanol (0.1% NH₃H₂O) as phase B; gradient: B 40%; flow rate: 150 mL/min; back pressure: 100 bar) to give EVO29751P1 (54 mg, yield: 10.4%, retention time: 2.021 min) (LCMS (ESI) m/z = 627.17 [M+H]⁺; ¹H NMR (400 MHz, DMSO-*d*₆) δ 9.30 (s, 1H), 8.86 (d, *J* = 4.3 Hz, 1H), 8.48 (d, *J =* 6.8 Hz, 1H), 7.60 (t, *J= 53.1* Hz, 1H), 7.14 (dd, *J =* 5.2, 1.4 Hz, 1H), 5.66 - 5.22 (m, 1H), 4.76 - 4.62 (m, 1H), 4.45 - 4.33 (m, 1H), 4.32 - 4.14 (m, 3H), 3.91 - 3.65 (m, 3H), 3.36 (d, *J* = 4.0 Hz, 1H), 3.11 (dt, *J =* 64.2, 10.8 Hz, 1H), 1.44 (q, *J =* 4.6, 3.8 Hz, 3H), 1.31 (dd, *J= 5.7, 3.5* Hz, 4H), 1.04 - 0.93 (m, 6H)) and

EVO29751P2 (115 mg, yield: 22.1%, retention time: 2.792 min) (LCMS (ESI) m/z = 627.17 [M+H]⁺; ¹H NMR (400 MHz, DMSO-*d*₆) δ 9.34 (s, 1H), 8.88 (d, *J =* 1.0 Hz, 1H), 8.48 (d, *J* = 12.6 Hz, 1H), 7.61 (*t, J* = 53.1 Hz, 1H), 7.15 (d, *J =* 13.9 Hz, 1H), 5.02 (dd, *J =* 19.2, 7.9 Hz, 1H), 4.81 (d, *J* = 7.9 Hz, 1H), 4.54 - 4.08 (m, 5H), 3.85 (d, *J =* 13.1 Hz, 1H), 3.73 (d, *J =* 12.2 Hz, 2H), 3.44 - 3.34 (m, 1H), 3.29 - 2.99 (m, 1H), 1.43 (dd, *J =* 12.7, 4.8 Hz, 3H), 1.30 (dd, *J =* 8.5, 5.2 Hz, 4H), 0.96 (dd, *J =* 19.5, 5.9 Hz, 6H)).

### Example 20

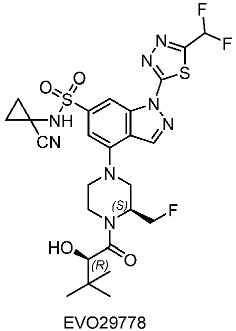

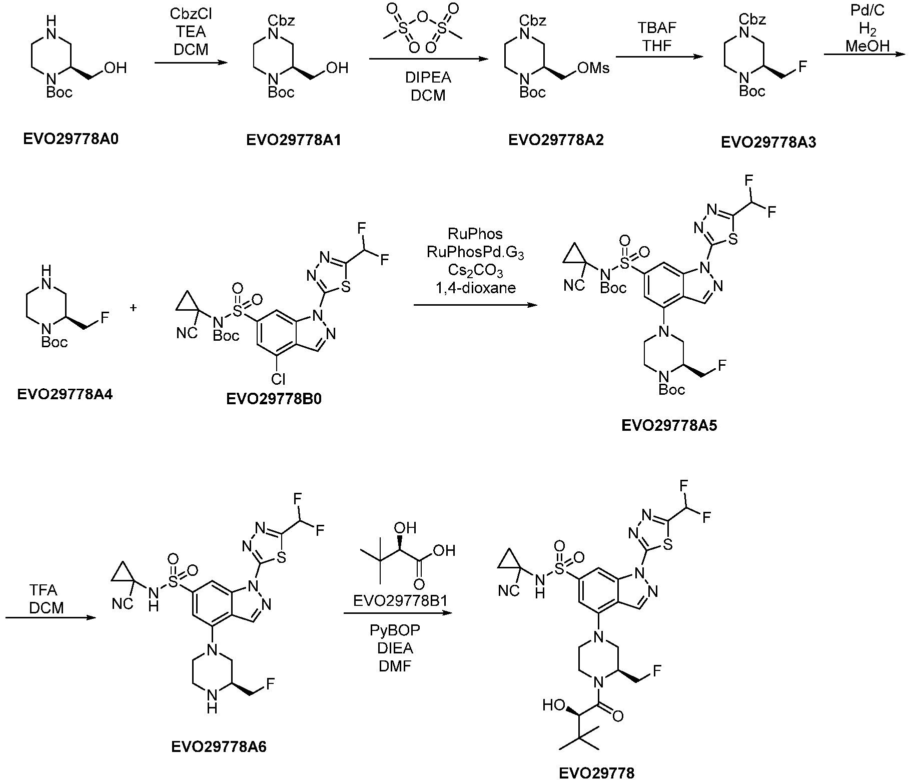

### Step 1:

Compound EVO29778A0 (2.5 g, 11.56 mol) and TEA (2.3 g, 23.12 mmol) were added to DCM (25 mL), and under an argon atmosphere, CbzCl (2.1 g, 12.72 mmol) was added under an ice bath. The mixture was stirred under the ice bath for 2 h. To the system was added 1 N HCl (10 mL), and the mixture was extracted with DCM (10 mL × 3). The organic phase was washed with a saturated sodium bicarbonate solution and then with saturated brine, and concentrated under reduced pressure to give a crude product, which was then purified by column chromatography to give EVO29778A1 (3.5 g, 86.4%).

### Step 2:

Compound EVO29778A1 (3.5 g, 9.99 mol) and DIPEA (3.2 g, 24.97 mmol) were added to DCM (30 mL), and under an argon atmosphere, methanesulfonic anhydride (2.6 g, 14.98 mmol) was added under an ice bath. The mixture was stirred under the ice bath for 2 h. To the system was added 1 N HCl (10 mL) and the mixture was extracted with DCM (10 mL × 3). The organic phase was washed with a saturated sodium bicarbonate solution and then with saturated brine, and concentrated under reduced pressure to give a crude product of EVO29778A2 (4.3 g).

### Step 3:

Compound EVO29778A2 (1.8 g, 4.20 mol) and TBAF (21 mL, 1 M in THF) were added to THF (22 mL), and the mixture was stirred at 70 °C for 3 h. To the system was added water (20 mL), and the mixture was extracted with EA (10 mL × 3). The organic phase was washed with saturated brine and concentrated under reduced pressure to give a crude product, which was then purified by column chromatography to give EVO29778A3 (300 mg, 20.2%).

### Step 4:

Compound EVO29778A3 (300 mg, 4.20 mol) and Pd/C (90 mg) were added to methanol (10 mL), and the mixture was purged with hydrogen and stirred at room temperature overnight. The mixture was then filtered through celite, and the filtrate was concentrated to dryness by rotary evaporation to give a crude product of EVO29778A4 (200 mg).

### Step 5:

Compound EVO29778B0 (156 mg, 0.29 mol), compound EVO29778A4 (64 mg, 0.29 mmol), Ruphos (109.4 mg, 0.23 mmol), RuPhos Pd G3 (98.1 mg, 0.11 mmol), and cesium carbonate (382.1 mg, 1.17 mmol) were added to 1,4-dioxane (10 mL), and under an argon atmosphere, the mixture was stirred at 100 °C for 4 h. To the system was added water (10 mL), and the mixture was extracted with ethyl acetate (10 mL × 3). The organic phase was concentrated under reduced pressure to give a crude product, which was then purified by column chromatography to give EVO29778A5 (163 mg, 77.9%).

### Step 6:

Compound EVO29778A5 (163 mg, 0.22 mmol) was added to DCM (1 mL), followed by addition of TFA (2 mL). The mixture was stirred at room temperature for 4 h. To the system was added a sodium bicarbonate solution to adjust the pH to neutrality, and the mixture was extracted with DCM (10 mL × 2), dried over anhydrous sodium sulfate, and concentrated to dryness by rotary evaporation to give EVO29778A6 (116 mg, 100%).

### Step 7:

Compound EVO29778A6 (116 mg, 0.22 mmol), compound EVO29778B1 (44.8 mg, 0.33 mmol), and PyBOP (188.4 mg, 0.36 mmol) were added to DMF (5 mL), followed by addition of DIPEA (87.7 mg, 0.67 mmol). The mixture was stirred at room temperature overnight. To the system was added water (10 mL), and the mixture was extracted with ethyl acetate (10 mL × 2). The organic phase was concentrated under reduced pressure to give a crude product, which was then purified by preparative liquid chromatography to give EVO29778 (50 mg, 35.2%). LCMS: [M+H]⁺ 627.15. ¹H NMR (400 MHz, DMSO-d6) δ 9.38 (s, 1H), 8.85 (d, J = 6.2 Hz, 1H), 8.55 (d, J = 15.3 Hz, 1H), 7.64 (t, J = 53.1 Hz, 1H), 7.21 (dd, J = 12.4, 1.3 Hz, 1H), 5.39 (dd, J = 176.0, 7.4 Hz, 1H), 5.03 - 4.58 (m, 3H), 4.45 (t, J = 15.4 Hz, 1H), 4.15 (d, J = 7.4 Hz, 3H), 3.77 - 3.37 (m, 1H), 3.32 - 2.96 (m, 2H), 1.47 (t, J = 4.0 Hz, 2H), 1.35 (t, J = 4.0 Hz, 2H), 1.00 (d, J = 9.0 Hz, 9H).

### Example 21

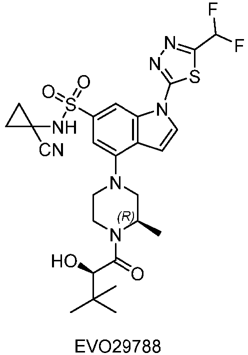

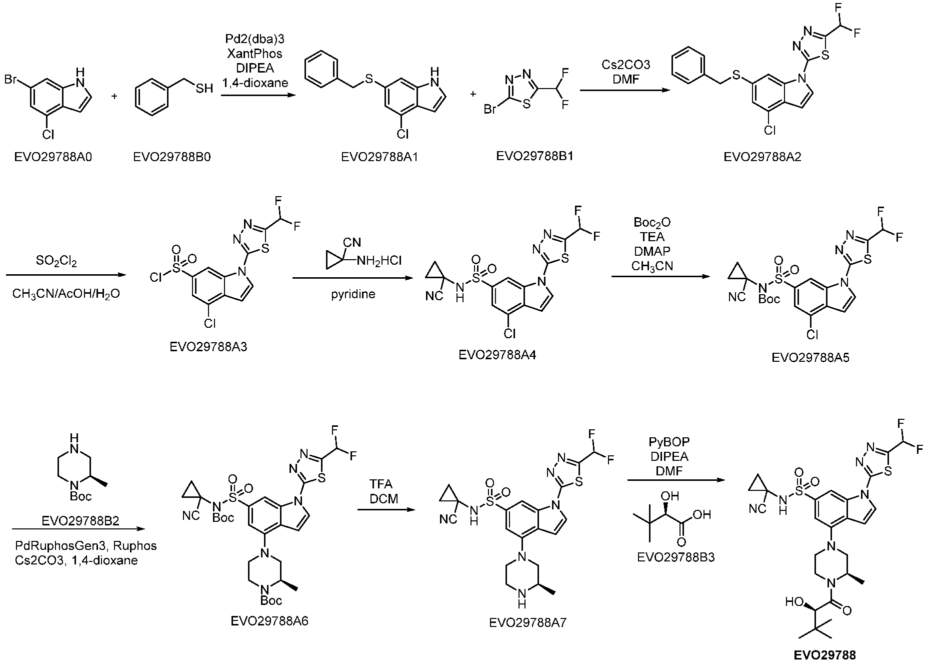

### Step 1:

To a reaction flask were added compound EVO29788A0 (3.0 g, 13.02 mmol), compound EVO29788B0 (1.9 g, 15.62 mmol), Xantphos (1.1 g, 1.95 mmol), Pd₂(dba)₃ (893.9 mg, 0.97 mmol), DIPEA (5.0 g, 39.05 mmol), and 1,4-dioxane (30 mL). The mixture was purged three times with argon, then heated to 90 °C, and allowed to react for 3 h. The reaction mixture was cooled to room temperature, filtered through celite, and concentrated to dryness by rotary evaporation to give a crude product, which was then purified by flash silica gel column chromatography (DCM/PE = 0-50%) to give EVO29788A1 (2.3 g, 64.5%).

### Step 2:

To a reaction flask were added compound EVO29788A1 (2.3 g, 8.40 mmol), compound EVO29788B1 (2.1 g, 10.08 mmol), cesium carbonate (5.4 g, 16.8 mmol), and DMF (20 mL). The mixture was purged with argon, then heated to 60 °C, and allowed to react for 2 h. Water (20 mL) was added to quench the reaction, and the aqueous phase was extracted with ethyl acetate (20 mL × 3). The organic phase was washed with saturated brine (20 mL) and concentrated to dryness by rotary evaporation to give a crude product, which was then purified by flash silica gel column chromatography (EA/PE = 0-50%) to give EVO29788A2 (1.7 g, 51.9%).

### Step 3:

Compound EVO29788A2 (600 mg, 1.47 mmol) was added to acetonitrile/water/acetic acid (10 mL, 200:5:2.5), and the mixture was cooled under an ice bath. SO₂Cl₂ (794.1 mg, 5.88 mmol) was slowly added, and after the addition, the mixture was allowed to react under the ice bath for 2 h. The reaction mixture was concentrated to give EVO29788A3 (565 mg, 100%).

### Step 4:

Compound EVO29788A3 (565 mg, 1.47 mmol) and 1-amino-1-cyclopropanecarbonitrile hydrochloride (348.7 mg, 2.94 mmol) were added to pyridine (10 mL), and the mixture was allowed to react at room temperature overnight. The reaction mixture was extracted with ethyl acetate (50 mL), washed with a 1 N hydrochloric acid solution (10 mL × 3), dried over anhydrous sodium sulfate, and filtered, and the filtrate was concentrated to dryness by rotary evaporation to give EVO29788A4 (518 mg, 81.9%).

### Step 5:

Compound EVO29788A4 (518.0 mg, 1.21 mmol), Boc₂O (315.6 mg, 1.45 mmol), TEA (365.8 mg, 3.62 mmol), and DMAP (14.7 mg, 0.12 mmol) were added to acetonitrile (10 mL), and the mixture was allowed to react at room temperature for 30 min. The reaction mixture was concentrated to remove the solvent, and the residue was purified by flash silica gel column chromatography (EA/PE = 0-20%) to give EVO29788A5 (310 mg, 48.5%).

### Step 6:

Compound EVO29788A5 (190mg, 0.35 mol), compound EVO29788B2 (143.6 mg, 0.71 mmol), Ruphos (133.8 mg, 0.28 mmol), RuPhos Pd G3 (119.9 mg, 0.14 mmol), and cesium carbonate (584 mg, 1.79 mmol) were added to 1,4-dioxane (10 mL), and under an argon atmosphere, the mixture was stirred at 95°C for 4 h. To the system was added water (10 mL), and the mixture was extracted with ethyl acetate (10 mL × 3). The organic phase was concentrated under reduced pressure to give a crude product, which was then purified by column chromatography to give EVO29788A6 (180 mg, 72.3%).

### Step 7:

Compound EVO29788A6 (180 mg, 0.25 mmol) was added to DCM (1 mL), followed by addition of TFA (2 mL). The mixture was stirred at room temperature for 2 h. To the system was added a sodium bicarbonate solution to adjust the pH to neutrality, and the mixture was extracted with DCM (10 mL × 2), dried over anhydrous sodium sulfate, and concentrated to dryness by rotary evaporation to give EVO29788A7 (124 mg, 96.8%).

### Step 8:

Compound EVO29788A7 (124 mg, 0.25 mmol), compound EVO29788B3 (99.6 mg, 0.75 mmol), and PyBOP (392.2 mg, 0.75 mmol) were added to DMF (5 mL), followed by addition of DIPEA (194.8 mg, 1.51 mmol). The mixture was stirred at room temperature overnight. To the system was added water (10 mL), and the mixture was extracted with ethyl acetate (10 mL × 2). The organic phase was concentrated under reduced pressure to give a crude product, which was then purified by preparative liquid chromatography to give EVO29788 (51 mg, 33.4%). LCMS: [M+H]⁺ 608.45. ¹H NMR (400 MHz, DMSO-d6) δ 9.14 (s, 1H), 8.65 (s, 1H), 8.21 (d, J = 3.6 Hz, 1H), 7.67 (t, J = 53.1 Hz, 1H), 7.25 (d, J = 1.5 Hz, 1H), 7.07 (t, J = 4.5 Hz, 1H), 5.27 - 4.70 (m, 2H), 4.55 - 4.04 (m, 2H), 3.68 - 3.46 (m, 3H), 3.26 - 2.71 (m, 2H), 1.58 - 1.29 (m, 7H), 0.99 (d, J = 4.9 Hz, 9H).

### Example 22

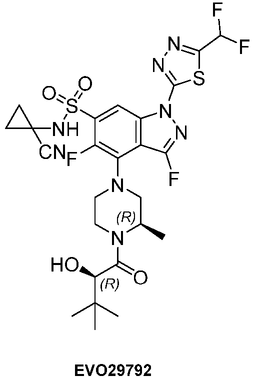

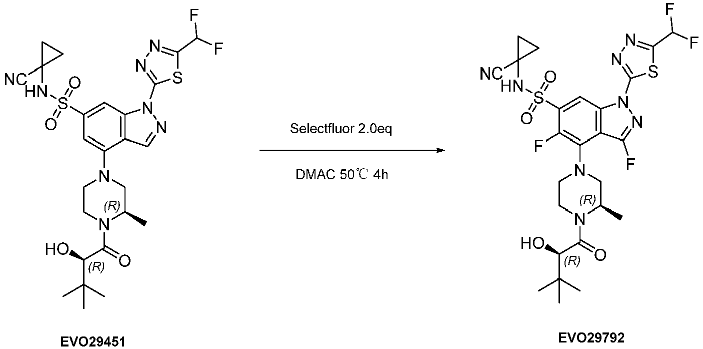

### Step 1:

To a 100 mL single-neck flask were added EVO29451 (500.0 mg, 0.82 mmol), DMAC (20 mL), and 1-chloromethyl-4-fluoro-1,4-diazoniabicyclo[2.2.2]octane bis(tetrafluoroborate) (580.0 mg, 1.64 mmol), and the mixture was stirred at 50 °C for 4 h. After the reaction was completed, 60 mL of water was added, and the mixture was extracted twice with 80 mL of ethyl acetate. The organic phases were combined, washed once with saturated brine, dried over anhydrous sodium sulfate, filtered, and concentrated under reduced pressure. The residue was subjected to preparative medium-pressure chromatography (PE:EA = 1:1). The fractions were evaporated to dryness to give a yellow oil, which was then separated and purified by preparative high performance liquid chromatography to give EVO29792 as a yellow solid (45.0 mg, yield: 8.7%). LCMS (ESI) m/z = 645.1 [M+H]⁺.

¹H NMR (400 MHz, DMSO-*d*₆) δ 9.02 (s, 1H), 7.63 (t, *J =* 53.1 Hz, 1H), 4.80 (d, *J =* 7.5 Hz, 1H), 4.71 (s, 1H), 4.43-4.19 (m, 1H), 4.15 - 3.99 (m, 1H), 3.84 (d, *J =* 10.9 Hz, 1H), 3.77-3.62 (m, 2H), 3.36 (d, *J= 11.2* Hz, 1H), 1.57 - 1.46 (m, 3H),1.43 - 1.26 (m, 4H), 0.99 (s, 9H).

### Example 23

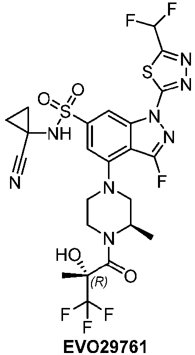

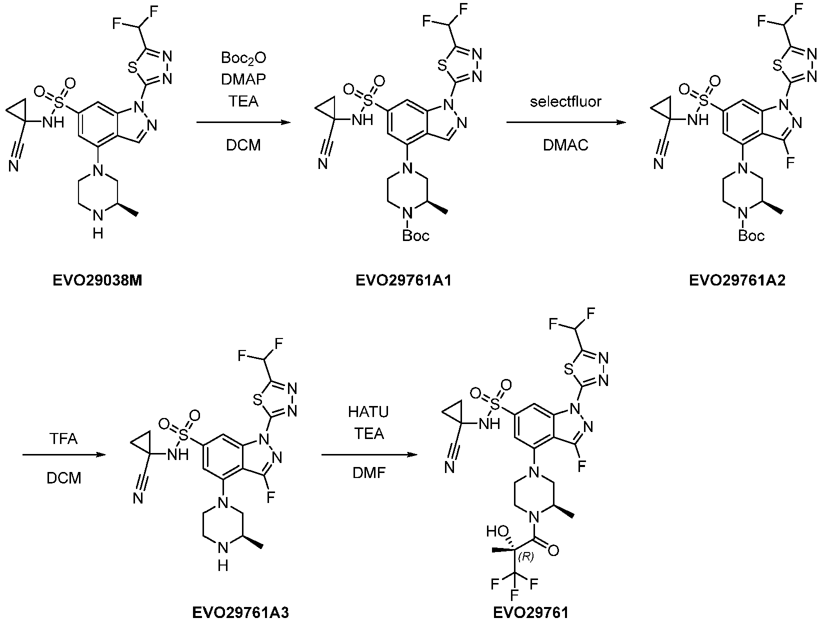

### Step 1:

EVO29038M (500.0 mg, 1.01 mmol) was dissolved in DCM (10 mL), and then DMAP (12.4 mg, 101.10 µmol), TEA (133.0 mg, 1.31 mmol), and Boc₂O (264.8 mg, 1.21 mmol) were added. The mixture was allowed to react at room temperature for 2 h. Water was then added, and the mixture was extracted twice with DCM. The organic phases were combined, washed once with saturated brine, dried over anhydrous sodium sulfate, filtered, and concentrated under reduced pressure, and the residue was subjected to preparative medium-pressure chromatography (PE:EA= 2:1). The fractions were evaporated to dryness to give EVO29761A1 as a yellow solid (601.0 mg, yield: 99%). LCMS (ESI) m/z = 595.1 [M+H]⁺.

### Step 2:

EVO29761A1 (300.0 mg, 504.49 µmol) was dissolved in DMAC (4 mL), and then selectfluor (357.4 mg, 1.01 mmol) was added. The mixture was allowed to react at 60 °C for 6 h under an argon atmosphere. Water was added, and the mixture was extracted twice with ethyl acetate. The organic phases were combined, washed once with saturated brine, dried over anhydrous sodium sulfate, filtered, and concentrated under reduced pressure, and the residue was subjected to preparative medium-pressure chromatography (PE:EA = 1:1). The fractions were evaporated to dryness to give EVO29761A2 as a yellow oil (168.0 mg, yield: 54%). LCMS (ESI) m/z = 513.1 [M+H-Boc]⁺.

### Step 3:

EVO29761A2 (168.0 mg, 274.22 µmol) was dissolved in DCM (1 mL), and then TFA(1.5 g, 13.07 mmol) was added. The mixture was allowed to react at room temperature for 2 h, then neutralized with a saturated aqueous sodium bicarbonate solution, and extracted twice with ethyl acetate. The organic phases were combined, washed once with saturated brine, dried over anhydrous sodium sulfate, filtered, and concentrated under reduced pressure to give EVO29761A3 as a yellow oil (118.0 mg, yield: 84%).

### Step 4:

(S)-2-Trifluoromethyl-2-hydroxypropanoic acid (36.4 mg, 230.23 µmol) and EVO29761A3 (118.0 mg, 230.23 µmol) were dissolved in DMF (5 mL), and then TEA (35.0 mg, 345.34 µmol) and HATU (113.8 mg, 299.30 µmol) were added. The mixture was allowed to react at room temperature for 3 h. Water was then added, and the mixture was extracted twice with ethyl acetate. The organic phases were combined, washed once with saturated brine, dried over anhydrous sodium sulfate, filtered, and concentrated under reduced pressure, and the residue was subjected to preparative medium-pressure chromatography (PE:EA = 1:1). The fractions were evaporated to dryness to give a brown liquid (50.0 mg), which was then separated and purified by preparative high performance liquid chromatography to give EVO29761 as a yellow solid (9.4 mg, yield: 6%). LCMS (ESI) m/z = 653.3 [M+H]⁺. ¹H NMR (400 MHz, DMSO-*d*₆) δ 9.66 (s, 1H), 8.97 (d, *J =* 9.0 Hz, 1H), 8.58 (*d, J* = 4.4 Hz, 1H), 7.62 (t, *J =* 53.0 Hz, 1H), 7.22 (s, 1H), 5.15 - 4.74 (m, 1H), 4.74 - 4.24 (m, 1H), 3.69 (d, *J* = 11.4 Hz, 1H), 3.63 - 3.36 (m, 3H), 3.26 (s, 1H), 1.66 - 1.26 (m, 10H).

### Example 24

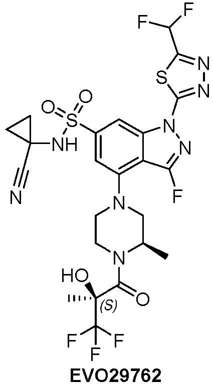

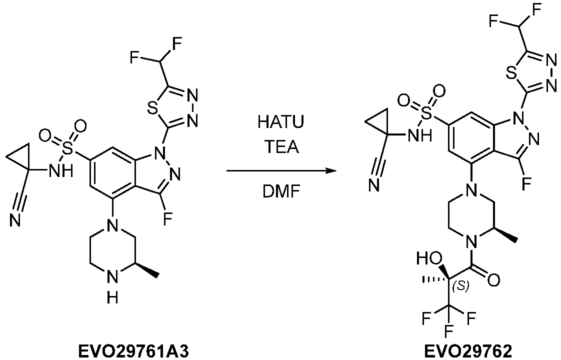

### Step 1:

(*R*)-2-Trifluoromethyl-2-hydroxypropanoic acid (82.7 mg, 522.90 µmol) and EVO29761A3 (134.0 mg, 261.45 µmol) were dissolved in DMF (5 mL), and then TEA (79.4 mg, 784.34 µmol) and HATU (218.7 mg, 575.18 µmol) were added. The mixture was allowed to react at room temperature for 3 h. Water was then added, and the mixture was extracted twice with ethyl acetate. The organic phases were combined, washed once with saturated brine, dried over anhydrous sodium sulfate, filtered, and concentrated under reduced pressure, and the residue was subjected to preparative medium-pressure chromatography (PE:EA= 1:1). The fractions were evaporated to dryness to give a brown liquid (47.0 mg), which was then separated and purified by preparative high performance liquid chromatography to give EVO29762 as a yellow solid (16.3 mg, yield: 6%). LCMS (ESI) m/z = 653.3 [M+H]⁺. ¹H NMR (400 MHz, DMSO-*d*₆) δ 9.04 (s, 1H), 8.58 (s, 1H), 7.61 (t, *J =* 53.0 Hz, 1H), 7.21 (s, 1H), 5.26 - 4.75 (m, 1H), 4.74 - 4.30 (m, 1H), 3.69 (d, *J =* 10.9 Hz, 1H), 3.53 (s, 1H), 3.47 - 3.35 (m, 2H), 3.19 (s, 1H), 1.65 - 1.27 (m, 10H).

### Example 25

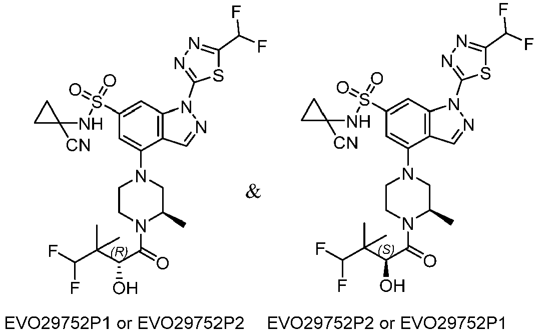

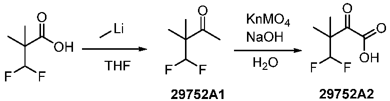

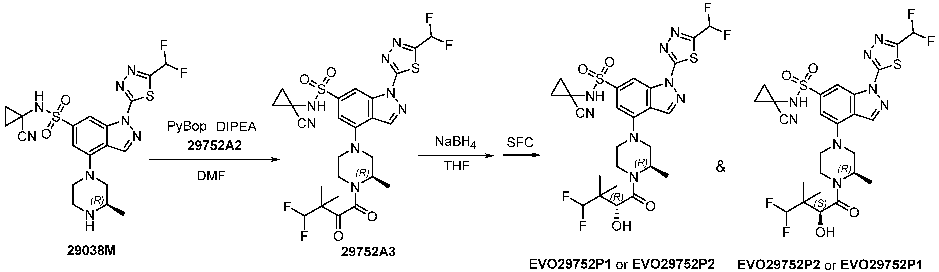

### Step 1:

To a solution of compound 3,3-fluoro-2,2-dimethylpropanoic acid (0.8 g, 5.83 mmol) in anhydrous tetrahydrofuran (20 mL) was added methyllithium (2.5 M in diethyoxymethane) (4.9 mL, 12.25 mmol) at - 78 °C under an argon atmosphere, and after the addition, the mixture was naturally warmed to room temperature and stirred for 10 h. A 1 M HCl solution (20 mL) was added to the reaction mixture, and the resulting mixture was extracted with ethyl acetate. The organic phase was concentrated to remove the solvent to give 29752A1 as a pale yellow oil (0.33 g, yield: 41.9%) (crude).

### Step 2:

Under an ice bath, sodium hydroxide (0.19 g, 4.85 mmol) was added to a solution of compound 29752A1 (0.33 g, 2.42 mmol) in water (10 mL), and then potassium permanganate (0.69 g, 4.36 mmol) was slowly added. The mixture was stirred at room temperature for 7 h. Ethanol (3 mL) was added to the reaction mixture, and the resulting mixture was reversely extracted with ethyl acetate. The aqueous phase was adjusted to pH 2 with 1 M HCl and then extracted with ethyl acetate. The organic phase was concentrated to remove the solvent to give 29752A2 as a colorless oil (0.29 g, yield: 72.1%). LCMS (ESI) m/z = 165.1 [M-1]⁻.

### Step 3:

Under an ice bath, DIPEA (0.45 g, 3.51 mmol) and PyBop (1.37 g, 2.63 mmol) were added to a solution of compound 29752A2 (0.29 g, 1.75 mmol) in DMF (5 mL), and the mixture was stirred for 0.5 h. 29038M (0.79 g, 1.59 mmol) was added to the reaction mixture, and the resulting mixture was stirred at room temperature for 4 h. The reaction mixture was concentrated to remove the solvent, and the residue was separated and purified by silica gel column chromatography (ethyl acetate/petroleum ether) to give 29752A3 as a pale yellow solid (0.32 g, yield: 32.0%). LCMS (ESI) m/z = 643.17 [M+H]⁺.

### Step 4:

Under an ice bath, NaBH₄ (22 mg, 0.59 mmol) was added to a solution of compound 29752A3 (0.32 g, 0.49 mmol) in tetrahydrofuran (5 mL), and the mixture was stirred at room temperature for 1 h. Water was added to the reaction mixture, and the resulting mixture was extracted with ethyl acetate. The organic phase was concentrated to remove the solvent, and the residue was separated and purified by silica gel column chromatography (ethyl acetate/petroleum ether) and subjected to resolution by SFC (instrument: WATERS 150 preparative SFC (SFC-26); chromatography column: ChiralPak IG, 250 × 30 mm I.D., 10 µm; mobile phases: CO₂ as phase A, ethanol (0.1% NH₃H₂O) as phase B; gradient: B 40%; flow rate: 150 mL/min; back pressure: 100 bar) to give EVO29752P1 (66 mg, yield: 20.6%, RT = 1.342 min) (LCMS (ESI) m/z = 645.17 [M+H]⁺; ¹H NMR (400 MHz, DMSO-*d*₆) δ 9.33 (s, 1H), 8.86 (d, *J* = 4.4 Hz, 1H), 8.48 (d, *J =* 8.6 Hz, 1H), 7.61 (t, *J =* 53.1 Hz, 1H), 7.21 - 7.09 (m, 1H), 6.21 - 5.50 (m, 2H), 4.69 (d, *J = 9.5* Hz, 1H), 4.35 (d, *J =* 7.5 Hz, 1H), 4.26 - 4.12 (m, 1H), 3.85 (d, *J = 11.0* Hz, 1H), 3.73 (t, *J* = 12.6 Hz, 2H), 3.39 - 3.33 (m, 1H), 3.25 - 3.00 (m, 1H), 1.49 - 1.41 (m, 3H), 1.32 (q, *J =* 3.4, 2.6 Hz, 4H), 1.12 - 0.98 (m, 6H)) and

EVO29752P2 (137 mg, yield: 42.8%, RT = 1.819 min) (LCMS (ESI) m/z = 645.17 [M+H]⁺; ¹H NMR (400 MHz, DMSO-*d*₆) δ 9.33 (s, 1H), 8.88 (d, *J =* 0.9 Hz, 1H), 8.48 (d, *J =* 13.1 Hz, 1H), 7.60 (t, *J =* 53.1 Hz, 1H), 7.14 (d, *J =* 14.5 Hz, 1H), 5.97 (t, *J =* 57.3 Hz, 1H), 5.34 (t, *J =* 9.8 Hz, 1H), 4.83 - 4.07 (m, 3H), 3.87 (t, *J =* 14.7 Hz, 1H), 3.73 (d, *J =* 12.2 Hz, 2H), 3.44 - 3.33 (m, 1H), 3.14 (dt, *J =* 77.5, 11.0 Hz, 1H), 1.44 (p, *J =* 5.5, 4.9 Hz, 3H), 1.31 (td, *J =* 7.5, 6.7, 4.7 Hz, 4H), 1.01 (d, *J* = 23.8 Hz, 6H)).

### Example 26

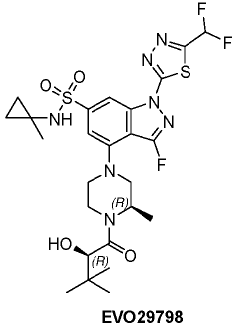

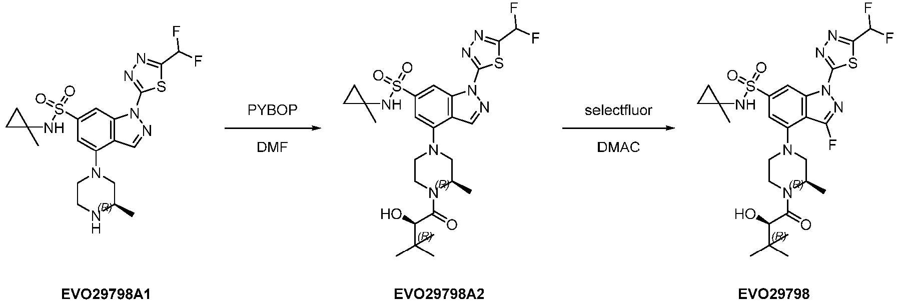

### Step 1:

(*R*)-2-Hydroxy-3,3-dimethylbutyric acid (82.0 mg, 620.40 µM) was dissolved in 5 mL of DMF, and then PyBOP (322.8 mg, 620.40 µM) and DIPEA (156.9 mg, 1.55 mM) were added. The mixture was stirred at room temperature for 10 min, and **EVO29798A1** (250.0 mg, 517.00 µM) was added. The mixture was allowed to react at room temperature for 17 h. Water was added, and the mixture was extracted twice with ethyl acetate. The organic phases were combined, washed once with saturated brine, dried over anhydrous sodium sulfate, filtered, and concentrated under reduced pressure. The residue was subjected to preparative medium-pressure chromatography (PE:EA = 1:1). The fractions were evaporated to dryness to give a yellow solid (130.0 mg, yield: 42%). LCMS (ESI) m/z = 598.1 [M+H]⁺.

### Step 2:

**EVO29798A2** (130.0 mg, 217.50 µmol) was dissolved in DMAC (4 mL), and then selectfluor (123.3 mg, 348.00 µmol) was added. The mixture was allowed to react at 60 °C for 6 h under an argon atmosphere. Water was added, and then a solid was precipitated. The mixture was filtered under vacuum to give a yellow solid (140.0 mg), which was then separated and purified by preparative high performance liquid chromatography to give **EVO29798** as a yellow solid (24.4 mg, yield: 18%). LCMS (ESI) m/z = 616.2 [M+H]⁺. ¹H NMR (400 MHz, DMSO-*d*₆) δ 8.91 (d, *J* = 3.6 Hz, 1H), 8.60 (s, 1H), 8.53 (d, *J* = 4.6 Hz, 1H), 7.60 (t, *J* = 53.1 Hz, 1H), 5.18 - 4.77 (m, 1H), 4.72 (s, 1H), 4.54 - 4.18 (m, 1H), 4.16 - 3.98 (m, 1H), 3.72 - 3.35 (m, 3H), 3.29 - 3.10 (m, 1H), 1.45 (d, *J* = 6.5 Hz, 1H), 1.30 (d, *J* = 6.7 Hz, 2H), 1.15 (s, 3H), 0.96 (s, 9H), 0.76 - 0.62 (m, 2H), 0.51 - 0.39 (m, 2H).

### Example 27

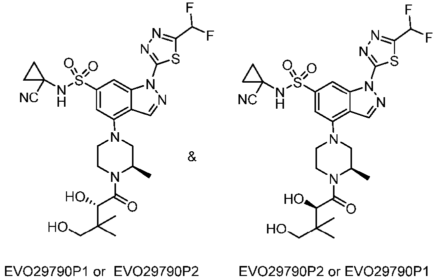

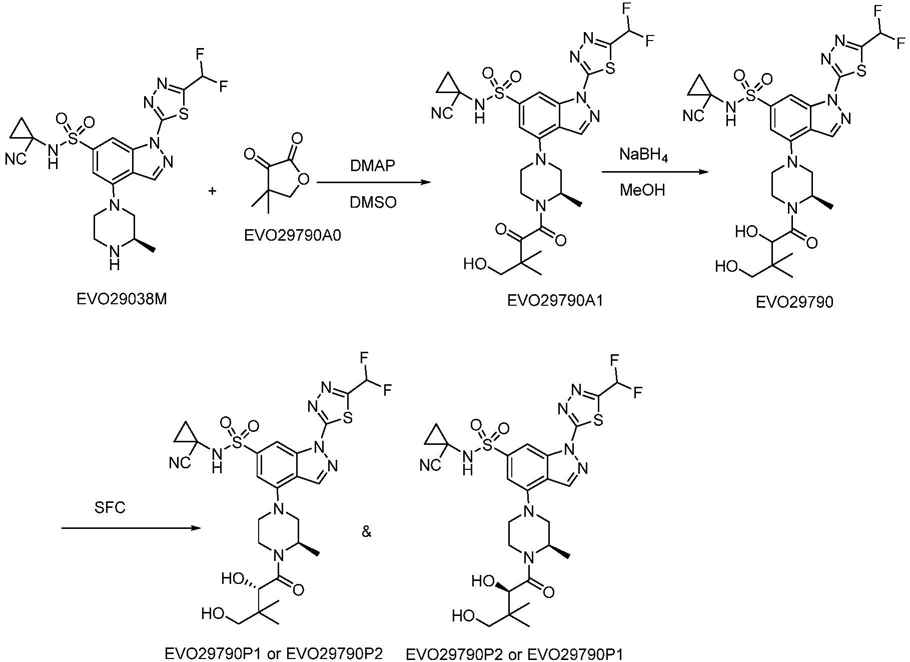

### Step 1:

A solution of EVO29038M (670 mg, 1.35 mmol), EVO29790A0 (433.96 mg, 3.39 mmol), and DMAP (49.65 mg, 0.406 mmol) in DMSO (10 mL) was allowed to react at 110 °C overnight and then cooled to room temperature. Water was added to the reaction mixture, and the resulting mixture was extracted with ethyl acetate. The organic phase was concentrated to remove the solvent, and the residue was purified by silica gel column chromatography (ethyl acetate/petroleum ether) to give product EVO29790A1 (300 mg, yield: 35.56%). LCMS (ESI) m/z = 623.22 [M+H]⁺.

### Step 2:

To compound EVO29790A1 (300.00 mg, 0.482 mmol) in methanol (10 mL) was slowly added sodium borohydride (36.45 mg, 0.964 mmol), and the mixture was allowed to react at room temperature for 1 h. Saturated aqueous ammonium chloride (20 mL) was added to the reaction mixture, and the resulting mixture was extracted with ethyl acetate (50 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 2:1 to 1:2) to give product EVO29790 (180.00 mg, yield: 59.81%). LCMS (ESI) m/z = 625.41 [M+H]⁺.

### Step 3:

Compound EVO29790 (180.00 mg) was subjected to resolution by supercritical fluid chromatography (SFC) (chromatography column: ChiralCel OX, 250 × 30 mm I.D., 10 µm; mobile phases: CO₂ as phase A, ethanol (0.1% NH₃H₂O) as phase B; gradient: B 35%; flow rate: 150 mL/min; back pressure: 100 bar; column temperature: 38 °C; wavelength: 220 nm; cycle time: about 5 min; injection volume: 2.00 mL) to give EVO29790P1 (65.30 mg, retention time: 1.712 min) and EVO29790P2 (35.42 mg, retention time: 2.722 min). LCMS (ESI) m/z = 625.2 [M+H]⁺.

EVO29790P1 ¹H NMR (400 MHz, DMSO-d6) δ 9.33 (s, 1H), 8.90 (s, 1H), 8.50 (d, J = 8 Hz, 1H), 7.63 (t, J = 52 Hz, 1H), 7.17 (d, J = 8 Hz, 1H), 4.85 - 4.74 (m, 1H), 4.69 - 4.43 (m, 2H), 4.36 - 4.11 (m, 1H), 3.94 - 3.74 (m, 3H), 3.32-3.02 (m, 2H), 1.49-1.40 (m, 3H), 1.37-1.31 (m, 4H), 0.90 - 0.88 (m, 6H).

EVO29790P2 ¹H NMR (400 MHz, DMSO-d6) δ 9.36 (s, 1H), 8.89 (s, 1H), 8.50 (s, 1H), 7.63 (t, J = 52 Hz, 1H), 7.17 (s, 1H), 4.82 (d, J = 4Hz, 1H), 4.75-4.66 (m, 2H), 4.41 - 4.23 (m, 3H), 3.89-3.86 (m, 1H), 3.80-3.63 (m, 2H), 3.25 - 3.17 (m, 2H), 1.49 - 1.46 (m, 3H), 1.35-1.30 (m, 5H), 0.91-0.89 (m, 6H).

### Example 28

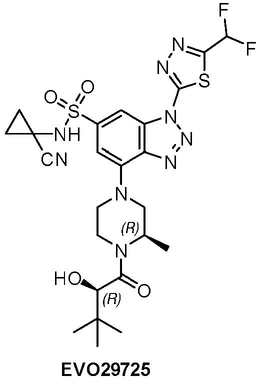

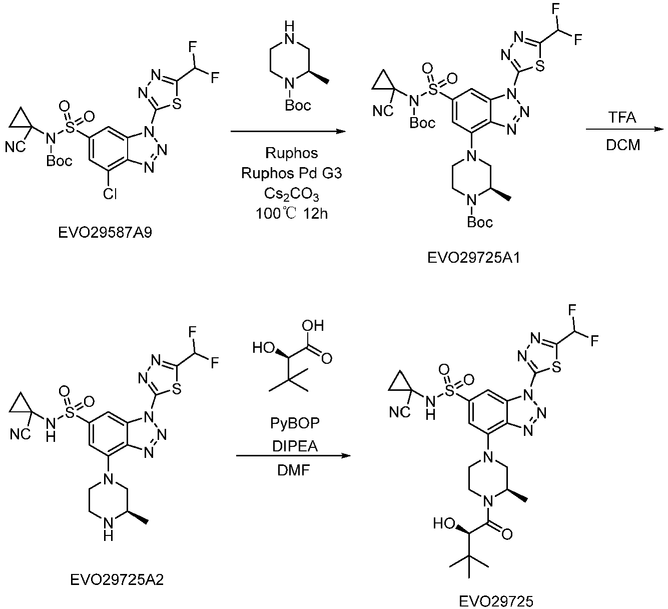

### Step 1:

To a solution of compound EVO29587A9 (500.00 mg, 0.94 mmol) in 1,4-dioxane (50 mL) were added (R)-1-N-Boc-2-methylpiperazine (282.38 mg, 0.41 mmol), 2-dicyclohexylphosphino-2',6'-diisopropoxy-1,1'-biphenyl (175.44 mg, 0.38 mmol), methanesulfonato(2-dicyclohexylphosphino-2',6'-diisopropoxy-1,1'-biphenyl)(2-amino-1,1'-biphenyl-2-yl)palladium(II) (157.23 mg, 0.19 mmol), and cesium carbonate (1.53 g, 4.70 mmol). The mixture was stirred at 100 °C for 12 h under an argon atmosphere, then cooled to room temperature, and filtered to collect a filtrate. The filtrate was concentrated by rotary evaporation to remove the solvent, and the residue was separated and purified by silica gel column chromatography (petroleum ether/ethyl acetate = 1:1) to give EVO29725A1 as a yellow solid (0.52 g, yield: 79.67%). LCMS (ESI) m/z = 696.2 [M+H]⁺.

### Step 2:

To a solution of compound EVO29725A1 (0.52 g, 0.75 mmol) in dichloromethane (3 mL) was added trifluoroacetic acid (6 mL) at room temperature, and the reaction mixture was stirred at 25 °C for 2 h. After the reaction was completed, the reaction mixture was adjusted to pH 8 with saturated sodium bicarbonate and then extracted with ethyl acetate (20 mL × 2). The organic phases were combined, dried over anhydrous sodium sulfate, and concentrated by rotary evaporation to remove the solvent to give EVO29725A2 as a brown solid (0.37 g, yield: 95.67%).

### Step 3:

To a solution of compound EVO29725A2 (0.10 g, 0.20 mmol) in N,N-dimethylformamide (5 mL) were sequentially added (R)-2-hydroxy-3,3-dimethylbutyric acid (32.00 mg, 0.24 mmol), *N,N-*diisopropylethylamine (64.35 mg, 0.60 mmol), and (benzotriazol-1-yloxy)tripyrrolidinophosphonium hexafluorophosphate (130.00 mg, 0.24 mmol) at room temperature. The reaction mixture was stirred at 25 °C for 16 h. After the reaction was completed, water (20 mL) was added to quench the reaction, and the mixture was extracted with ethyl acetate (20 mL × 2). The organic phases were combined, dried over anhydrous sodium sulfate, and concentrated by rotary evaporation to remove the solvent, and the residue was separated and purified by preparative high performance liquid chromatography to give EVO29725 as a bright yellow solid (84.00 mg, yield: 64.89%, purity: 98.90%). ¹H NMR (400 MHz, DMSO-d6) δ 8.21 (dd, J = 7.2, 1.4 Hz, 1H), 7.22 (dd, *J* = 11.9, 1.5 Hz, 1H), 4.93 (d, *J* = 7.7 Hz, 1H), 4.77 (s, 1H), 4.47 (dd, *J* = 80.3, 11.9 Hz, 3H), 4.14 (d, *J* = 7.7 Hz, 1H), 3.66 (dd, J = 12.5, 3.6 Hz, 1H), 3.41 (d, *J* = 10.6 Hz, 1H), 3.21 (q, *J* = 12.3 Hz, 1H), 1.52 - 1.45 (m, 2H), 1.42 - 1.31 (m, 3H), 1.25 (d, *J* = 6.6 Hz, 2H), 0.99 (d, *J* = 4.2 Hz, 9H).

### Example 29

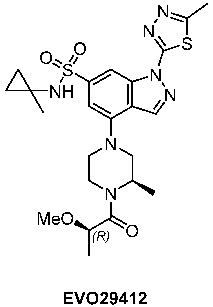

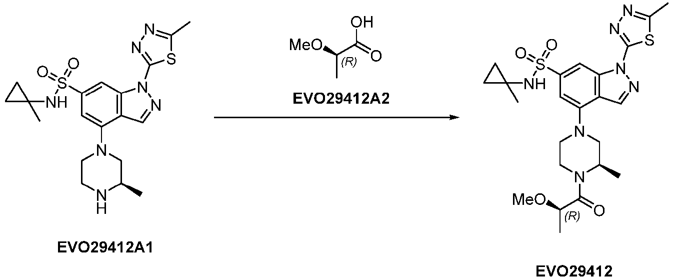

Compound EVO29412A1 (100 mg, 0.23 mmol) was dissolved in N,N-dimethylformamide (3 mL), and then EVO29412A2 (36 mg, 0.34 mmol), *O*-(7-azabenzotriazol-1-yl)-*N*,*N*,*N*',*N*'-tetramethyluronium hexafluorophosphate (129 mg, 0.34 mmol), and N,N-diisopropylethylamine (149 mg, 1.15 mmol) were added. The mixture was allowed to react at room temperature for 2 h. Water (50 mL) was added to the reaction mixture, and the resulting mixture was extracted with ethyl acetate (30 mL × 2). The organic phases were combined, washed once with saturated brine (50 mL), dried over anhydrous sodium sulfate, filtered, and concentrated, and the residue was separated and purified by silica gel column chromatography (petroleum ether/ethyl acetate = 1:3) to give EVO29412 (55.1 mg, yield: 43.08%). LCMS (ESI) m/z = 534.1 [M+H]⁺. ¹H NMR (DMSO-*d*₆, 400 MHz): δ 8.72 (s, 1H), 8.44 (s, 1H), 8.27 (s, 1H), 7.10 (s, 1H), 4.69 (s, 1H), 4.22 (t, *J* = 59.8 Hz, 2H), 3.80 (d, *J =* 11.2 Hz, 1H), 3.70 (d, *J =* 12.4 Hz, 2H), 3.30 - 3.21 (m, 4H), 3.17 - 2.87 (m, 1H), 2.75 (s, 3H), 1.45 (s, 1H), 1.28 (t, *J =* 10.6 Hz, 5H), 1.07 (s, 3H), 0.72 - 0.58 (m, 2H), 0.46 - 0.33 (m, 2H).

### Example 30

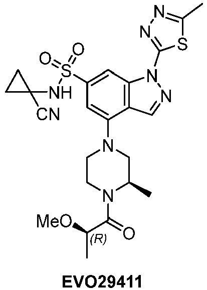

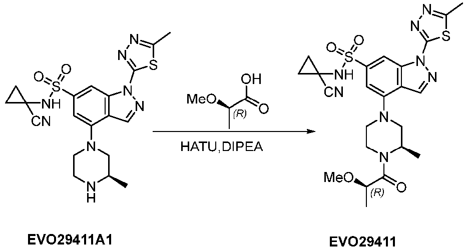

### Step 1:

To a solution of compound EVO29411A1 (100 mg, 0.22 mmol) in N,N-dimethylformamide (5 mL) were added *O*-(7-azabenzotriazol-1-yl)-*N*,*N*,*N*',*N*'-tetramethyluronium hexafluorophosphate (125 mg, 0.33 mmol), N,N-diisopropylethylamine (140 mg, 1.09 mmol), and 2-methoxypropanoic acid (50 mg, 0.44 mmol) at room temperature, and the reaction mixture was stirred at 25 °C for 1 h. Water (30 mL) was added to the reaction mixture, and the resulting mixture was extracted with ethyl acetate (30 mL × 3). The organic phases were combined, dried over anhydrous sodium sulfate, filtered, and concentrated, and the residue was separated and purified by silica gel column chromatography (petroleum ether/ethyl acetate = 1:2) to give EVO29411 as a pale yellow solid (60 mg, yield: 50.80%). LCMS (ESI) m/z = 545.1 [M+H]⁺. ¹H NMR (DMSO-*d*₆, 400 MHz) δ 9.31 (s, 1H), 8.76 (s, 1H), 8.48 (s, 1H), 7.11 (s, 1H), 4.40 (dd, *J =* 188.4, 62.5 Hz, 3H), 3.84 (d, *J =* 11.4 Hz, 1H), 3.73 (d, *J* = 12.5 Hz, 1H), 3.28 - 2.90 (m, 6H), 2.76 (s, 3H), 1.49 - 1.36 (m, 3H), 1.34 - 1.24 (m, 7H).

### Example 31

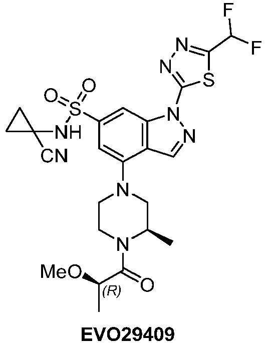

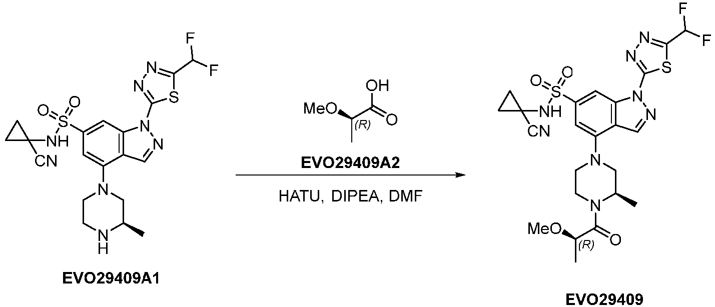

### Step 1:

Compound EVO29409A1 (100 mg, 0.20 mmol), compound EVO29409A2 (31.5 mg, 0.30 mmol), *O*-(7-azabenzotriazol-1-yl)-*N*,*N*,*N*',*N*'-tetramethyluronium hexafluorophosphate (115.3 mg, 0.30 mmol), and N,N-diisopropylethylamine (78.7 mg, 0.61 mmol) were dissolved in N,N-dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed with water (20 mL × 2) and then with saturated brine (20 mL), dried over anhydrous sodium sulfate, and concentrated to dryness by rotary evaporation under vacuum to give a crude product, which was then purified by silica gel column chromatography (petroleum ether/ethyl acetate = 1:1) to give EVO29409 (63.3 mg, yield: 54.50%). LCMS (ESI) m/z = 581.1 [M+H]⁺. ¹H NMR (DMSO-*d*₆, 400 MHz): δ 9.32 (s, 1H), 8.86 (s, 1H), 8.48 (s, 1H), 7.60 (t, *J* = 53.1 Hz, 1H), 7.15 (s, 1H), 4.63 (d, *J* = 43.0 Hz, 1H), 4.16 (d, *J* = 83.2 Hz, 2H), 3.80 (dd, *J =* 51.6, 12.0 Hz, 2H), 3.29 - 2.94 (m, 5H), 1.49 - 1.19 (m, 11H).

### Example 32

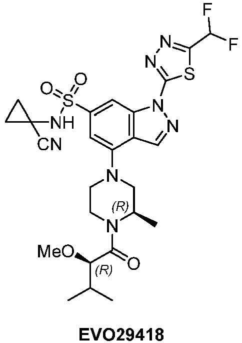

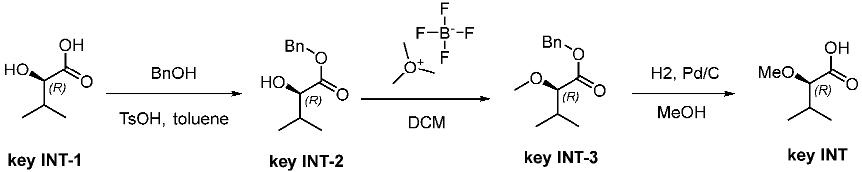

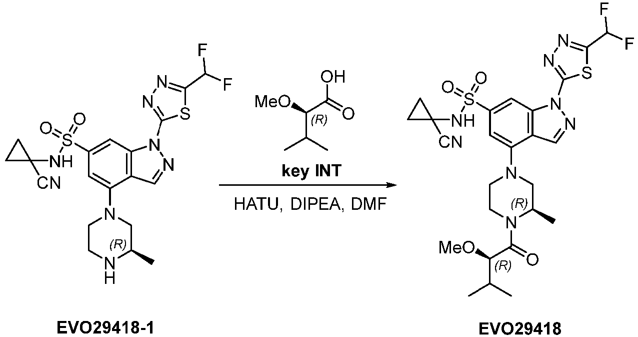

### Step 1:

Compound key INT-1 (2.0 g, 16.9 mmol), benzyl alcohol (5 mL), and p-toluenesulfonic acid monohydrate (321.5 mg, 1.69 mmol) were dissolved in a toluene solution (20 mL), and the reaction mixture was refluxed for reaction at 115 °C for 16 h. The reaction mixture was then cooled to room temperature and concentrated under reduced pressure, and the residue was purified by silica gel column chromatography (petroleum ether:ethyl acetate = 20:1) to give key INT-2 as a colorless oil (2.7 g, yield: 7.1%). ¹H NMR (400 MHz, DMSO) δ 7.54 - 7.20 (m, 5H), 5.33 (d, *J* = 5.9 Hz, 1H), 5.23 - 5.03 (m, 2H), 3.85 (t, *J* = 5.4 Hz, 1H), 2.01 - 1.84 (m, 1H), 0.83 (dd, *J =* 21.1, 6.8 Hz, 6H).

### Step 2:

Compound key INT-2 (2.7 g, 13.0 mmol) and 1,8-bis(dimethylamino)naphthalene (5.6 g, 25.9 mmol) were dissolved in a dichloromethane solution (100 mL), and then trimethyloxonium tetrafluoroborate (3.8 g, 25.9 mmol) was added. The reaction mixture was allowed to react at room temperature for 16 h. The reaction mixture was then filtered, washed with dichloromethane (50 mL × 2), and concentrated under reduced pressure, and the residue was purified by silica gel column chromatography (petroleum ether:ethyl acetate = 20:1) to give key INT-3 as a colorless oil (2.7 g, yield: 93.4%). ¹H NMR (400 MHz, DMSO) δ 7.48 - 7.23 (m, 5H), 5.17 (q, *J =* 12.3 Hz, 2H), 3.62 (d, *J =* 5.3 Hz, 1H), 3.26 (s, 3H), 2.06 - 1.86 (m, 1H), 0.85 (dd, *J =* 15.1, 6.9 Hz, 6H).

### Step 3:

To compound key INT-3 (2.7 g, 12.1 mmol) in methanol (50 mL) was added palladium on carbon (2.0 g), and the reaction mixture was purged three times with hydrogen and allowed to react at room temperature for 2 h. The reaction mixture was then directly filtered, washed twice with methanol (50 mL), and concentrated under reduced pressure to give a crude product of key INT (1.54 g, yield: 96.3%), which was directly used in the next step.

### Step 4:

Compound EVO29418-1 (150 mg, 0.30 mmol), compound key INT (60.2 mg, 0.45 mmol), O-(7-azabenzotriazol-1-yl)-*N*,*N*,*N*',*N*' -tetramethyluronium hexafluorophosphate (172.9 mg, 0.45 mmol), and *N,N-*diisopropylethylamine (117.4 mg, 0.91 mmol) were dissolved in *N,N-*dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:1) to give EVO29418 as a yellow solid (91.5 mg, yield: 50.1%). LCMS (ESI) m/z = 609.1 [M+H]⁺. ¹H NMR (400 MHz, DMSO) δ 9.32 (s, 1H), 8.85 (s, 1H), 8.48 (s, 1H), 7.60 (t, *J =* 53.1 Hz, 1H), 7.15 (s, 1H), 4.71 (s, 1H), 4.35 (dd, *J =* 73.9, 13.0 Hz, 1H), 3.86 (d, *J =* 10.8 Hz, 1H), 3.72 (t, *J =* 13.4 Hz, 2H), 3.34 - 3.10 (m, 6H), 1.96 (d, *J =* 6.5 Hz, 1H), 1.44 (t, *J* = 6.6 Hz, 3H), 1.32 (d, *J* = 3.2 Hz, 4H), 0.91 (dd, *J =* 40.1, 6.5 Hz, 6H).

### Example 33

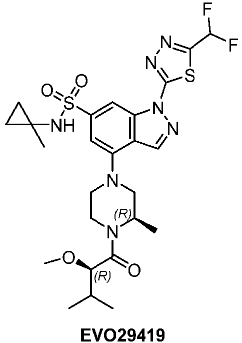

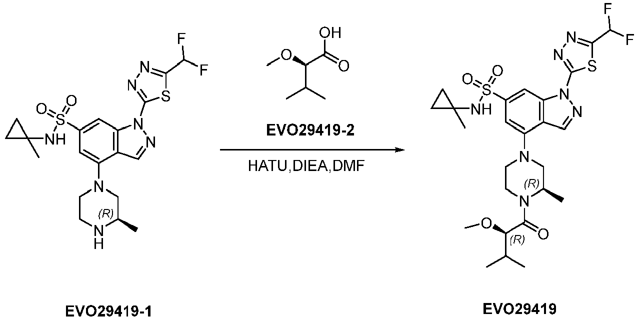

### Step 1:

To a solution of compound EVO29419-1 (80 mg, 0.16 mmol) in N,N-dimethylformamide (2 mL) were added *O*-(7-azabenzotriazol-1-yl)-*N*,*N*,*N*',*N*'-tetramethyluronium hexafluorophosphate (70 mg, 0.25 mmol), *N,N-*diisopropylethylamine (64 mg, 0.50 mmol), and EVO29419-2 (26 mg, 0.20 mmol) at room temperature, and the reaction mixture was stirred at 25 °C for 2 h. The reaction mixture was concentrated to dryness by rotary evaporation to remove the solvent. Water (20 mL) was added, and the mixture was extracted with ethyl acetate (3 × 20 mL), followed by liquid separation. The organic phases were combined, dried over anhydrous sodium sulfate, and separated and purified by silica gel column chromatography (petroleum ether/ethyl acetate = 2:1) to give EVO29419 as a yellow solid (34.7 mg, yield: 36%). LCMS (ESI) m/z = 598.7 [M+H]⁺. ¹H NMR (400 MHz, DMSO) δ 8.81 (s, 1H), 8.44 (s, 1H), 8.31 (s, 1H), 7.60 (t, *J* = 53.1 Hz, 1H), 7.14 (s, 1H), 4.72 (s, 1H), 4.35 (dd, *J* = 72.3, 13.1 Hz, 1H), 3.88 - 3.59 (m, 4H), 3.29 - 2.95 (m, 5H), 1.98 (d, *J* = 7.2 Hz, 1H), 1.43 (s, 1H), 1.32 (d, *J =* 6.1 Hz, 2H), 1.08 (s, 3H), 0.96 (d, *J* = 6.3 Hz, 3H), 0.86 (d, *J =* 6.7 Hz, 3H), 0.66 (s, 2H), 0.40 (s, 2H).

### Example 34

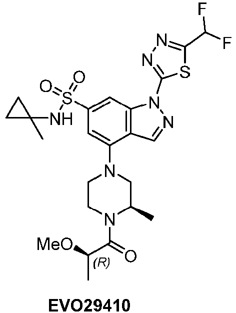

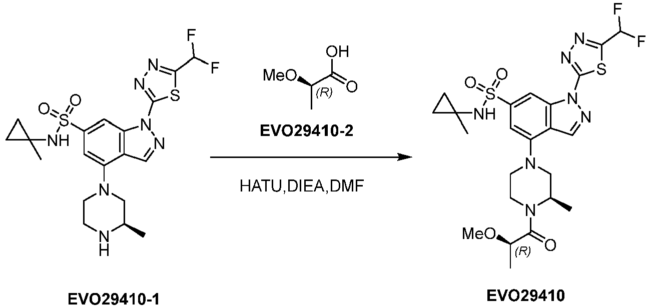

### Step 1:

Compound EVO29410-1 (100.0 mg, 0.20 mmol) was dissolved in N,N-dimethylformamide (2 mL), and then *O*-(7-azabenzotriazol-1-yl)-*N*,*N*,*N*',*N*'-tetramethyluronium hexafluorophosphate (115.0 mg, 0.30 mmol) and N,N-diisopropylethylamine (130.0 mg, 0.60 mmol) were added, followed by addition of EVO29410-2 (35.0 mg, 0.20 mmol). The mixture was stirred for reaction at room temperature for 2 h. Water (30 mL) was added, and the mixture was extracted with ethyl acetate (20 mL × 2). The organic phases were combined, washed with saturated brine (20 mL), dried over anhydrous sodium sulfate, filtered, and concentrated, and the residue was separated and purified by column chromatography (petroleum ether/ethyl acetate = 1:1) to give compound EVO29410 (5 mg, yield: 4%). LCMS (ESI) m/z = 570 [M+H]⁺. ¹H NMR (400 MHz, DMSO-*d*₆) δ 8.84 (s, 1H), 8.45 (s, 1H), 8.33 (s, 1H), 7.61 (t, *J =* 53.0 Hz, 1H), 7.15 (s, 1H), 4.64 (d, *J* = 42.6 Hz, 1H), 4.17 (d, *J* = 82.9 Hz, 2H), 3.78 (dd, *J =* 46.2, 5H), 3.26 (s, 3H), 1.45 (s, 6H), 1.09 (s, 3H), 0.67 (s, 2H), 0.42 (s, 2H).

### Example 35

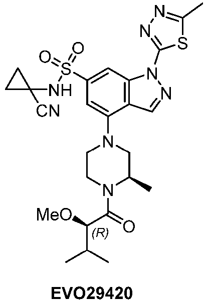

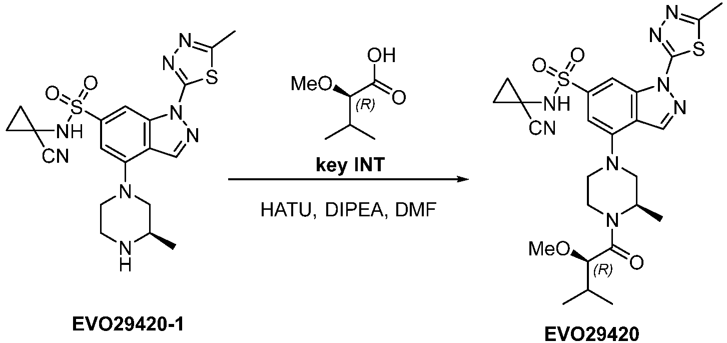

### Step 1:

Compound EVO29420-1 (100.0 mg, 0.22 mmol), compound key INT (43.2 mg, 0.33 mmol), *O-*(7-azabenzotriazol-1-yl)-*N*,*N*,*N*',*N*'-tetratmethyluronium hexafluorophosphate (124.3 mg, 0.33 mmol), and N,N-diisopropylethylamine (84.4 mg, 0.65 mmol) were dissolved in N,N-dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:1) to give EVO29420 as a yellow solid (35 mg, yield: 28%). LCMS (ESI) m/z = 573.1 [M+H]⁺. ¹H NMR (400 MHz, DMSO-*d*₆) δ 9.27 (s, 1H), 8.75 (s, 1H), 8.49 (s, 1H), 7.11 (s, 1H), 4.72 (s, 1H), 4.36 (dd, *J =* 73.5, 12.8 Hz, 1H), 3.90 - 3.66 (m, 3H), 3.27 (s, 6H), 2.75 (s, 3H), 1.96 (d, *J =* 6.3 Hz, 1H), 1.44 (d, *J* = 2.4 Hz, 3H), 1.33 (d, *J =* 7.4 Hz, 4H), 0.91 (dd, *J =* 39.9, 6.5 Hz, 6H).

### Example 36

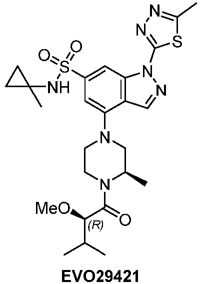

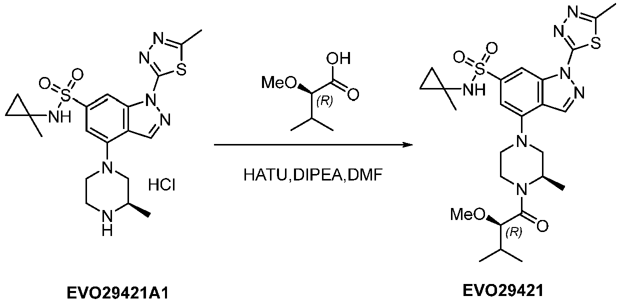

### Step 1:

To EVO29421A1 (100.0 mg, 0.22 mmol), (S)-2-methoxy-3-methylbutyric acid (40.0 mg, 0.30 mmol) and diisopropylethylamine (173.4 mg, 1.34 mmol) dissolved in N,N-dimethylformamide (3 mL) was added *O*-(7-azabenzotriazol-1-yl)-*N*,*N*,*N*',*N*' -tetramethyluronium hexafluorophosphate (114.0 mg, 0.30 mmol) in portions, and the reaction mixture was stirred at room temperature for 1.5 h. Water (70 mL) was added, and the mixture was extracted with ethyl acetate (2 × 50 mL). The organic phases were combined, dried over anhydrous sodium sulfate, filtered, and concentrated, and the residue was separated and purified by silica gel column chromatography (dichloromethane:methanol = 100/1) to give EVO29421 as a yellow oil (20 mg, yield: 16%). LCMS (ESI) m/z = 562.1 [M+H]⁺. ¹H NMR(400 MHz, DMSO-*d*₆): δ 8.71 (s, 1H), 8.45 (s, 1H), 8.26 (s, 1H), 7.10 (s, 1H), 4.73 (s,1H), 4.52 - 4.19 (m, 1H), 3.89 - 3.57 (m, 4H), 3.27 (s, 3H), 3.11 (d, *J*=11.9 Hz, 1H), 2.75 (s, 3H), 1.96 (dd, *J*=14.0*, J*=7.1 Hz, 1H), 1.44 (s, 1H), 1.31 (dd, *J*=21.6, 11.0 Hz, 3H), 1.07 (s, 3H), 0.96 (d, *J*=6.4 Hz, 3H), 0.86 (d, *J*=6.7 Hz, 3H), 0.64 (d, *J*=11.8 Hz, 2H), 0.41 (d, *J=5.8* Hz, 2H).

### Example 37

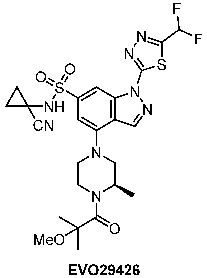

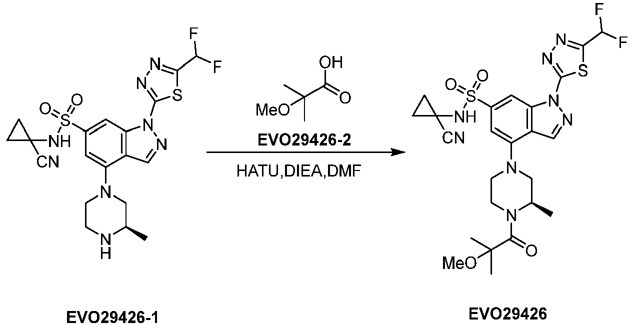

### Step 1:

Compound EVO29426-1 (100.0 mg, 0.20 mmol) was dissolved in N,N-dimethylformamide (2 mL), and then *O*-(7-azabenzotriazol-1-yl)-N,N,*N*'*,*N'-tetramethyluronium hexafluorophosphate (115.0 mg, 0.30 mmol) and N,N-diisopropylethylamine (130.0 mg, 0.60 mmol) were added, followed by addition of EVO29426-2 (30.0 mg, 0.20 mmol). The mixture was stirred for reaction at room temperature for 2 h. Water (30 mL) was added, and the mixture was extracted with ethyl acetate (20 mL × 2). The organic phase was washed with saturated brine (20 mL), dried over anhydrous sodium sulfate, filtered, and concentrated, and the residue was separated and purified by column chromatography (petroleum ether:ethyl acetate = 3:1) to give compound EVO29426 (11 mg, yield: 9%). LCMS (ESI) m/z = 595.1 [M+H]⁺. ¹H NMR (400 MHz, DMSO-*d*₆) δ 8.87 (s, 1H), 8.47 (s, 1H), 7.60 (t, *J =* 53.1 Hz, 1H), 7.15 (s, 1H), 4.76 - 4.63 (m, 1H), 4.01 - 3.60 (m, 3H), 3.22 (s, 3H), 3.18 (s, 3H), 1.53 - 1.25 (m, 13H).

### Example 38

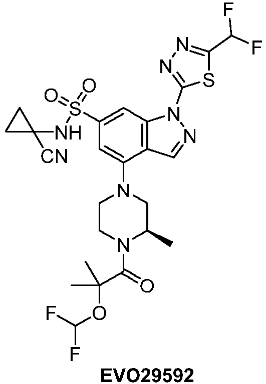

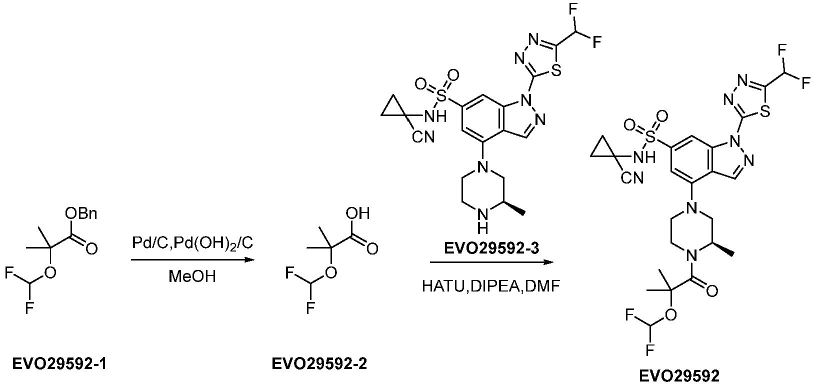

### Step 1:

At room temperature, compound EVO29592-1 (460.0 mg, 1.88 mmol) was dissolved in methanol (8 mL), and then palladium on carbon (10%, 30.0 mg, 281.90 µmol) and palladium hydroxide on carbon (10%, 30.0 mg, 213.62 µmol) were added. The mixture was purged three times with hydrogen and allowed to react at room temperature overnight. After the reaction was completed, the reaction mixture was filtered through celite, and the filter cake was washed twice with methanol (10 mL). The filtrates were combined and concentrated to give EVO29592-2 as a white solid (280.0 mg, yield: 96.4%). LCMS (ESI) m/z = 153.1 [M-H]⁻.

### Step 2:

At room temperature, compound EVO29592-2 (280.0 mg, 1.81 mmol) and EVO29592-3 (825.0 mg, 1.67 mmol) were dissolved in *N,N*-dimethylformamide (10 mL), and then *O*-(7-azabenzotriazol-1-yl)-*N,N,N',N'*-tetramethyluronium hexafluorophosphate (825.0 mg, 2.17 mmol), and *N,N*-diisopropylethylamine (650.0 mg, 5.02 mmol) were added. The mixture was allowed to react at room temperature for 2 h. Water (60 mL) was added to the reaction mixture to quench the reaction, and the resulting mixture was stirred for ten minutes. A solid was then precipitated, and the mixture was filtered. The filter cake was washed with water (15 mL), dried, and separated and purified by preparative liquid chromatography to give EVO29592 as a yellow solid (650.0 mg, yield: 61.7%). LCMS (ESI) m/z = 631.2 [M+H]⁺; HPLC: 99.6%; ¹H NMR (400 MHz, DMSO-*d*₆): δ 9.34 (s, 1H), 8.87 (s, 1H), 8.49 (s, 1H), 7.61 (t, *J =* 53.0 Hz, 1H), 7.14 (s, 1H), 6.82 (t, *J* = 74.2 Hz, 1H), 4.96 - 4.62 (m, 1H), 4.44 - 4.30 (m, 1H), 3.85 (d, *J* = 12.0 Hz, 1H), 3.75 (d, *J* = 12.4 Hz, 2H), 3.30 - 2.86 (m, 2H), 1.63 - 1.51 (m, 6H), 1.50 - 1.39 (m, 3H), 1.39 - 1.26 (m, 4H).

### Example 39

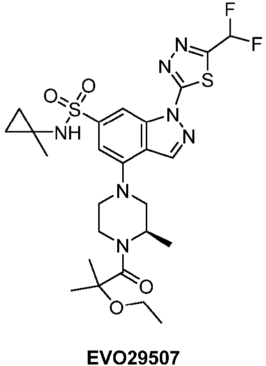

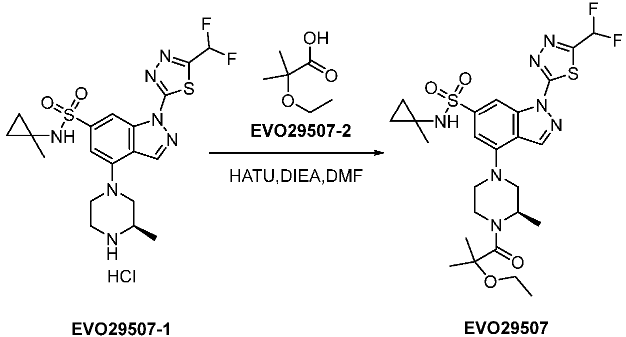

Compound EVO29507-1 (50 mg, 0.10 mmol), compound EVO29507-2 (17 mg, 0.12 mmol), *O-*(7-azabenzotriazol-1-yl)-*N*,*N*,*N*',*N*'-tetramethyluronium hexafluorophosphate (59 mg, 0.15 mmol), and *N,N-*diisopropylethylamine (67 mg, 0.52 mmol) were dissolved in N,N-dimethylformamide (2 mL), and the reaction mixture was allowed to react at room temperature for 2 h. Water (20 mL) was added to the reaction mixture, and the resulting mixture was extracted with ethyl acetate (3 × 20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:1) to give EVO29507 as a yellow solid (20 mg, yield: 32.3%). LCMS (ESI) m/z = 598.7 [M+H]⁺. ¹H NMR (400 MHz, DMSO) δ 8.84 (s, 1H), 8.45 (d, *J =* 12.5 Hz, 1H), 8.31 (s, 1H), 7.60 (t, *J =* 53.1 Hz, 1H), 7.15 (d, *J =* 10.3 Hz, 1H), 5.36 - 4.37 (m, 2H), 3.93 - 3.66 (m, 3H), 3.50 - 3.34 (m, 2H), 3.18 (dd, *J =* 40.2, 30.5 Hz, 2H), 1.48 (s, 1H), 1.38 (d, *J =* 14.4 Hz, 6H), 1.30 (d, *J =* 6.5 Hz, 2H), 1.16 (t, *J =* 6.9 Hz, 3H), 1.08 (s, 3H), 0.66 (s, 2H), 0.41 (s, 2H).

### Example 40

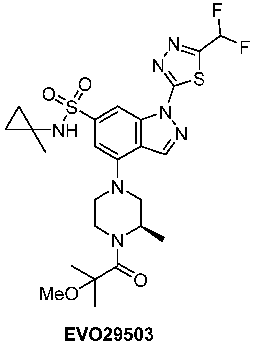

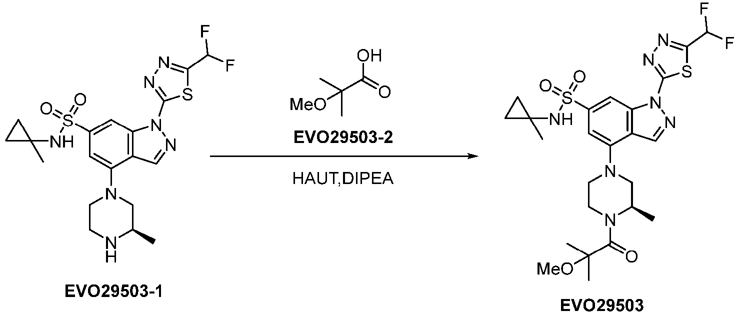

Compound EVO29503-1 (100 mg, 0.21 mmol), compound EVO29503-2 (55.2 mg, 0.42 mmol), *O*-(7-azabenzotriazol-1-yl)-*N*,*N*,*N*',*N*'-tetramethyluronium hexafluorophosphate (152.9 mg, 0.42 mmol), and N,N-diisopropylethylamine (117.4 mg, 0.91 mmol) were dissolved in N,N-dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 1:1) to give EVO29503 as a yellow solid (55 mg, yield: 45.8%). LCMS (ESI) m/z = 584.0 [M+H]⁺. ¹H NMR (400 MHz, DMSO) δ 8.83 (s, 1H), 8.43 (s, 1H), 8.29 (s, 1H), 7.60 (t, *J =* 53.1 Hz, 1H), 7.14 (s, 1H), 5.23 - 4.36 (m, 2H), 3.94 - 3.62 (m, 3H), 3.33 (s, 1H), 3.20 (d, *J =* 14.9 Hz, 4H), 1.47 (s, 1H), 1.37 (d, *J =* 12.5 Hz, 6H), 1.31 (d, *J =* 6.5 Hz, 2H), 1.08 (s, 3H), 0.71 - 0.60 (m, 2H), 0.47 - 0.30 (m, 2H).

### Example 41

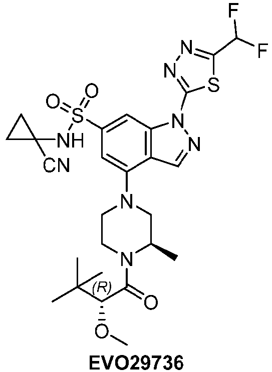

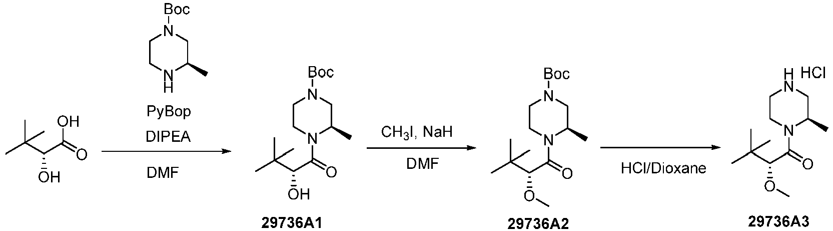

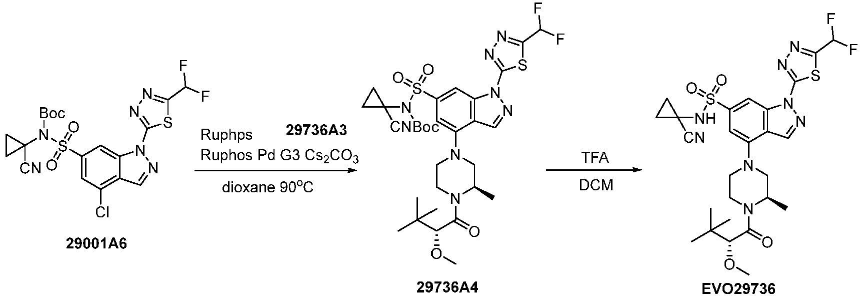

### Step 1:

Under an ice bath, PyBop (0.94 g, 1.81 mmol) was added to a solution of compound (R)-2-hydroxy-3,3-dimethylbutyric acid (0.20 g, 1.51 mmol), (R)-4-Boc-2-methylpiperazine (0.33 g, 1.66 mmol) and DIPEA (0.31 mL, 1.81 mmol) in DMF (5 mL), and the mixture was stirred at room temperature for 5 h. Water was added to the reaction mixture, and the resulting mixture was extracted with ethyl acetate. The organic phase was concentrated to remove the solvent, and the residue was separated and purified by silica gel column chromatography (ethyl acetate/petroleum ether) to give 29736A1 as a white solid (0.40 g, yield: 84.0%). LCMS (ESI) m/z = 259.4 [M+H-56]⁺.

### Step 2:

Under an ice bath, 60% NaH (0.03 g, 0.76 mmol) was added to a solution of compound 29736A1 (0.20 g, 0.64 mmol) in DMF (2 mL), and the mixture was stirred for 0.5 h. Methyl iodide (0.11 g, 0.76 mmol) was added to the reaction mixture, and the resulting mixture was stirred at room temperature for 2 h. Water was added to the reaction mixture, and the resulting mixture was extracted with ethyl acetate. The organic phase was concentrated to remove the solvent, and the residue was separated and purified by silica gel column chromatography (ethyl acetate/petroleum ether) to give 29736A2 as a white solid (0.20 g, yield: 95.7%). LCMS (ESI) m/z = 274.4 [M+H-56]⁺.

### Step 3:

To a solution of compound 29736A2 (0.20 g, 0.64 mmol) in ethyl acetate (0.5 mL) was added a solution of hydrogen chloride in 1,4-dioxane (4 M, 1 mL) at room temperature, and the mixture was stirred for 2 h. The reaction mixture was concentrated to remove the solvent to give 29246A3 as a white solid (a crude product, which was directly used in the next step). LCMS (ESI) m/z = 229.2 [M+H]⁺.

### Step 4:

To a solution of compound 29001A6 (100.00 mg, 0.19 mmol) in 1,4-dioxane (3 mL) were added methanesulfonato(2-dicyclohexylphosphino-2',6'-diisopropoxy-1,1'-biphenyl)(2-amino-1,1'-biphenyl-2-yl)palladium(II) (31.50 mg, 0.04 mmol), 2-dicyclohexylphosphino-2',6'-diisopropoxy-1,1'-biphenyl (35.10 mg, 0.08 mmol), 29736A3 (63.40 mg, 0.24 mmol), and cesium carbonate (183.90 mg, 0.56 mmol) at room temperature, and the reaction mixture was stirred at 90 °C for 3 h. The reaction mixture was concentrated to remove the solvent, and the residue was separated and purified by silica gel column chromatography (ethyl acetate/petroleum ether) to give 29736A4 as a pale yellow solid (72.00 mg, yield: 52.9%). LCMS (ESI) m/z = 723.1 [M+H]⁺.

### Step 5:

To a solution of compound 29736A4 (72.00 mg, 0.10 mmol) in dichloromethane (1 mL) was added trifluoroacetic acid (0.20 mL) at room temperature, and the reaction mixture was stirred at 25 °C for 1 h. The reaction mixture was concentrated to remove the solvent, and the residue was dissolved in ethyl acetate/aqueous sodium bicarbonate solution (5 mL/5 mL), followed by liquid separation. The organic phase was washed once with saturated brine, dried over anhydrous sodium sulfate, and concentrated to give a crude product, which was then subjected to preparative reversed-phase chromatography to give EVO29736 as a pale yellow solid (23.20 mg, yield: 37.4%). LCMS (ESI) m/z = 622.2 [M+H]⁺. ¹H NMR (400 MHz, DMSO-*d*₆) δ 9.33 (s, 1H), 8.95 - 8.79 (m, 1H), 8.54 - 8.41 (m, 1H), 7.61 (t, *J =* 53.1 Hz, 1H), 7.19 - 7.08 (m, 1H), 4.78 (d, *J* = 6.5 Hz, 1H), 4.59 - 4.33 (m, 1H), 4.00 - 3.68 (m, 4H), 3.30 - 3.25 (m, 3.5H), 3.22 - 2.95 (m, 1.5H), 1.49 - 1.42 (m, 2.5H), 1.36-1.28 (m, 4.5H), 0.96 (s, 9H).

### Example 42

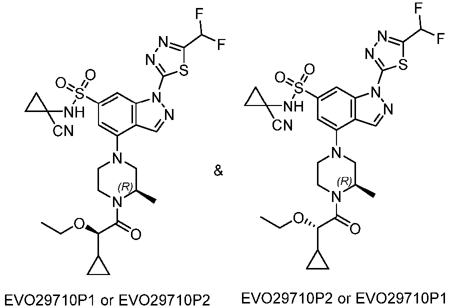

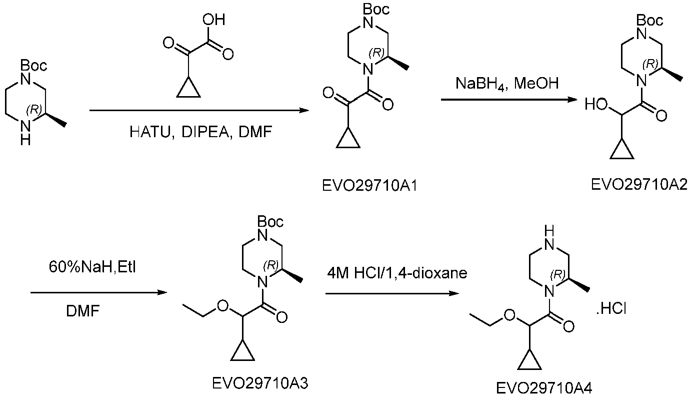

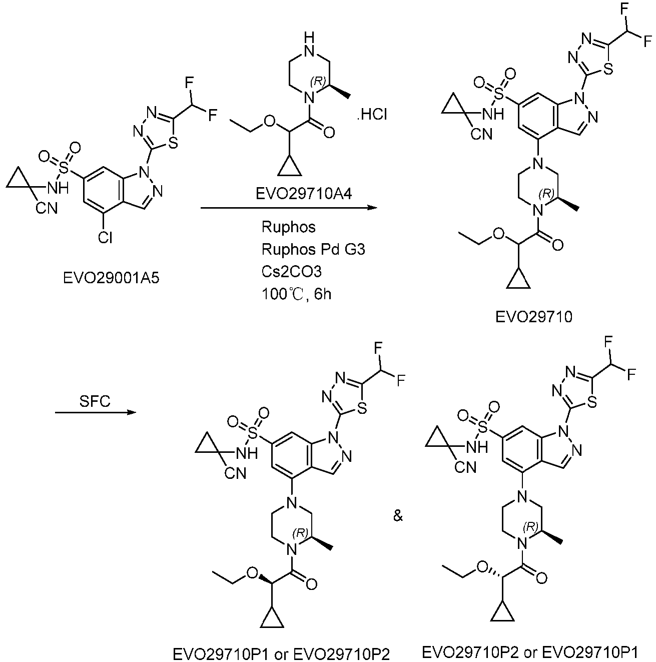

### Step 1:

(R)-1-Boc-3-methylpiperazine (4.00 g, 19.97 mmol), 2-cyclopropyl-2-oxoacetic acid (2.51 g, 21.97 mmol), *O*-(7-azabenzotriazol-1-yl)-*N*,*N*,*N*',*N*'-tetramethyluronium hexafluorophosphate (9.11 g, 23.97 mmol), and *N,N-*diisopropylethylamine (7.74 g, 59.92 mmol) were dissolved in N,N-dimethylformamide (20 mL), and the reaction mixture was allowed to react at room temperature for 2 h. Water (100 mL) was added to the reaction mixture, and the resulting mixture was extracted with ethyl acetate (100 mL). The organic phase was washed once with water (50 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether:ethyl acetate = 2:1) to give EVO29710A1 (5.45 g, yield: 92.08%).

### Step 2:

A solution of EVO29710A1 (5.45 g, 18.39 mmol) in methanol (100 mL) was cooled to 0 °C, and then sodium borohydride (730.47 mg, 19.31 mmol) was slowly added. The mixture was allowed to react at room temperature for 1 h. A saturated NH₄Cl solution was added to the reaction mixture, and the resulting mixture was stirred for 10 min. Silica gel was added, and the mixture was concentrated to dryness by rotary evaporation. The residue was purified by silica gel column chromatography (dichloromethane:methanol = 50:1 to 20:1) to give EVO29710A2 (5.02 g, yield: 91.49%).

### Step 3:

A solution of EVO29710A2 (5.02 g, 16.82 mmol) in *N,N-*dimethylformamide (50 mL) was cooled to 0 °C, and then 60% NaH (1.00 g, 25.24 mmol) was added in portions. The mixture was allowed to react for 1 h with the temperature maintained at 0 °C. EtI (3.15 g, 20.19 mmol) was added dropwise, and the mixture was allowed to react overnight. Water (250 mL) was added to the reaction mixture, and the resulting mixture was extracted with ethyl acetate (200 mL). The organic phase was washed once with water (100 mL × 2) and then with saturated brine (50 mL), dried over anhydrous sodium sulfate, and concentrated to dryness by rotary evaporation under vacuum to give a crude reaction product, which was then purified by silica gel column chromatography (dichloromethane:methanol = 50:1 to 20:1) to give EVO29710A3 (4.86 g, yield: 88.49%). LCMS (ESI) m/z = 271.23 [M+H-56]⁺.

### Step 4:

A 4 M solution of EVO29710A3 (3.70 g, 11.33 mmol) in HCl/1,4-dioxane (40 mL) was allowed to react at room temperature for 5 h and then concentrated to dryness by rotary evaporation under reduced pressure to give a crude product of EVO29710A4, which was used directly in the next step.

### Step 5:

To the crude product of EVO29710A4 from the previous step were added EVO29001A5 (4.44 g, 10.30 mmol), 1,4-dioxane (150 mL), 2-dicyclohexylphosphino-2',6'-diisopropoxy-1,1'-biphenyl (1.92 g, 4.12 mmol), methanesulfonato(2-dicyclohexylphosphino-2',6'-diisopropoxy-1,1'-biphenyl)(2-amino-1,1'-biphenyl-2-yl)palladium(II) (1.72 g, 2.06 mmol), and cesium carbonate (16.78 g, 51.51 mmol). The mixture was allowed to react at 100 °C for 8 h under an argon atmosphere, then cooled to room temperature, and filtered through celite under vacuum. The filtrate was concentrated to dryness by rotary evaporation, and the residue was pre-purified by silica gel column chromatography (petroleum ether/ethyl acetate = 1:1 to 2:3), and then separated and purified by preparative high performance liquid chromatography to give EVO29710 (2.20 g, yield: 34.41%).

EVO29710 (2.2 g) was subjected to resolution by supercritical fluid chromatography (SFC) (chromatography column: ChiralCel OD, 300 × 50 mm I.D., 10 µm; mobile phases: CO₂ as phase A, ethanol as phase B; gradient: B 40%; flow rate: 200 mL/min; back pressure: 100 bar; column temperature: 38 °C; wavelength: 300 nm; cycle time: about 8 min; injection volume: 17.00 mL) to give EVO29710P1 (1.27 g, retention time: 3.110 min) and EVO29710P2 (730 mg, retention time: 3.473 min). LCMS (ESI) m/z = 621.2 [M+H]⁺.

### EVO29710P1:

¹H NMR (400 MHz, DMSO-*d*₆) δ 9.36 (s, 1H), 8.90 (s, 1H), 8.54-8.50 (m, 1H), 7.64 (t, J = 52 Hz, 1H), 7.21-7.17 (m, 1H), 4.79-4.73 (m, 1H), 4.39-4.24 (m, 1H), 3.91-3.88 (m, 1H), 3.81-3.74 (m, 1H), 3.64-3.44 (m, 4H), 3.30-3.19 (m, 1H), 1.50-1.45 (m, 3H), 1.39-1.33 (m, 4H), 1.22-1.16 (m, 4H), 1.09 (t, J = 8 Hz, 2H), 0.63-0.42 (m, 2H), 0.34-0.30 (m, 1H).

### EVO29710P2:

¹H NMR (400 MHz, DMSO-*d*₆) δ 9.36 (s, 1H), 8.89 (s, 1H), 8.53 (s, 1H), 7.64 (t, J = 52 Hz, 1H), 7.20 (s, 1H), 4.74-4.71 (m, 1H), 4.35-4.32 (m, 1H), 3.94-3.90 (m, 1H), 3.81-3.74 (m, 1H), 3.64-3.45 (m, 4H), 3.23-3.03 (m, 1H),1.60-1.30 (m, 7H), 1.28-1.09 (m, 4H), 0.67-0.31 (m, 5H).

### Example 43

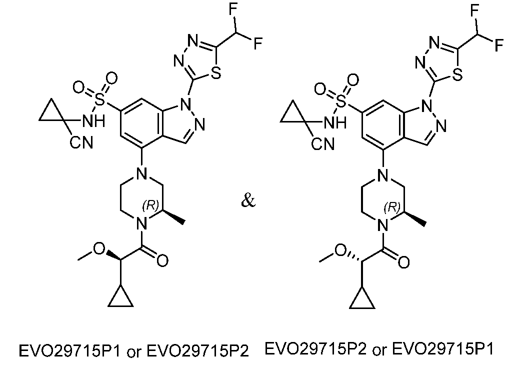

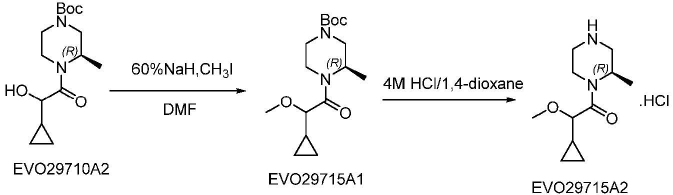

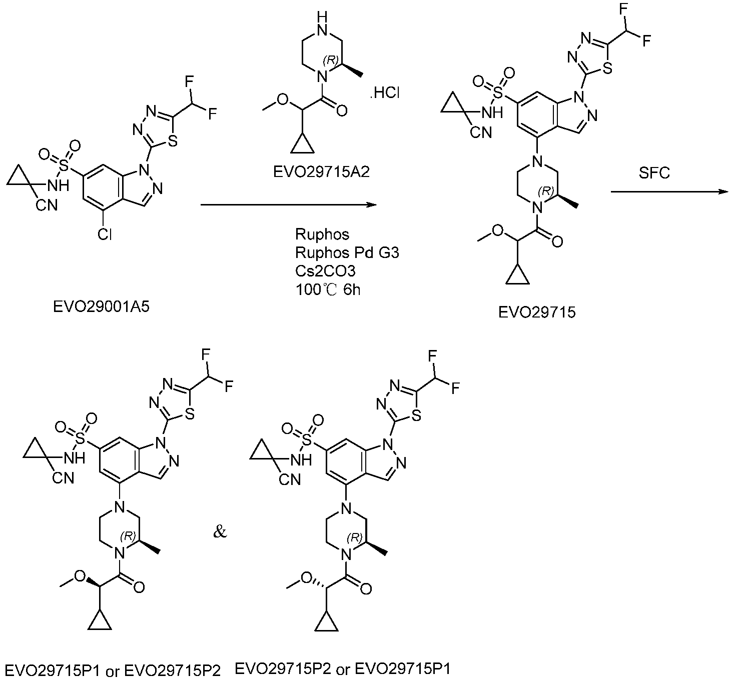

### Step 1:

A solution of EVO29710A2 (2.60 g, 8.71 mmol) in *N,N*-dimethylformamide (30 mL) was cooled to 0 °C, and then 60% NaH (592.50 mg, 14.81 mmol) was added in portions. The mixture was allowed to react for 1 h with the temperature maintained at 0 °C. CH₃I (1.86 g, 13.07 mmol) was added dropwise, and the mixture was allowed to react overnight. Water (150 mL) was added to the reaction mixture, and the resulting mixture was extracted with ethyl acetate (100 mL). The organic phase was washed once with water (500 mL × 2) and then with saturated brine (50 mL), dried over anhydrous sodium sulfate, and concentrated to dryness by rotary evaporation under vacuum to give a crude reaction product, which was then purified by silica gel column chromatography (dichloromethane:methanol = 50:1 to 20:1) to give EVO29715A1 (2.50 g, yield: 91.84%). LCMS (ESI) m/z = 313.13 [M+H]⁺.

### Step 2:

A 4 M solution of EVO29715A1 (1.48 g, 4.74 mmol) in HCl/1,4-dioxane (10 mL) was allowed to react at room temperature for 5 h and then concentrated to dryness by rotary evaporation under reduced pressure to give a crude product of EVO29715A2, which was used directly in the next step.

### Step 3:

To the crude product of EVO29715A2 from the previous step were added EVO29001A5 (1.82 g, 4.22 mmol), 1,4-dioxane (50 mL), 2-dicyclohexylphosphino-2',6'-diisopropoxy-1,1'-biphenyl (788.48 mg, 1.69 mmol), methanesulfonato(2-dicyclohexylphosphino-2',6'-diisopropoxy-1,1'-biphenyl)(2-amino-1,1'-biphenyl-2-yl)palladium(II) (706.62 mg, 0.84 mmol), and cesium carbonate (6.88 g, 21.12 mmol). The mixture was allowed to react at 100 °C for 8 h under an argon atmosphere, then cooled to room temperature, and filtered through celite under vacuum. The filtrate was concentrated to dryness by rotary evaporation, and the residue was purified by silica gel column chromatography (petroleum ether:ethyl acetate = 1:1 to 2:3) to give EVO29715 (970.00 mg, yield: 37.85%).

EVO29715 (970 mg) was subjected to resolution by supercritical fluid chromatography (SFC) (chromatography column: ChiralPak AD, 250 × 30 mm I.D., 10 µm; mobile phases: CO₂ as phase A, isopropanol (0.1% NH₃H₂O) as phase B; gradient: B 25%; flow rate: 150 mL/min; back pressure: 100 bar; column temperature: 38 °C; wavelength: 220 nm; cycle time: about 12 min; injection volume: 3.00 mL) to give EVO29715P1 (501 mg, retention time: 3.538 min) and EVO29715P2 (268 mg, retention time: 3.916 min). LCMS (ESI) m/z = 607.2 [M+H]⁺.

### EVO29715P1:

¹H NMR (400 MHz, DMSO-*d*₆) δ 9.35 (s, 1H), 8.90 (s, 1H), 8.54-8.50 (m, 1H), 7.64 (t, J = 52 Hz, 1H), 7.21-7.16 (m, 1H), 4.79-4.66 (m, 1H), 4.39-4.36 (m, 1H), 4.23-4.19 (m, 1H) 3.93-3.85 (m, 1H), 3.80-3.76 (m, 2H), 3.62-3.51 (m, 1H), 3.31-3.25 (m, 3H), 1.50-1.46 (m, 3H), 1.36-1.33 (m, 4H), 1.22-1.13 (m, 1H), 1.11-1.05 (m, 2H), 0.63-0.42 (m, 3H).

### EVO29715P2:

¹H NMR (400 MHz, DMSO-*d*₆) δ 9.36 (s, 1H), 8.90 (s, 1H), 8.52 (s, 1H), 7.64 (t, J = 52 Hz, 1H), 7.19 (s, 1H), 4.71 (br s, 1H), 4.41-4.39 (m, 1H), 4.26-4.20 (m, 1H), 3.91-3.89 (m, 1H), 3.78-3.68 (m, 2H), 3.51-3.48 (m, 1H), 3.36-3.33 (m, 5H), 1.50-1.45 (m, 3H), 1.38-1.33 (m, 4H), 1.19 (br s, 1H), 0.66-0.59 (m, 1H), 0.56-0.44 (m, 1H), 0.33-0.27 (m, 1H).

### Example 44

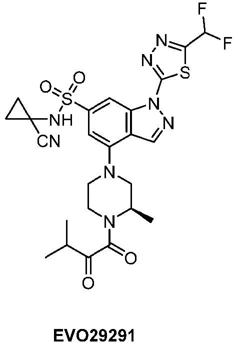

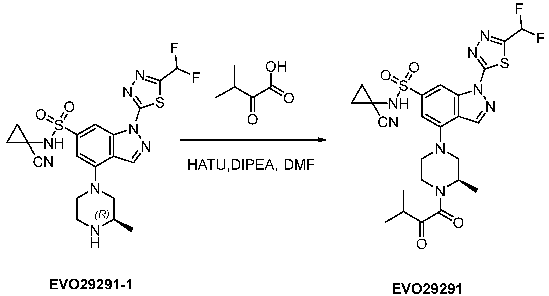

### Step 1:

To a solution of compound EVO29291-1 (150.0 mg, 0.30 mmol) in N,N-dimethylformamide (5 mL) were added *O*-(7-azabenzotriazol-1-yl)-*N*,*N*,*N*',*N*'-tetramethyluronium hexafluorophosphate (173.0 mg, 0.46 mmol), *N,N-*diisopropylethylamine (196.0 mg, 1.52 mmol), and 3-methyl-2-oxobutanoic acid (43.0 mg, 0.36 mmol) at room temperature, and the reaction mixture was stirred at 25 °C for 2 h. Water (30 mL) was added to the reaction mixture, and the resulting mixture was extracted with ethyl acetate (30 mL × 3). The organic phases were combined, dried over anhydrous sodium sulfate, and filtered, and the residue was separated and purified by silica gel column chromatography (petroleum ether:ethyl acetate = 1:1) to give EVO29291 (102.0 mg, yield: 55.9%). LCMS (ESI) m/z = 593.1 [M+H]⁺. ¹H NMR (400 MHz, DMSO-*d*₆) δ 9.30 (s, 1H), 8.84 (d, *J* = 2.2 Hz, 1H), 8.52 (d, *J =* 9.3 Hz, 1H), 7.59 (t, *J* = 53.1 Hz, 1H), 7.18 (d, *J =* 9.4 Hz, 1H), 4.75 - 4.22 (m, 1H), 3.98 - 3.67 (m, 3H), 3.59 - 3.30 (m, 2H), 3.20 - 3.03 (m, 2H), 1.44 (t, *J =* 6.0 Hz, 3H), 1.37 (t, *J* = 7.2 Hz, 2H), 1.32 (dd, *J =* 8.3*,* 5.4 Hz, 2H), 1.18 - 1.11 (m, 6H).

### Example 45

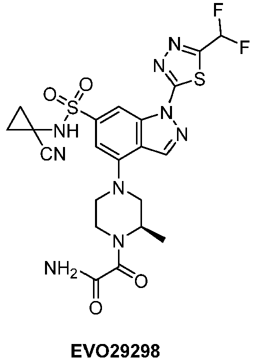

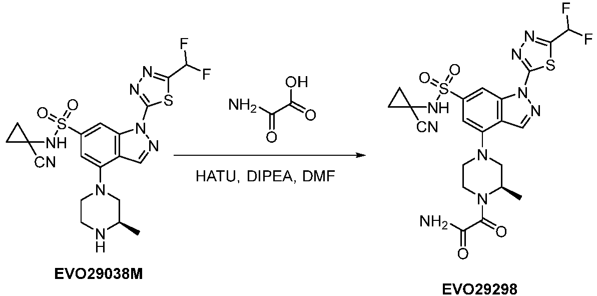

### Step 1:

Compound EVO29038M (150.0 mg, 0.30 mmol) was dissolved in *N,N-*dimethylformamide (2 mL), and then oxamic acid (41.0 mg, 0.46 mmol), *O*-(7-azabenzotriazol-1-yl)-*N*,*N*,*N*',*N*'-tetramethyluronium hexafluorophosphate (173.0 mg, 0.46 mmol), and N,N-diisopropylethylamine (294.0 mg, 2.28 mmol) were added. The mixture was stirred for reaction at room temperature for 2 h. LCMS showed the main peak, which indicates the product. The reaction mixture was directly separated and purified by reversed-phase column chromatography (water:acetonitrile = 2:1) to give EVO29298 (68.5 mg, yield: 40.0%). LCMS (ESI) m/z = 568.1 [M+H]⁺. ¹H NMR (400 MHz, DMSO-*d*₆) δ 9.34 (s, 1H), 8.86 (d, *J* = 3.2 Hz, 1H), 8.50 (d, *J* = 10.8 Hz, 1H), 8.14 (d, *J =* 5.8 Hz, 1H), 7.82 - 7.44 (m, 2H), 7.17 (d, *J =* 10.8 Hz, 1H), 4.64 (s, 1H), 4.26 (d, *J* = 13.3 Hz, 1H), 3.97 - 3.65 (m, 3H), 3.29 (s, 3H), 3.26 - 2.96 (m, 2H), 1.45 (d, *J =* 3.6 Hz, 2H), 1.35 (d, *J* = 6.8 Hz, 2H).

### Example 46

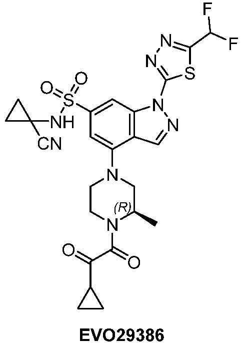

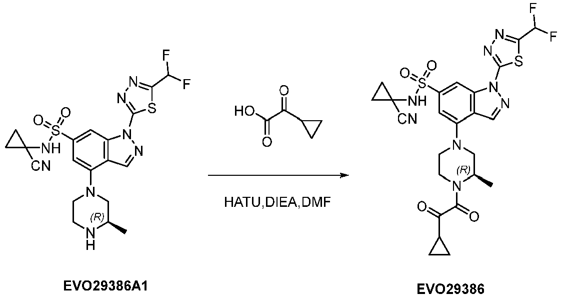

### Step 1:

To a solution of compound EVO29386-1 (100 mg, 0.20 mmol) in *N,N-*dimethylformamide (5 mL) were added *O*-(7-azabenzotriazol-1-yl)-*N*,*N*,*N*',*N*'-tetramethyluronium hexafluorophosphate (115 mg, 0.30 mmol), *N,N-*diisopropylethylamine (78 mg, 0.61 mmol), and 2-cyclopropyl-2-oxoacetic acid (28 mg, 0.24 mmol) at room temperature, and the reaction mixture was stirred at 25 °C for 2 h. The reaction mixture was concentrated to dryness by rotary evaporation to remove the solvent. Water (50 mL) was added, and the mixture was extracted with ethyl acetate (30 mL × 3), followed by liquid separation. The organic phases were combined, dried over anhydrous sodium sulfate, and separated and purified by silica gel column chromatography (petroleum ether/ethyl acetate = 1:1) to give EVO29386 as a pale yellow solid (57 mg, yield: 47.50%). LCMS (ESI) m/z = 591.0 [M+H]+. ¹H NMR (DMSO-*d*₆, 400 MHz) δ 9.37 (s, 1H), 8.86 (s, 1H), 8.51 (d, *J* = 8.4 Hz, 1H), 7.61 (t, *J* = 53.1 Hz, 1H), 7.18 (d, *J* = 9.5 Hz, 1H), 4.73 - 4.21 (m, 1H), 4.02 - 3.86 (m, 1H), 3.82 - 3.55 (m, 2H), 3.40 (dd, *J =* 23.0, 19.2 Hz, 2H), 3.23 - 3.04 (m, 1H), 2.40 - 2.27 (m, 1H), 1.48 - 1.43 (m, 3H), 1.38 (d, *J* = 6.7 Hz, 2H), 1.31 (dd, *J =* 8.0, 5.4 Hz, 2H), 1.21 - 1.10 (m, 4H).

### Example 47

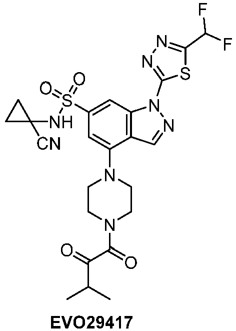

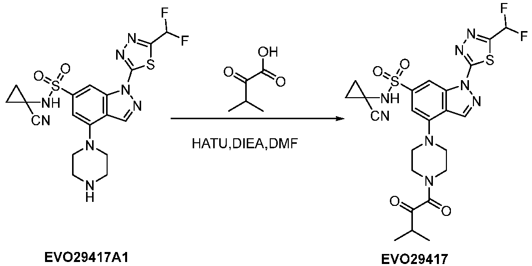

### Step 1:

To EVO29417A1 (150.0 mg, 0.31 mmol), 3-methyl-2-oxobutanoic acid (43.5 mg, 0.38 mmol) and *N,N*-diisopropylethylamine (121.0 mg, 0.94 mmol) dissolved in *N,N*-dimethylformamide (3 mL) was added *O*-(7-azabenzotriazol-1-yl)-*N*,*N*,*N*',*N*'-tetramethyluronium hexafluorophosphate (142.5 mg, 0.38 mmol) in portions, and the reaction mixture was stirred at room temperature for 1.5 h. Water (70 mL) was added, and the mixture was extracted with ethyl acetate (50 mL × 2). The organic phases were combined, dried over anhydrous sodium sulfate, filtered, and concentrated, and the residue was separated and purified by silica gel column chromatography (dichloromethane/methanol = 70:1) to give EVO29417 as an off-white solid (110.0 mg, yield: 61.00%). LCMS (ESI) m/z = 579.1 [M+H]⁺. ¹H NMR(DMSO-*d*₆, 400 MHz): δ 9.36 (s, 1H), 8.98 (s, 1H), 8.54 (s, 1H), 7.61 (s, 1H), 7.20 (s, 1H), 3.81 (s, 2H), 3.60 (s, 2H), 3.49 (d, *J*=17.7 Hz, 4H), 3.16 - 3.04 (m, 1H), 1.44 (d, *J*=4.7 Hz, 2H), 1.33 (d, *J*=4.8 Hz, 2H), 1.08 (s, 9H), 1.15 (s, 3H), 1.13 (s, 3H).

### Example 48

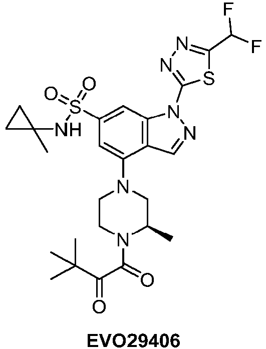

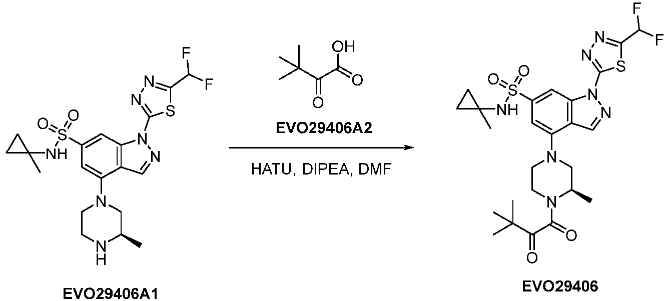

### Step 1:

Compound EVO29406A1 (100 mg, 0.21 mmol), compound EVO29406A2 (32.3 mg, 0.25mmol), *O*-(7-azabenzotriazol-1-yl)-*N*,*N*,*N*',*N*'-tetramethyluronium hexafluorophosphate (117.8 mg, 0.31 mmol), and *N,N*-diisopropylethylamine (80.0 mg, 0.62 mmol) were dissolved in *N,N*-dimethylformamide (5 mL), and the reaction mixture was allowed to react at room temperature for 2 h. Water (20 mL) was added to the reaction mixture, and the resulting mixture was extracted with ethyl acetate (20 mL). The organic phase was washed once with water (20 mL × 2) and then with saturated brine (20 mL), dried over anhydrous sodium sulfate, and concentrated to dryness by rotary evaporation under vacuum to give a crude reaction product, which was then purified by silica gel column chromatography (petroleum ether/ethyl acetate = 1:1) to give EVO29406 as a yellow solid (47.3 mg, yield: 37.80%). LCMS (ESI) m/z = 596.0 [M+H]⁺. ¹H NMR (DMSO-*d*₆, 400 MHz): δ 8.81 (d, *J* = 4.4 Hz, 1H), 8.47 (d, *J* = 8.9 Hz, 1H), 8.33 (s, 1H), 7.60 (t, *J =* 53.1 Hz, 1H), 7.17 (d, *J* = 9.0 Hz, 1H), 4.74 - 4.22 (m, 1H), 3.79 (ddd, *J* = 39.3, 27.7, 10.9 Hz, 3H), 3.38 (s, 1H), 3.32 - 2.95 (m, 2H), 1.40 (dd, *J =* 21.4, 6.6 Hz, 3H), 1.23 (d, *J* = 4.6 Hz, 9H), 1.08 (s, 3H), 0.73 - 0.58 (m, 2H), 0.41 (s, 2H).

### Example 49

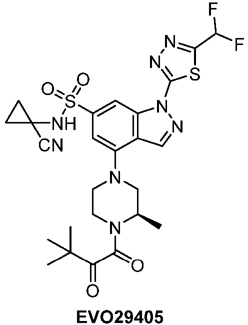

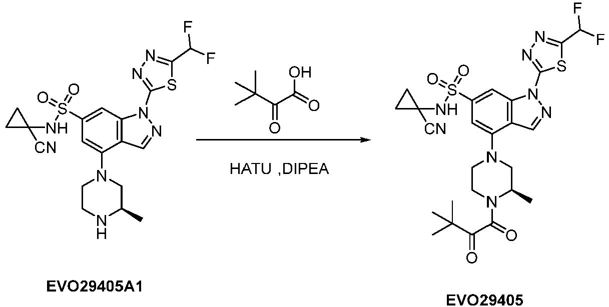

### Step 1:

To a solution of compound EVO29405A1 (100 mg, 0.20 mmol) in N,N-dimethylformamide (5 mL) were added *O*-(7-azabenzotriazol-1-yl)-*N*,*N*,*N*',*N*'-tetramethyluronium hexafluorophosphate (115 mg, 0.30 mmol), N,N-diisopropylethylamine (130 mg, 1.01 mmol), and trimethylpyruvic acid (86 mg, 0.40 mmol) at room temperature, and the reaction mixture was stirred at 25 °C for 2 h. Water (30 mL) was added to the reaction mixture, and the resulting mixture was extracted with ethyl acetate (30 mL × 3). The organic phases were combined, dried over anhydrous sodium sulfate, filtered, and concentrated, and the residue was separated and purified by silica gel column chromatography (petroleum ether/ethyl acetate = 1:1) to give EVO29405 as a pale yellow solid (65 mg, yield: 54.20%). LCMS (ESI) m/z = 607.1 [M+H]⁺. ¹H NMR (DMSO-d₆, 400 MHz): δ 9.35 (s, 1H), 8.86 (d, *J* = 3.7 Hz, 1H), 8.52 (d, *J* = 9.7 Hz, 1H), 7.61 (t, *J* = 53.1 Hz, 1H), 7.18 (d, *J* = 10.1 Hz, 1H), 4.74 - 4.20 (m, 1H), 3.97 - 3.67 (m, 3H), 3.38 (dd, *J =* 21.7, 8.0 Hz, 1H), 3.30 - 3.20 (m, 1H), 3.17 - 2.99 (m, 1H), 1.48 - 1.41 (m, 3H), 1.38 (d, *J =* 6.8 Hz, 2H), 1.32 (dd, *J =* 8.3, 5.3 Hz, 2H), 1.24 (d, *J* = 4.5 Hz, 9H).

### Example 50

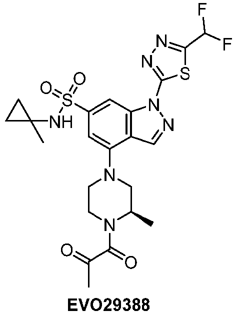

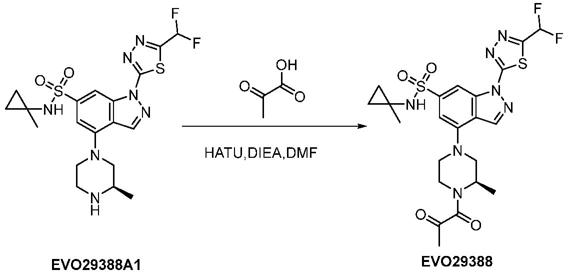

### Step 1:

To a solution of EVO29388A1 (100 mg, 0.20 mmol), pyruvic acid (27.3 mg, 0.31 mmol) and *N,N-*diisopropylethylamine (155 mg, 1.20 mmol) dissolved in N,N-dimethylformamide (3 mL) was added O-(7-azabenzotriazol-1-yl)-*N*,*N*,*N*',*N*'-tetramethyluronium hexafluorophosphate (91.2 mg, 0.24 mmol) in portions, and the reaction mixture was stirred at room temperature for 1.5 h. Water (70 mL) was added, and the mixture was extracted with ethyl acetate (50 mL × 2). The organic phases were combined, dried over anhydrous sodium sulfate, filtered, and concentrated, and the residue was separated and purified by silica gel column chromatography (dichloromethane/methanol = 70:1) to give EVO29388 as an off-white solid (28.4 mg, yield: 24.90%). LCMS (ESI) m/z = 554.1 [M+H]⁺. ¹H NMR(DMSO-*d*₆, 400 MHz): δ 8.82 (s, 1H), 8.47 (d, J=10.1 Hz, 1H), 8.34 (d, J=4.0 Hz, 1H), 7.60 (t, J=53.1 Hz, 1H), 7.16 (d, J=9.1 Hz, 1H), 4.71 - 4.20 (m, 1H), 4.12 - 3.85 (m, 1H), 3.83 - 3.61 (m, 3H), 3.42 (d, *J*=12.2 Hz, 1H), 3.30 (s, 1H), 3.24 - 3.01 (m, 1H), 2.46 - 2.40 (m, 3H), 1.48 - 1.41 (m, 3H), 1.09 (s, 3H), 0.71 - 0.61 (m, 2H), 0.41 (d, *J*=1.4Hz, 2H).

### Example 51

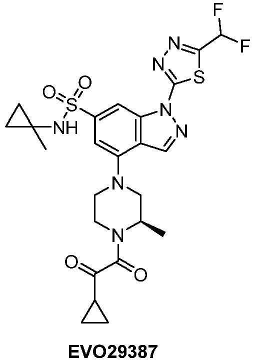

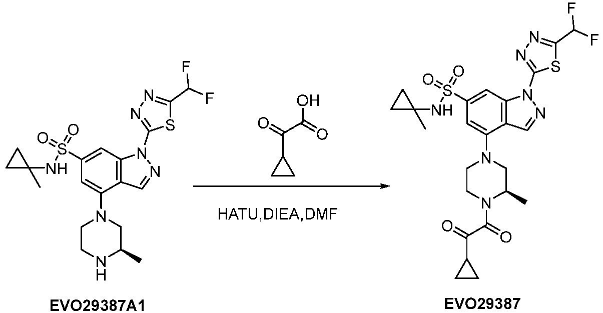

### Step 1:

To a solution of EVO29387A1 (100 mg, 0.21 mmol), 3-cyclopropyl-2-oxoacetic acid (35.4 mg, 0.31 mmol) and *N,N*-diisopropylethylamine (133.5 mg, 1.04 mmol) dissolved in *N,N*-dimethylformamide (3 mL) was added *O*-(7-azabenzotriazol-1-yl)-*N*,*N*,*N*',*N*'-tetramethyluronium hexafluorophosphate (117.8 mg, 0.31 mmol) in portions, and the reaction mixture was stirred at room temperature for 1.5 h. Water (70 mL) was added, and the mixture was extracted with ethyl acetate (50 mL × 2). The organic phases were combined, dried over anhydrous sodium sulfate, filtered, and concentrated, and the residue was separated and purified by silica gel column chromatography (dichloromethane/methanol = 70:1) to give EVO29387 as an off-white solid (110 mg, yield: 61.00%). LCMS (ESI) m/z = 580.1 [M+H]⁺. ¹H NMR(DMSO-*d*₆, 400 MHz): δ 8.82 (s, 1H), 8.47 (d, *J*=8.3 Hz, 1H), 8.33 (d, *J*=2.8 Hz, 1H), 7.60 (t, *J*=53.1 Hz, 1H), 7.16 (d, *J=8.8* Hz, 1H), 4.76 - 4.22 (m, 1H), 3.91 - 3.85 (m, 3H), 3.62 - 3.45 (m, 1H), 3.44 - 3.39 (m, 1H), 3.29 (s, 1H), 3.23 - 3.04 (m, 1H), 2.39 - 2.27 (m, 1H), 1.45 - 1.38 (m, 3H), 1.20 - 1.10 (m, 4H), 1.08 (s, 3H), 0.72 - 0.59 (m, 2H), 0.41 (s, 2H).

### Example 52

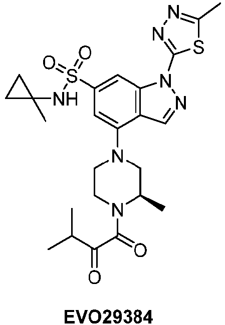

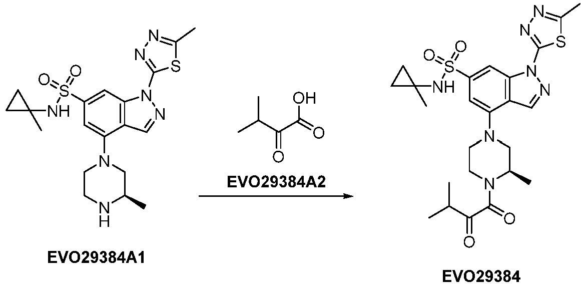

Step 1: Compound EVO29384A1 (100 mg, 0.23 mmol) was dissolved in N,N-dimethylformamide (3 mL), and then EVO29384A2 (40 mg, 0.34 mmol), *O*-(7-azabenzotriazol-1-yl)-*N*,*N*,*N*',*N*' tetramethyluronium hexafluorophosphate (129 mg, 0.34 mmol), and N,N-diisopropylethylamine (149 mg, 1.15 mmol) were added. The mixture was allowed to react at room temperature for 2 h. LCMS showed the main peak, which indicates the product. Water (50 mL) was added to the reaction mixture, and the resulting mixture was extracted with ethyl acetate (30 mL × 2). The organic phases were combined, washed once with saturated brine (50 mL), dried over anhydrous sodium sulfate, filtered, and concentrated, and the residue was separated and purified by silica gel column chromatography (petroleum ether/ethyl acetate = 2:1) to give EVO29384 (41.6 mg, yield: 33.28%). LCMS (ESI) m/z = 546.1 [M+H]⁺. ¹H NMR (DMSO-*d*₆*,* 400 MHz): δ 8.71 (d, *J =* 4.0 Hz, 1H), 8.47 (d, *J* = 7.8 Hz, 1H), 8.30 (d, *J =* 2.6 Hz, 1H), 7.12 (d, *J* = 7.7 Hz, 1H), 3.97 - 3.64 (m, 3H), 3.46 (dd, *J* = 36.0, 12.8 Hz, 1H), 3.31 - 3.15 (m, 1H), 3.13 - 2.96 (m, 2H), 2.75 (s, 3H), 1.49 - 1.37 (m, 3H), 1.18 - 1.10 (m, 6H), 1.07 (s, 3H), 0.83 (dd, *J* = 9.5, 6.8 Hz, 1H), 0.71 - 0.57 (m, 2H), 0.40 (d, *J =* 1.6 Hz, 2H).

### Example 53

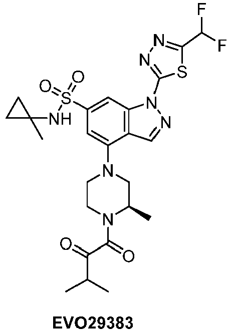

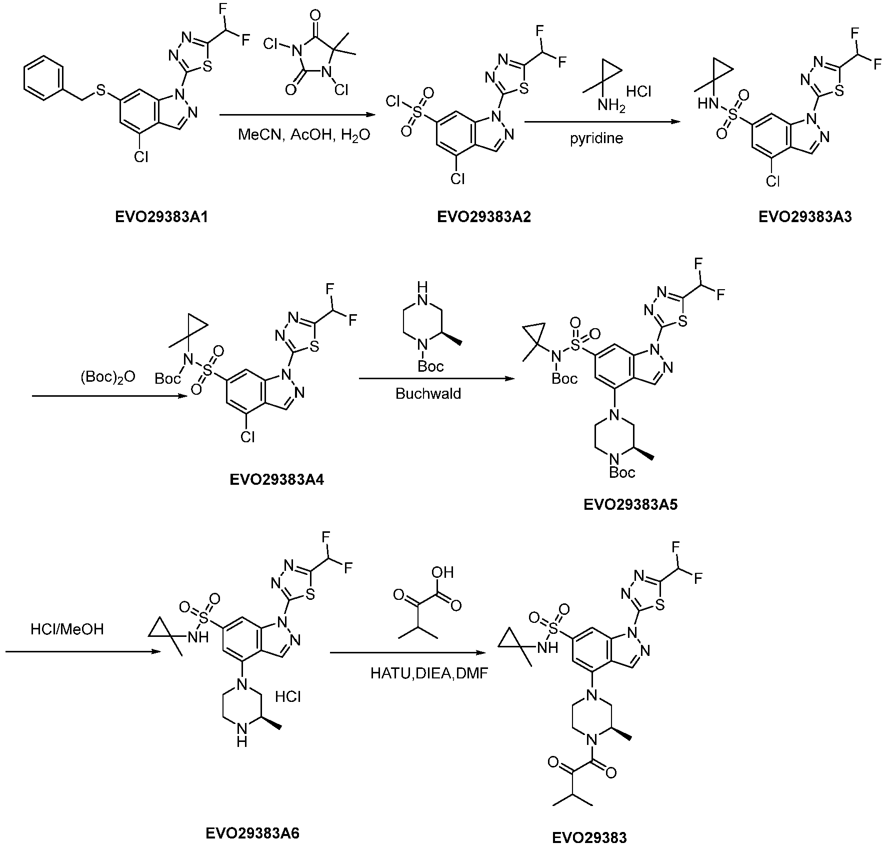

### Step 1:

In a reaction flask, EVO29383A1 (5 g, 12.25 mmol) was dissolved in acetonitrile (30 mL), and then water (2.5 mL) and acetic acid (5 mL) were added under an ice bath. 1,3-Dichloro-5,5-dimethylhydantoin (3.62 g, 18.38 mmol) was then added in portions to the reaction flask, and the reaction mixture was stirred at 25 °C for 2 h. The reaction mixture was concentrated, and an appropriate amount of water (100 mL) was added. The mixture was then extracted with ethyl acetate (100 mL × 3), followed by liquid separation. The organic phases were combined, dried over anhydrous sodium sulfate, filtered, and concentrated, and the residue was separated and purified by silica gel column chromatography (petroleum ether/ethyl acetate = 2:1) to give EVO29383A2 (4.6 g, yield: 97.00%). LCMS (ESI) m/z = 386.2 [M+H]⁺.

### Step 2:

1-Methylcyclopropanamine hydrochloride (1.88 g, 17.57 mmol) was dissolved in dichloromethane (100 mL), and then triethylamine (5.9 g, 58.59 mmol) was added under an ice bath. EVO29383A2 (4.5 g, 11.72 mmol) was then added in portions to the reaction, and the reaction mixture was stirred at 25 °C for 2 h. The reaction mixture was concentrated, and the residue was purified by silica gel column chromatography (petroleum ether/ethyl acetate = 2:1) to give EVO29383A3 (2.7 g, yield: 55.00%). LCMS (ESI) m/z = 420.8 [M+H]⁺.

### Step 3:

To a solution of compound EVO29383A3 (2.6 g, 6.21 mmol) in dichloromethane (20 mL) were added di-tert-butyl dicarbonate (2.7 g, 12.41 mmol), 4-dimethylaminopyridine (75 mg, 0.62 mmol), and triethylamine (1.25 g, 12.41 mmol) at room temperature, and the reaction mixture was stirred at 25 °C for 2 h. The reaction mixture was concentrated, and the residue was separated and purified by silica gel column chromatography (petroleum ether/ethyl acetate = 5:1) to give EVO29383A4 as a pale yellow solid (2.1 g, yield: 66.00%). LCMS (ESI) m/z = 520.9 [M+H]⁺.

### Step 4:

To a solution of compound EVO29383A4 (2.1 g, 5.01 mmol) in 1,4-dioxane (30 mL) were added methanesulfonato(2-dicyclohexylphosphino-2',6'-diisopropoxy-1,1'-biphenyl)(2-amino-1,1'-biphenyl-2-yl)palladium(II) (1.67 g, 2.01 mmol), 2-dicyclohexylphosphino-2',6'-diisopropoxy-1,1'-biphenyl (1.87 g, 4.01 mmol), cesium carbonate (4.88 g, 15.04 mmol), and (*R*)-1-N-Boc-2-methylpiperazine (2.00 g, 10.02 mmol) at room temperature, and the reaction mixture was stirred at 90 °C for 2 h. The reaction mixture was concentrated, and the residue was separated and purified by silica gel column chromatography (petroleum ether/ethyl acetate = 5:1) to give EVO29383A5 as a pale yellow solid (800 mg, yield: 30.00%). LCMS (ESI) m/z = 684.7 [M+H]⁺.

### Step 5:

To a solution of compound EVO29383A5 (800 mg, 1.17 mmol) in methanol (10 mL) was added a solution of hydrogen chloride in methanol (25 mL, 6 M) at room temperature, and the reaction mixture was stirred at 25 °C for 2 h. The reaction mixture was concentrated to give EVO29383A6 as a pale yellow solid (500 mg, yield: 90.00%). LCMS (ESI) m/z = 484.5 [M+H]⁺.

### Step 6:

To a solution of compound EVO29383A6 (50 mg, 0.11 mmol) in *N,N*-dimethylformamide (1 mL) were added *O*-(7-azabenzotriazol-1-yl)-*N*,*N*,*N*',N'-tetramethyluronium hexafluorophosphate (60 mg, 0.16 mmol), *N,N-*diisopropylethylamine (40 mg, 0.31 mmol), and 3-methyl-2-oxobutanoic acid (14 mg, 0.13 mmol) at room temperature, and the reaction mixture was stirred at 25 °C for 2 h. An appropriate amount of water (10 mL) was added to the reaction mixture, and then the resulting mixture was extracted with ethyl acetate (20 mL × 3), followed by liquid separation. The organic phases were combined, dried over anhydrous sodium sulfate, and concentrated, and the residue was separated and purified by silica gel column chromatography (petroleum ether/ethyl acetate = 2:1) to give EVO29383 as a pale yellow solid (30 mg, yield: 50.00%). LCMS (ESI) m/z = 582.6 [M+H]⁺. ¹H NMR (DMSO-*d*₆, 400 MHz): δ 8.82 (d, *J* = 4.3 Hz, 1H), 8.48 (d, *J* = 8.8 Hz, 1H), 8.33 (d, *J* = 2.5 Hz, 1H), 7.61 (t, *J =* 53.1 Hz, 1H), 7.17 (d, *J =* 8.3 Hz, 1H), 4.74 - 4.25 (m, 1H), 3.89 (d, *J* = 13.5 Hz, 1H), 3.84 - 3.66 (m, 2H), 3.58 = 3.37 (m, 1H), 3.24 (dd, *J =* 12.6, 3.2 Hz, 1H), 3.17 - 3.00 (m, 2H), 1.42 (dd, *J =* 25.5, 6.7 Hz, 3H), 1.18 - 1.11 (m, 6H), 1.09 (s, 3H), 0.66 (d, *J* = 5.7 Hz, 2H), 0.41 (s, 2H).

### Example 54

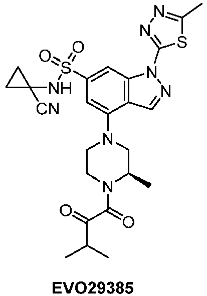

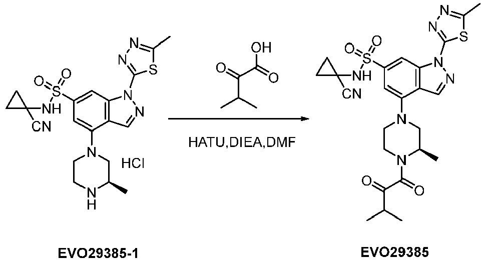

### Step 1:

To a solution of compound EVO29385-1 (100.0 mg, 0.22 mmol) in N,N-dimethylformamide (2 mL) were added *O*-(7-azabenzotriazol-1-yl)-*N*,*N*,*N'*,*N*'-tetramethyluronium hexafluorophosphate (124.0 mg, 0.33 mmol), N,N-diisopropylethylamine (85.0 mg, 0.66 mmol), and 3-methyl-2-oxobutanoic acid (30.0 mg, 0.26 mmol) at room temperature, and the reaction mixture was stirred at 25 °C for 2 h. An appropriate amount of water (10 mL) was added to the reaction mixture, and then the resulting mixture was extracted with ethyl acetate (3 × 20 mL), followed by liquid separation. The organic phases were combined, dried over anhydrous sodium sulfate, and concentrated, and the residue was separated and purified by silica gel column chromatography (petroleum ether/ethyl acetate = 1:1) to give EVO29385 as a pale yellow solid (44 mg, yield: 36%). LCMS (ESI) m/z = 557.6 [M+H]⁺. ¹H NMR (400 MHz, DMSO-*d*₆) δ 9.30 (s, 1H), 8.75 (d, *J =* 2.7 Hz, 1H), 8.52 (d, *J =* 8.4 Hz, 1H), 7.13 (d, *J* = 8.7 Hz, 1H), 4.74 - 4.27 (m, 1H), 3.95 - 3.83 (m, 1H), 3.82 - 3.64 (m, 2H), 3.56 - 3.37 (m, 1H), 3.22 (dd, *J =* 11.9, 3.3 Hz, 1H), 3.15 - 3.00 (m, 2H), 2.76 (s, 3H), 1.48 - 1.43 (m, 3H), 1.39 (d, *J* = 6.7 Hz, 2H), 1.31 (dd, *J* = 8.1*,* 5.1 Hz, 2H), 1.14 (dd, *J =* 9.9, 4.2 Hz, 6H).

### Example 55

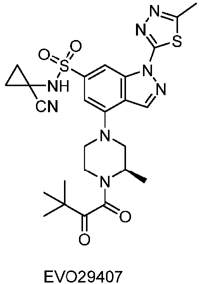

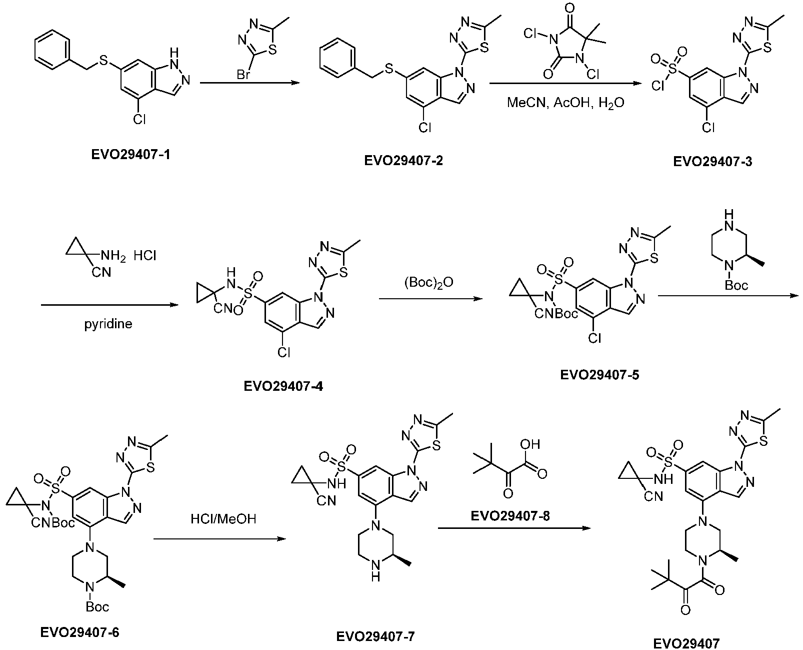

### Step 1:

To a mixture of EVO29407-1 (6.0 g, 3.6 mmol) and 1,2-dioxane were added 2-bromo-5-methyl-1,3,4-thiadiazole (11.8 g, 65.7 mmol), copper(I) iodide (2.1 mg, 10.95 mmol), *N,N-*dimethylcyclohexanediamine (3.2 g, 21.9 mmol), and tripotassium phosphate (13.9 g, 65.7 mmol). Under a nitrogen atmosphere, the mixture was heated to 110 °C and allowed to react for 16 h. TLC analysis (petroleum ether:ethyl acetate = 3:1) showed complete conversion of the starting materials. The reaction mixture was cooled to room temperature, and 150 mL of an ammonium hydroxide solution was added. The resulting mixture was stirred for half an hour, and then a solid was precipitated. The mixture was filtered, and 100 mL of water was added to the filtrate. The resulting mixture was extracted with ethyl acetate (100 mL × 3). The organic phases were combined, dried over anhydrous sodium sulfate, filtered, and concentrated, and the residue was separated and purified by silica gel column chromatography (petroleum ether:ethyl acetate = 4:1) to give EVO29407-2 (3 g, yield: 40%).

### Step 2:

EVO29407-2 (1.0 g, 2.68 mmol) was dissolved in acetonitrile (10 mL), and then water (0.7 mL) and acetic acid (2 mL) were added under an ice bath. 1,3-Dichloro-5,5-dimethylhydantoin (1.0 g, 4.8 mmol) was then added in small portions to the reaction, and the reaction mixture was stirred at 25 °C for 2 h. The reaction mixture was concentrated, and then an appropriate amount of water (100 mL) was added. The mixture was extracted with ethyl acetate (50 mL × 3), followed by liquid separation. The organic phases were combined, dried over anhydrous sodium sulfate, and concentrated, and the residue was purified by normal-phase column chromatography (petroleum ether:ethyl acetate = 3:1) to give EVO29407-3 (2 g, yield: 98%). LCMS (ESI) m/z = 348.1 [M+H]⁺.

### Step 3:

1-Cyanocyclopropylamine hydrochloride (870.0 mg, 7.33 mmol) was dissolved in pyridine (100 mL), and then EVO29407-3 (1.7 g, 4.98 mmol) was added in small portions to the reaction under an ice bath. The reaction mixture was stirred at 0 °C for 2 h. The reaction mixture was concentrated, and the residue was purified by normal-phase column chromatography (petroleum ether:ethyl acetate = 2:1) to give EVO29407-4 (1 g, yield: 63%). LCMS (ESI) m/z = 394.0 [M+H]⁺.

### Step 4:

To a solution of compound EVO29407-4 (1.0 g, 2.54 mmol) in dichloromethane (25 mL) were added di-tert-butyl dicarbonate (720.0 mg, 3.3 mmol), 4-dimethylaminopyridine (75.0 mg, 0.62 mmol), and triethylamine (770.0 mg, 7.62 mmol) at room temperature, and the reaction mixture was stirred for 2 h. The reaction mixture was concentrated, and the residue was separated and purified by silica gel column chromatography (petroleum ether/ethyl acetate = 16:9) to give EVO29407-5 as a white solid (1.0 g, yield: 80%). LCMS (ESI) m/z = 494.0 [M+H]⁺.

### Step 5:

To a solution of compound EVO29407-5 (1.0 g, 2.03 mmol) in 1,4-dioxane (30 mL) were added methanesulfonato(2-dicyclohexylphosphino-2',6'-diisopropoxy-1,1'-biphenyl)(2-amino-1,1'-biphenyl-2-yl)palladium(II) (686.0 mg, 0.82 mmol), 2-dicyclohexylphosphino-2',6'-diisopropoxy-1,1'-biphenyl (758.0 mg, 1.63 mmol), cesium carbonate (3.3 g, 10.15 mmol), and (R)-1-N-Boc-2-methylpiperazine (609.0 mg, 3.04 mmol) at room temperature, and the reaction mixture was stirred at 100 °C for 2 h. The reaction mixture was concentrated, and the residue was separated and purified by silica gel column chromatography (petroleum ether/ethyl acetate = 3:2) to give EVO29407-6 as a yellow solid (1 g, yield: 77%). LCMS (ESI) m/z = 658.1 [M+H]⁺.

### Step 6:

To a solution of compound EVO29407-6 (1.0 g, 1.52 mmol) in methanol (10 mL) was added a solution of hydrogen chloride in methanol (15 mL, 6 M) at room temperature, and the reaction mixture was stirred at 25 °C for 2 h. The reaction mixture was concentrated to give EVO29407-7 as a pale yellow solid (600 mg, yield: 86%). LCMS (ESI) m/z = 458.1 [M+H]⁺.

### Step 7:

To a solution of compound EVO29407-7 (100.0 mg, 0.22 mmol) in *N,N-*dimethylformamide (1 mL) were added *O*-(7-azabenzotriazol-1-yl)-*N*,*N*,*N*',*N*'-tetramethyluronium hexafluorophosphate (126.0 mg, 0.33 mmol), *N,N*-diisopropylethylamine (142.0 mg, 1.10 mmol), and EVO29407-8 (43.0 mg, 0.33 mmol) at room temperature, and the reaction mixture was stirred at 25 °C for 2 h. An appropriate amount of water (10 mL) was added to the reaction mixture, and then the resulting mixture was extracted with ethyl acetate (3 × 20 mL), followed by liquid separation. The organic phases were combined, dried over anhydrous sodium sulfate, and concentrated, and the residue was separated and purified by silica gel column chromatography (petroleum ether/ethyl acetate = 1:1) to give EVO29407 as a yellow solid (13 mg, yield: 10%). LCMS (ESI) m/z = 571.0 [M+H]⁺. ¹H NMR (400 MHz, DMSO-*d*₆) δ 8.73 (t, *J* = 5.9 Hz, 1H), 8.49 (t, *J* = 17.4 Hz, 1H), 7.13 (d, *J =* 9.1 Hz, 1H), 4.67 (s, 1H), 3.93 - 3.66 (m, 3H), 3.27 - 3.16 (m, 2H), 3.03 (ddd, *J =* 33.9, 11.8, 8.8 Hz, 1H), 2.75 (s, 3H), 1.43 (t, *J =* 6.2 Hz, 3H), 1.38 (d, *J =* 6.8 Hz, 2H), 1.30 (dd, *J =* 8.1, 5.1 Hz, 2H), 1.24 (d, *J =* 4.4 Hz, 10H).

### Example 56

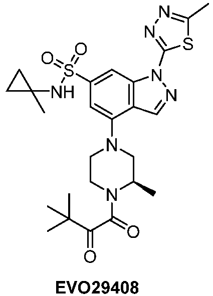

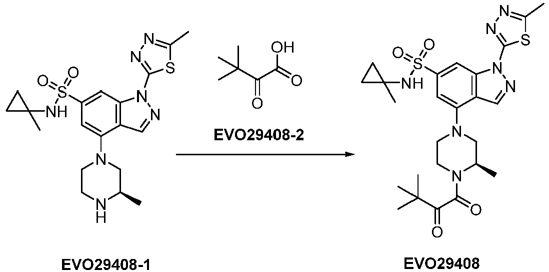

### Step 1:

To a solution of compound EVO29408-1 (100.0 mg, 0.23 mmol) in *N,N-*dimethylformamide (1 mL) were added *O*-(7-azabenzotriazol-1-yl)-*N*,*N*,*N*',*N'*-tetramethyluronium hexafluorophosphate (129.0 mg, 0.34 mmol), *N,N-*diisopropylethylamine (149.0 mg, 1.15 mmol), and EVO29408-2 (45.0 mg, 0.34 mmol) at room temperature, and the reaction mixture was stirred at 25 °C for 2 h. An appropriate amount of water (10 mL) was added to the reaction mixture, and then the resulting mixture was extracted with ethyl acetate (10 mL × 2), followed by liquid separation. The organic phases were combined, dried over anhydrous sodium sulfate, and concentrated, and the residue was separated and purified by silica gel column chromatography (petroleum ether/ethyl acetate = 2:1) to give EVO29408 as a pale yellow solid (15 mg, yield: 12%). LCMS (ESI) m/z = 560.1 [M+H]⁺. ¹H NMR (400 MHz, DMSO-*d*₆) δ 8.71 (d, *J =* 4.1 Hz, 1H), 8.48 (d, *J* = 8.0 Hz, 1H), 8.28 (d, *J* = 2.0 Hz, 1H), 7.13 (d, *J* = 8.2 Hz, 1H), 4.68 (s, 1H), 3.89 - 3.62 (m, 3H), 3.39 (d, *J* = 11.3 Hz, 1H), 3.22 (ddd, *J =* 27.3, 12.5, 3.3 Hz, 1H), 3.02 (ddd, *J =* 33.4, 11.7, 8.6 Hz, 1H), 2.75 (s, 3H), 1.41 (dd, *J =* 21.1, 6.6 Hz, 3H), 1.24 (d, *J =* 4.5 Hz, 9H), 1.07 (s, 3H), 0.74 - 0.54 (m, 2H), 0.40 (s, 2H).

### Effect Example. In Vitro PARG Assay Experiment

The *in vitro* PARG assay experiment was conducted in a standard 384-well plate with a total volume of 15 µL. To a 384-well plate containing the test compound was added 5 µL of an enzyme reaction mixture (50 mM Tris-HCL pH 7.5, 50 mM KCL, 0.01% Triton X-100, 0.01% BSA, and 4 pM His-GST tagged PARG). The plate was incubated at room temperature for 10 min, and then 5 µL of a substrate mixture (50 mM Tris-HCL pH 7.5, 50 mM KCL, 0.01% Triton X-100, 0.01% BSA, and 30 nM Bio PARylated PARP1) was added to initiate the reaction. The plate was further incubated at room temperature for 20 min, and then 5 µL of a detection reagent was added to terminate the reaction. The detection reagent was prepared from a buffer (50 mM Tris-HCl pH 7.5, 50 mM KCL, 0.01% Triton X-100, and 0.01% BSA), 3 µM compound PDD00017273, 1.5× Mab anti-6HIS XL665 (Cisbio: 61HISXLA), and 1.5× streptavidin terbium cryptate (Cisbio: 610SATLA). The final concentrations of the compound PDD00017273, the 1.5× Mab anti-6HIS XL665 (Cisbio: 61HISXLA), and the 1.5× streptavidin terbium cryptate were 1 µM, 0.5×, and 0.5×, respectively. After incubation in the dark at room temperature for 120 min, TR-FRET signals were measured at Ex 340, Em 665, and Em 615. The ratio for each well was calculated as Em 665/Em 615 (the ratio here corresponds to the ratio of fluorescence signals at the wavelengths). The compound inhibition rates were calculated based on the obtained data, and the results are shown in Table 1.

**Table 1**

| Compound | PARG TR-FRET IC₅₀ (uM) |
|---|---|
| EVO29284 | A |
| EVO29285 | A |
| EVO29334 | A |
| EVO29335 | A |
| EVO29339 | A |
| EVO29103 | A |
| EVO29336 | A |
| EVO29338 | A |
| EVO29314 | B |
| EVO29351 | A |
| EVO29337 | A |
| EVO29463 | A |
| EVO29462 | A |
| EVO29496 | A |
| EVO29451 | A |
| EVO29469 | A |
| EVO29534 | A |
| EVO29537 | A |
| EVO29757 | A |
| EVO29751P1 | A |
| EVO29751P2 | A |
| EVO29778 | A |
| EVO29788 | A |
| EVO29792 | B |
| EVO29761 | A |
| EVO29762 | A |
| EVO29752P1 | A |
| EVO29752P2 | A |
| EVO29798 | A |
| EVO29790P1 | A |
| EVO29790P2 | A |
| EVO29725 | B |
| EVO29412 | A |
| EVO29411 | A |
| EVO29409 | A |
| EVO29418 | A |
| EVO29419 | A |
| EVO29410 | A |
| EVO29420 | A |
| EVO29421 | A |
| EVO29426 | A |
| EVO29592 | A |
| EVO29507 | A |
| EVO29503 | A |
| EVO29736 | A |
| EVO29710P1 | A |
| EVO29710P2 | A |
| EVO29715P1 | A |
| EVO29715P2 | A |
| EVO29291 | A |
| EVO29298 | A |
| EVO29386 | B |
| EVO29417 | A |
| EVO29406 | A |
| EVO29405 | A |
| EVO29388 | A |
| EVO29387 | A |
| EVO29384 | A |
| EVO29383 | A |
| EVO29385 | A |
| EVO29407 | A |
| EVO29408 | A |

In the table, A indicates IC₅₀ < 0.1 µM; B indicates 0.1 µM ≤ IC₅₀ < 0.5 µM; C indicates 0.5 µM ≤ IC₅₀ < 1.0 µM; D indicates 1.0 µM ≤ IC₅₀ < 10.0 µM.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A compound represented by formula (I), a pharmaceutically acceptable salt thereof, a solvate thereof, or a solvate of the pharmaceutically acceptable salt thereof: 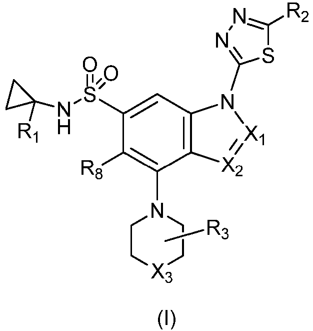 wherein
X₁ and X₂ are independently N or CR₇;
R₇ is independently hydrogen, deuterium, halogen, CN, OH, NRₐR_{b}, C₁-C₆ alkyl, or C₁-C₆ alkyl substituted with 1 or more R₇₋₂;
Rₐ and R_{b} are independently hydrogen or C₁-C₆ alkyl;
R₇₋₂ is independently deuterium, halogen, CN, OH, or NRₐR_{b};
R₈ is hydrogen or halogen;
R₁ is hydrogen, deuterium, halogen, OH, CN, NRₐR_{b}, C₂-C₄ alkynyl, C₁-C₆ alkyl, or C₁-C₆ alkyl substituted with 1 or more R₁₋₂;
R₁₋₂ is independently deuterium, halogen, OH, or NRₐR_{b};
R₂ is hydrogen, deuterium, OH, C₁-C₆ alkyl, C₃-C₆ cycloalkyl, C₁-C₆ alkyl substituted with 1 or more R₂₋₁, or C₃-C₆ cycloalkyl substituted with 1 or more R₂₋₂;
R₂₋₁ and R₂₋₂ are independently deuterium, OH, halogen, or NRₐR_{b};
R₃ is hydrogen, deuterium, OH, NRₐR_{b}, C₁-C₆ alkyl, or C₁-C₆ alkyl substituted with 1 or more R₃₋₂;
R₃₋₂ is independently deuterium, OH, NRₐR_{b}, or halogen;
X₃ is N-(C=O)-L-CR₅R₆-OR₁₁ or N-(C=O)-(C=O)-R₁₂;
L is (CR₉R₁₀)m;
R₉ and R₁₀ are independently hydrogen, deuterium, OH, CN, N(R₉₋₁)₂, C₁-C₆ alkyl, or C₁-C₆ alkyl substituted with one or more R₉₋₂;
R₉₋₁ is independently hydrogen or C₁-C₆ alkyl;
R₉₋₂ is independently deuterium, OH, N(R₉₋₁)₂, or halogen;
m is 0 or 1;
R₅ and R₆ are independently hydrogen, deuterium, OH, CN, NRₐR_{b}, C₁-C₆ alkyl, C₃-C₆ cycloalkyl, C₁-C₆ alkyl substituted with 1 or more R₅₋₂, or C₃-C₆ cycloalkyl substituted with 1 or more R₅₋₃;
R₅₋₂ and R₅₋₃ are independently deuterium, OH, NRₐR_{b}, or halogen; or
R₅ and R₆, together with the carbon atom to which they are attached, form C₃-C₆ cycloalkyl, 3- to 6-membered heterocycloalkyl, C₃-C₆ cycloalkyl substituted with 1 or more R₅₋₃, or 3- to 6-membered heterocycloalkyl substituted with 1 or more R₅₋₄, wherein in each "3- to 6-membered heterocycloalkyl", the number of the heteroatom is independently 1 or 2, and the type of the heteroatom is independently selected from one or more of N, O, and S;
R₅₋₃ and R₅₋₄ are independently deuterium, hydroxy, NRₐR_{b}, or halogen;
R₁₁ is hydrogen, C₁-C₆ alkyl, or C₁-C₆ alkyl substituted with 1 or more halogens;
R₁₂ is NRₐR_{b}, C₁-C₆ alkyl, C₃-C₆ cycloalkyl, C₁-C₆ alkyl substituted with 1 or more R₁₂₋₁, or C₃-C₆ cycloalkyl substituted with one or more R₁₂₋₂;
R₁₂₋₁ and R₁₂₋₂ are independently halogen, OH, CN, or NRₐR_{b}.

2. The compound represented by formula (I), the pharmaceutically acceptable salt thereof, the solvate thereof, or the solvate of the pharmaceutically acceptable salt thereof according to claim 1, wherein one or more of the following conditions are met:
(1) each "halogen" is independently F, Cl, Br, or I, such as F;
(2) each "C₁-C₆ alkyl" is independently methyl, ethyl, *n*-propyl, isopropyl, *n*-butyl, *sec*-butyl, or *tert-*butyl, such as methyl, ethyl, isopropyl, or *tert-butyl;*
(3) each "C₂-C₄ alkynyl" is independently ethynyl, propinyl, or propargyl, such as ethynyl;
(4) each "C₃-C₆ cycloalkyl" is independently cyclopropyl, cyclobutyl, cyclopentyl, or cyclohexyl, such as cyclopropyl; and
(5) each "3- to 6-membered heterocycloalkyl" is independently 3- to 6-membered heterocycloalkyl having 1 or 2 heteroatoms of N and/or O, such as oxiranyl, aziridinyl, oxetanyl, azetidinyl, tetrahydropyrrolyl, tetrahydrofuranyl, tetrahydropyranyl, piperidinyl, morpholinyl, or piperazinyl.

3. The compound represented by formula (I), the pharmaceutically acceptable salt thereof, the solvate thereof, or the solvate of the pharmaceutically acceptable salt thereof according to claim 1, wherein one or more of the following conditions are met:
(1) X₂ is CR₇;
(2) R₇ is independently hydrogen or halogen;
(3) R₈ is hydrogen;
(4) R₁ is hydrogen, CN, C₂-C₄ alkynyl, or C₁-C₆ alkyl, preferably CN, C₂-C₄ alkynyl, or C₁-C₆ alkyl, and further preferably CN or C₁-C₆ alkyl;
(5) R₂ is C₁-C₆ alkyl or C₁-C₆ alkyl substituted with 1, 2, or 3 R₂₋₁, preferably methyl or methyl substituted with 1, 2, or 3 R₂₋₁, and further preferably methyl substituted with 1, 2, or 3 R₂₋₁;
(6) R₂₋₁ is halogen;
(7) R₃ is hydrogen, C₁-C₆ alkyl, or C₁-C₆ alkyl substituted with 1, 2, or 3 R₃₋₂, preferably C₁-C₆ alkyl or C₁-C₆ alkyl substituted with 1, 2, or 3 R₃₋₂, and further preferably methyl or methyl substituted with 1, 2, or 3 R₃₋₂;
(8) R₃₋₂ is independently halogen;
(9) R₉ and R₁₀ are independently hydrogen, N(R₉₋₁)₂, or C₁-C₆ alkyl, such as N(R₉₋₁)₂ or C₁-C₆ alkyl, preferably C₁-C₆ alkyl;
(10) R₉₋₁ is independently hydrogen;
(11) m is 0;
(12) R₅ and R₆ are independently hydrogen, deuterium, C₁-C₆ alkyl, C₃-C₆ cycloalkyl, C₁-C₆ alkyl substituted with 1, 2, or 3 R₅₋₂, or C₃-C₆ cycloalkyl substituted with 1, 2, or 3 R₅₋₃, preferably hydrogen, C₁-C₆ alkyl, C₃-C₆ cycloalkyl, or C₁-C₆ alkyl substituted with 1, 2, or 3 R₅₋₂, and further preferably C₁-C₆ alkyl or C₁-C₆ alkyl substituted with 1, 2, or 3 R₅₋₂; and
(13) R₅₋₂ and R₅₋₃ are independently OH or halogen, preferably halogen.

4. The compound represented by formula (I), the pharmaceutically acceptable salt thereof, the solvate thereof, or the solvate of the pharmaceutically acceptable salt thereof according to claim 1, wherein one or two of the following conditions are met:
(1) R₁₀ is hydrogen; and
(2) R₅ is hydrogen or C₁-C₆ alkyl, and R₆ is hydrogen, C₁-C₆ alkyl, C₃-C₆ cycloalkyl, or C₁-C₆ alkyl substituted with 1 or more R₅₋₂, wherein R₅₋₂ is independently OH or halogen.

5. The compound represented by formula (I), the pharmaceutically acceptable salt thereof, the solvate thereof, or the solvate of the pharmaceutically acceptable salt thereof according to claim 1, wherein one or more of the following conditions are met:
(1) X₁ is N or CH;
(2) X₂ is N, CH, or CF, preferably CH or CF;
(3) R₈ is H or F, preferably H;
(4) R₁ is hydrogen, CN, ethynyl, or methyl, such as CN, ethynyl, or methyl, preferably CN or methyl, and further preferably CN;
(5) R₂ is methyl or CHF₂, preferably CHF₂;
(6) R₃ is hydrogen, methyl, or CH₂F, preferably methyl or CH₂F;
(7) 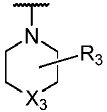 is 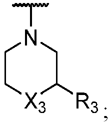
(8) R₅ is hydrogen or methyl;
(9) R₆ is hydrogen, methyl, ethyl, n-propyl, isopropyl, n-butyl, isobutyl, *tert-butyl,* cyclopropyl, methyl substituted with 1, 2, or 3 fluorine atoms, *tert-butyl* substituted with 1, 2, or 3 fluorine atoms, or *tert-*butyl substituted with 1, 2, or 3 OH groups, such as hydrogen, methyl, isopropyl, *tert-butyl,* cyclopropyl, CF₃, 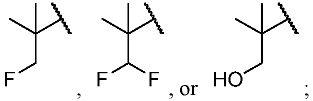 preferably, R₆ is methyl, *tert-butyl,* methyl substituted with 1, 2, or 3 fluorine atoms, or *tert-butyl* substituted with 1, 2, or 3 fluorine atoms, such as methyl, *tert*-butyl, CF₃, 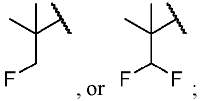
(10) R₁₁ is hydrogen, methyl, ethyl, or methyl substituted with 1, 2, or 3 fluorine atoms, such as hydrogen, methyl, ethyl, or CHF₂; and
(11) R₁₂ is isopropyl, amino, cyclopropyl, *tert-butyl,* or methyl.

6. The compound represented by formula (I), the pharmaceutically acceptable salt thereof, the solvate thereof, or the solvate of the pharmaceutically acceptable salt thereof according to claim 1, wherein one or more of the following conditions are met:
(1) 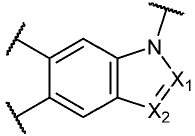 is 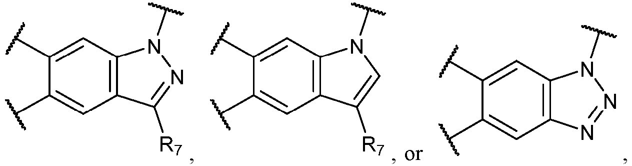 such as 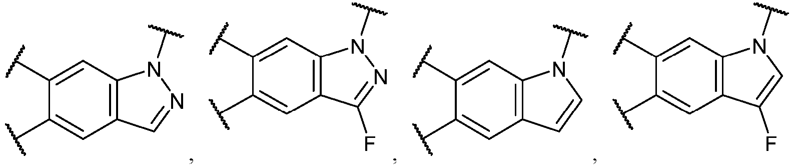 , or 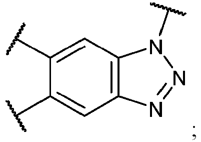
(2) 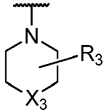 is 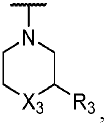 such as 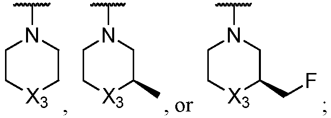 and
(3) 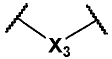 is

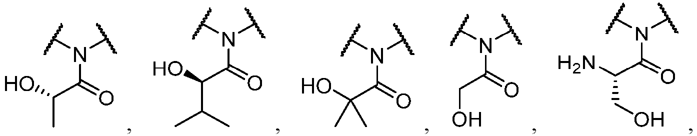

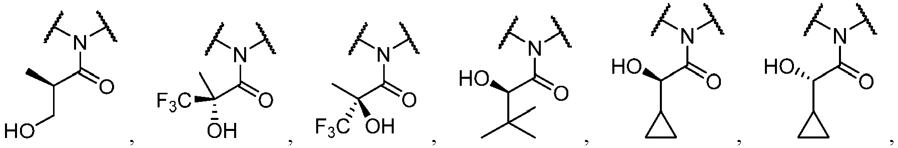

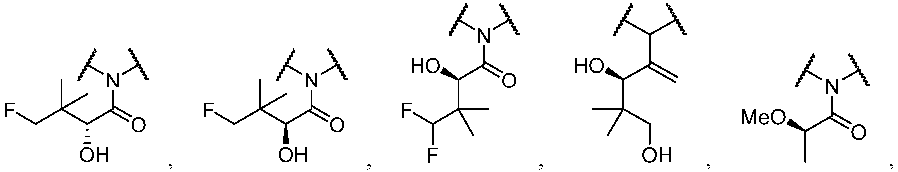

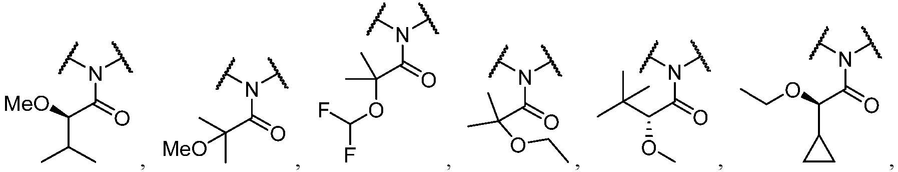

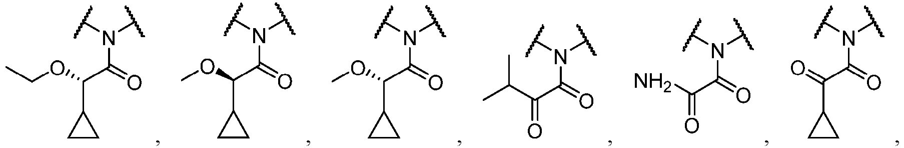

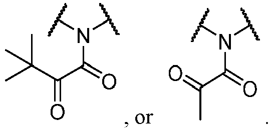

7. The compound represented by formula (I), the pharmaceutically acceptable salt thereof, the solvate thereof, or the solvate of the pharmaceutically acceptable salt thereof according to at least one of claims 1-6, wherein the compound represented by formula (I) is a compound represented by formula (I-1), (I-2) or (I-3): 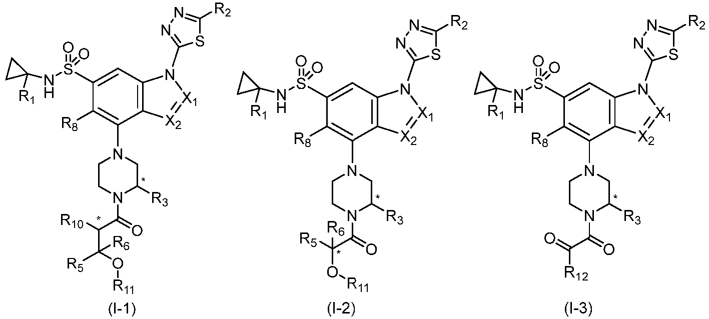 wherein the carbon atom marked with "*" indicates that when the carbon atom is a chiral carbon atom, it is independently in R configuration, S configuration, or a mixture thereof; X₁, X₂, R₈, R₁, R₂, R₃, R₁₀, R₅, R₆, R₁₁, and R₁₂ are as defined in at least one of claims 1-6.

8. The compound represented by formula (I), the pharmaceutically acceptable salt thereof, the solvate thereof, or the solvate of the pharmaceutically acceptable salt thereof according to at least one of claims 1-6, wherein the compound represented by formula (I) is a compound represented by formula (I-5), (I-6), (I-7), (I-8), (I-9), (I-10), (I-11), (I-12) or (I-13): 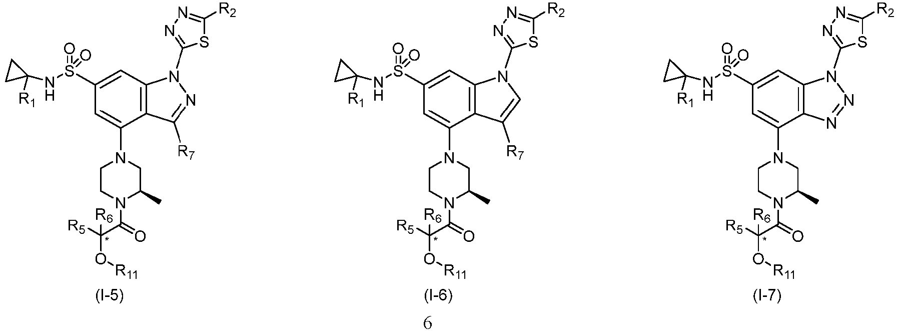 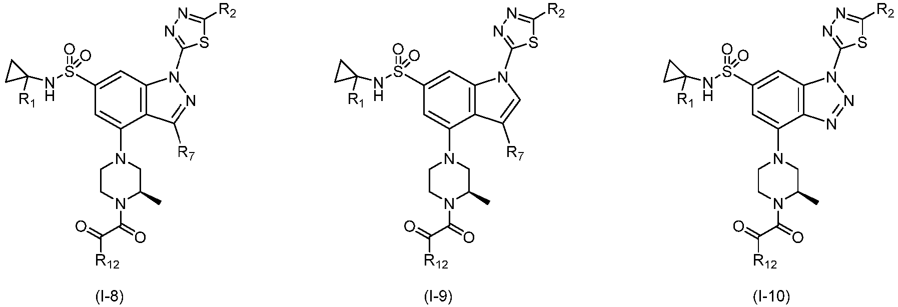 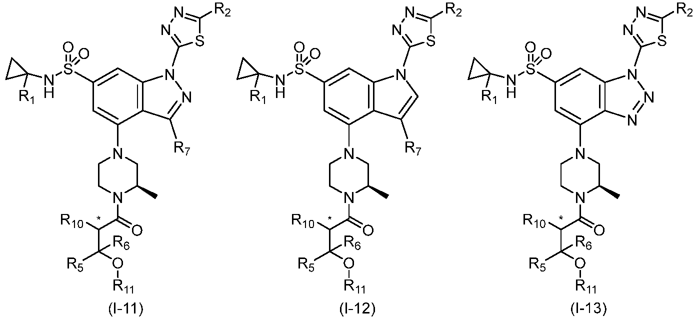
wherein the carbon atom marked with "*" indicates that when the carbon atom is a chiral carbon atom, it is independently in R configuration, S configuration, or a mixture thereof; R₁, R₂, R₇, R₅, R₆, R₁₁, and R₁₂ are as defined in at least one of claims 1-6;
preferably, the formulas described above meet one or two of the following conditions:
I: R₂ is CHF₂; and
II: R₁ is CN.

9. The compound represented by formula (I), the pharmaceutically acceptable salt thereof, the solvate thereof, or the solvate of the pharmaceutically acceptable salt thereof according to at least one of claims 1-8, wherein the compound represented by formula (I) is any one of the following compounds:

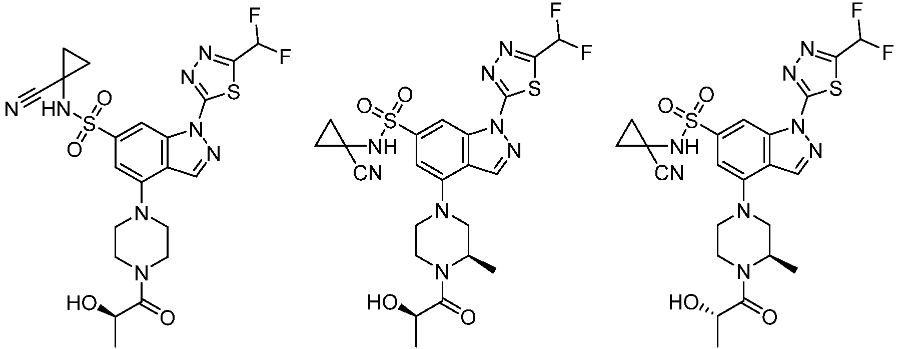

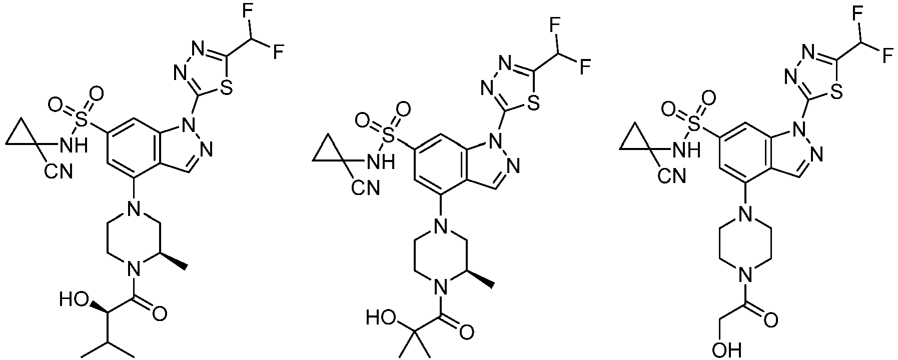

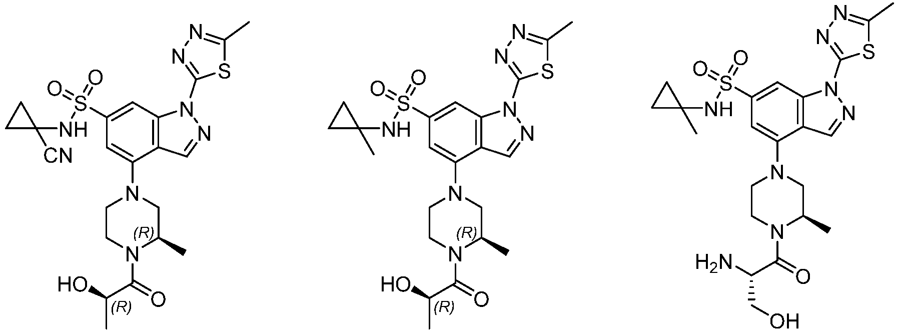

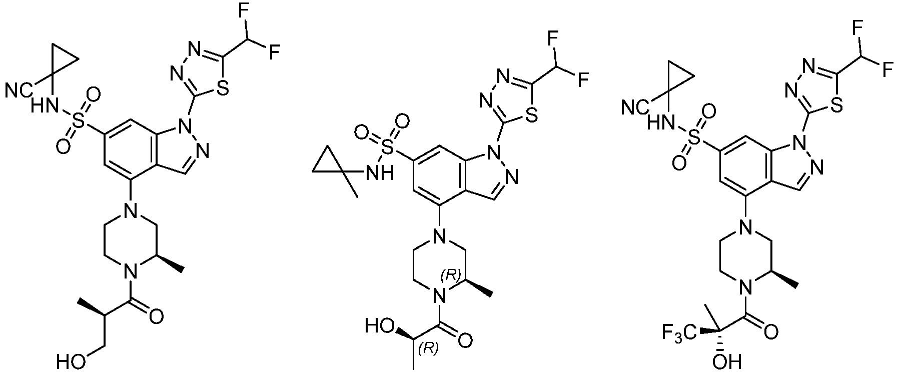

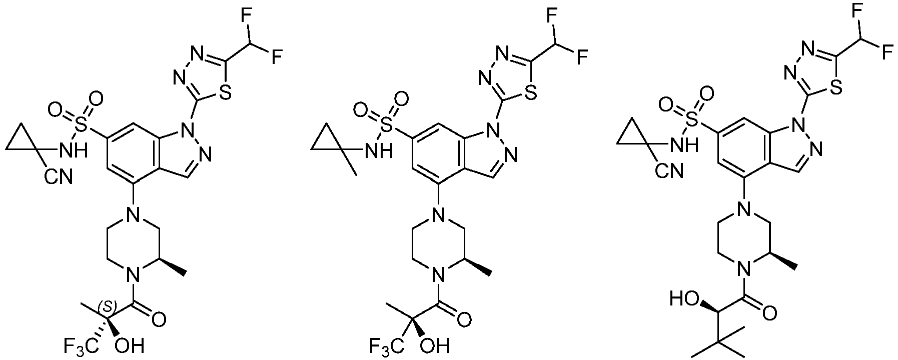

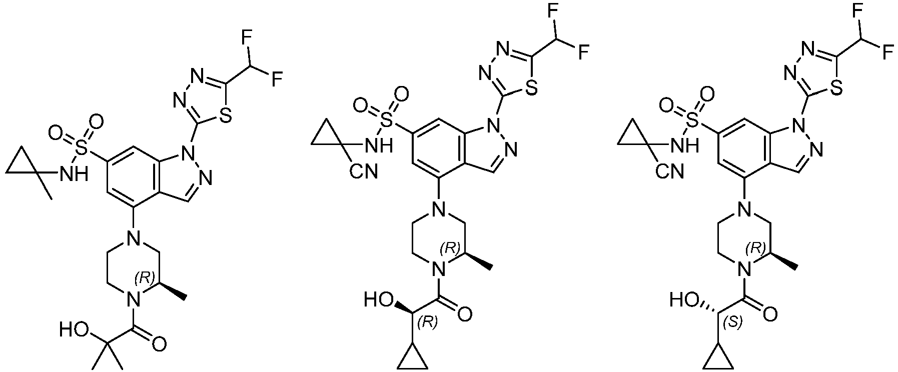

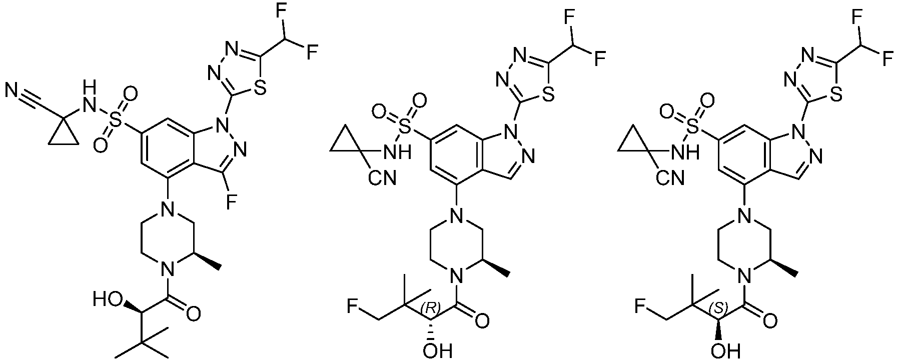

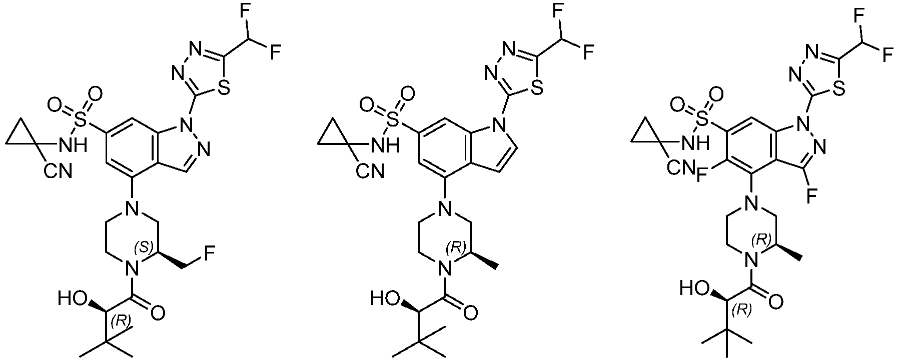

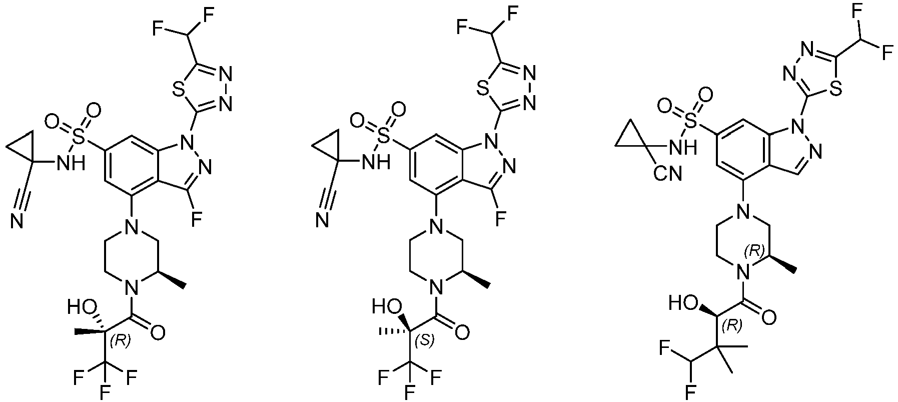

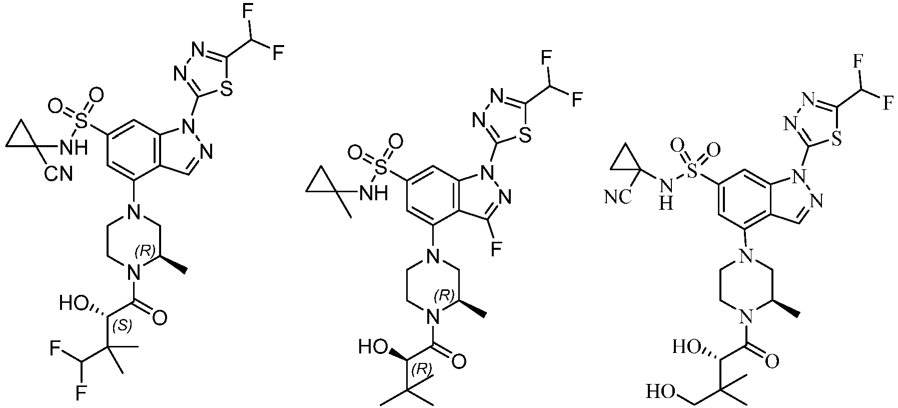

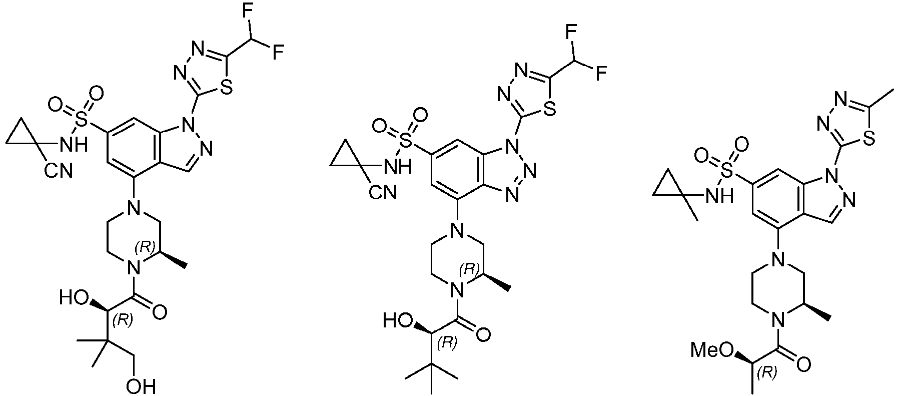

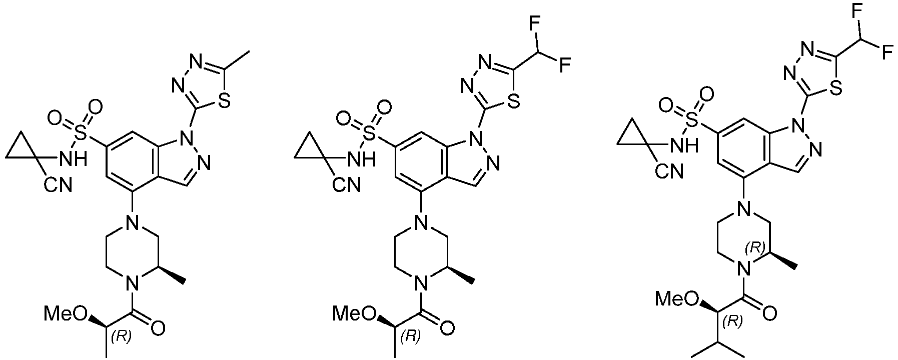

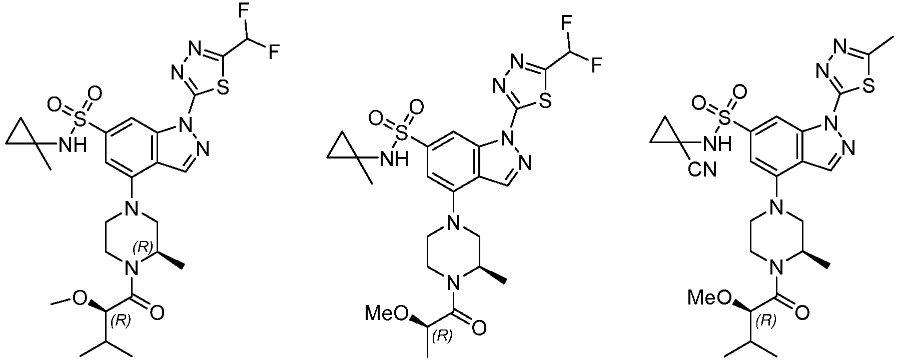

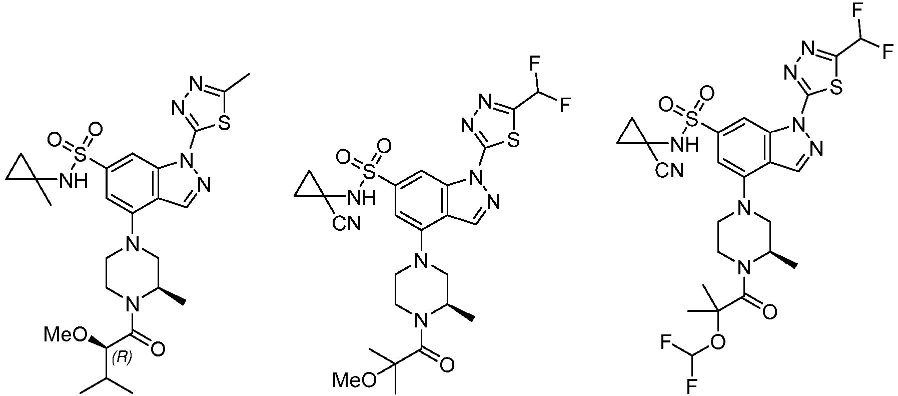

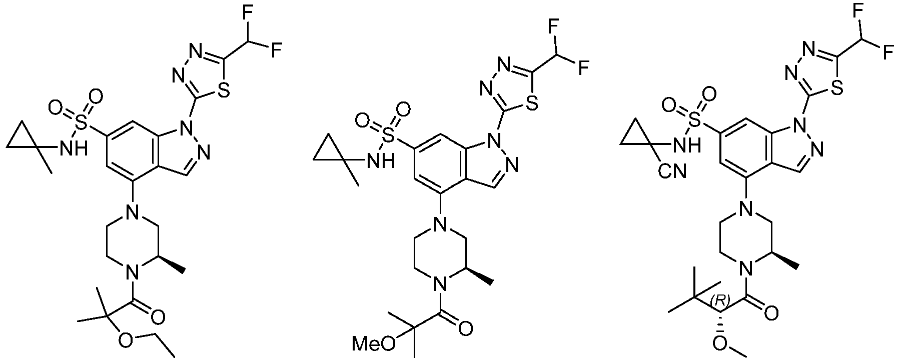

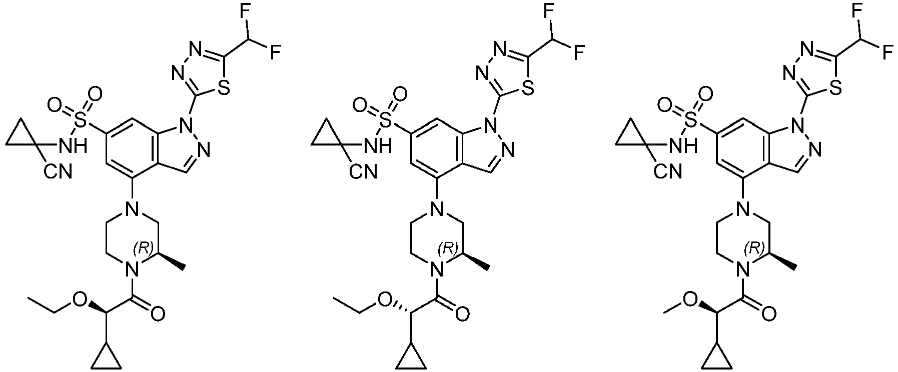

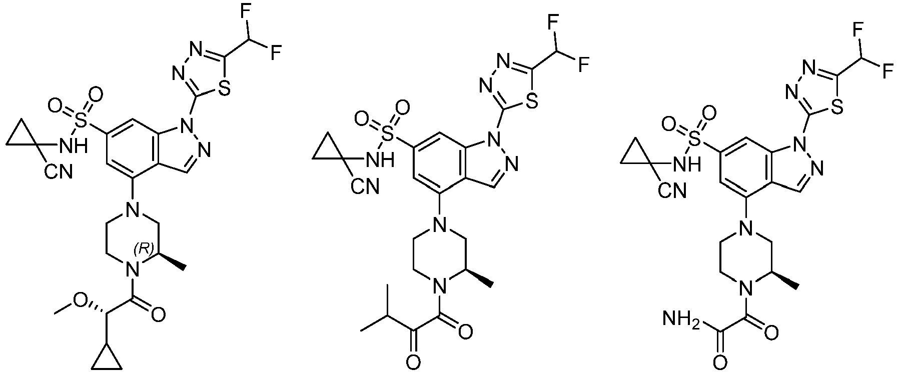

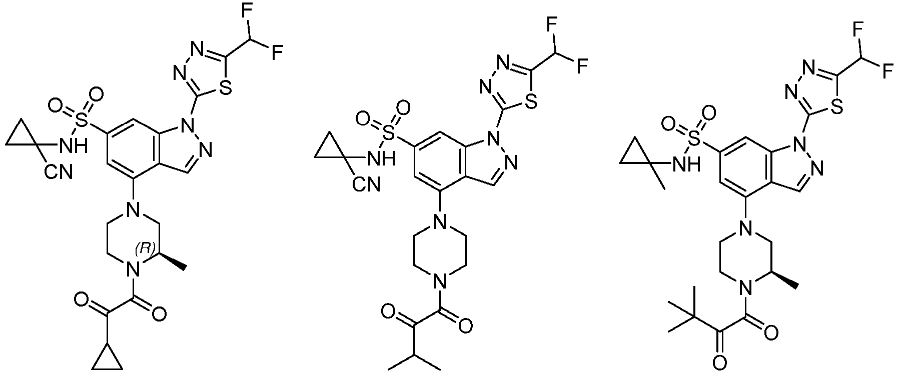

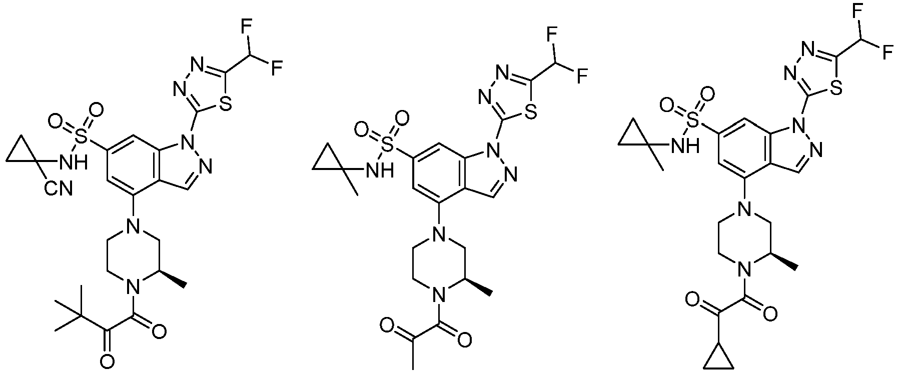

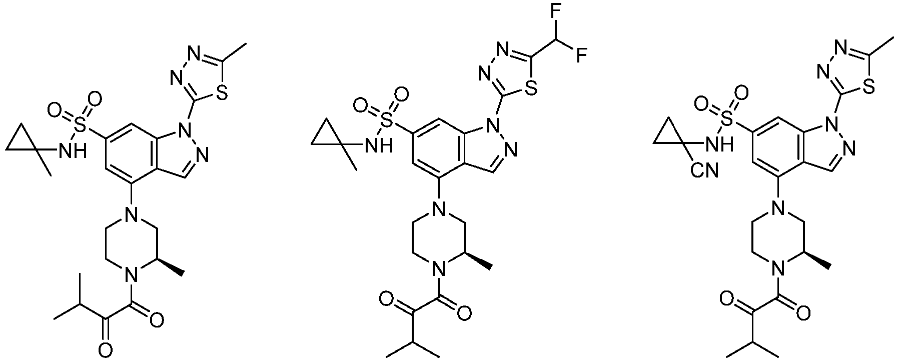

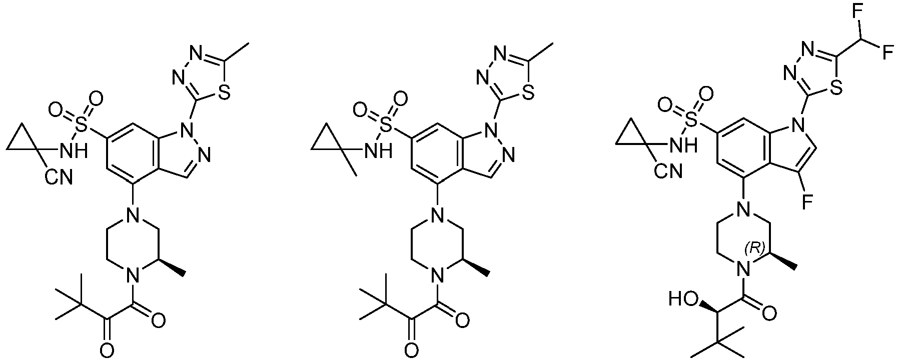

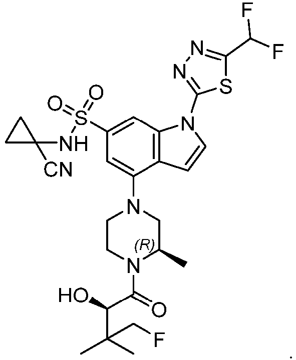

10. A pharmaceutical composition, comprising a substance X and a pharmaceutically acceptable auxiliary material, wherein the substance X is the compound represented by formula (I), the pharmaceutically acceptable salt thereof, the solvate thereof, or the solvate of the pharmaceutically acceptable salt thereof according to at least one of claims 1-9.

11. Use of a substance X or the pharmaceutical composition according to claim 10 in the preparation of a PARG inhibitor, wherein the substance X is the compound represented by formula (I), the pharmaceutically acceptable salt thereof, the solvate thereof, or the solvate of the pharmaceutically acceptable salt thereof according to at least one of claims 1-9.

12. Use of a substance X or the pharmaceutical composition according to claim 10 in the preparation of a medicament for treating a PARG-related disease, wherein the substance X is the compound represented by formula (I), the pharmaceutically acceptable salt thereof, the solvate thereof, or the solvate of the pharmaceutically acceptable salt thereof according to at least one of claims 1-9; preferably, the PARG-related disease is ovarian cancer, breast cancer, pancreatic cancer, prostate cancer, or gastric cancer.

13. Use of a substance X or the pharmaceutical composition according to claim 10 in the preparation of a medicament for preventing and/or treating a cancer, wherein the substance X is the compound represented by formula (I), the pharmaceutically acceptable salt thereof, the solvate thereof, or the solvate of the pharmaceutically acceptable salt thereof according to at least one of claims 1-9; preferably, the cancer is ovarian cancer, breast cancer, pancreatic cancer, prostate cancer, or gastric cancer.
